(19) Europäisches Patentamt
European Patent Office
Office européen des brevets

(11) EP 2 038 287 B1

(12) **EUROPEAN PATENT SPECIFICATION**

(45) Date of publication and mention of the grant of the patent:
**26.12.2012 Bulletin 2012/52**

(21) Application number: **07724484.6**

(22) Date of filing: **23.04.2007**

(51) Int Cl.:
***C07D 491/10*** *(2006.01)* ***C07D 491/20*** *(2006.01)*
***A61K 31/407*** *(2006.01)* ***A61P 25/28*** *(2006.01)*
***A61P 23/00*** *(2006.01)* ***A61P 29/00*** *(2006.01)*

(86) International application number:
**PCT/EP2007/003554**

(87) International publication number:
**WO 2007/121976 (01.11.2007 Gazette 2007/44)**

(54) **SPIRO[BENZOPYRAN]OR SPIRO[BENZOFURAN]DERIVATIVES WHICH INHIBIT THE SIGMA RECEPTOR**

SPIRO[BENZOPYRAN]- ODER SPIRO[BENZOFURAN]DERIVATE, DIE DEN SIGMA-REZEPTOR INHIBIEREN

DÉRIVÉS DE SPIRO[BENZOPYRANNE]OU DE SPIRO[BENZOFURANE]QUI INHIBENT LE RÉCEPTEUR SIGMA

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **21.04.2006 EP 06384007**

(43) Date of publication of application:
**25.03.2009 Bulletin 2009/13**

(73) Proprietor: **Laboratorios del. Dr. Esteve, S.A.**
**08041 Barcelona (ES)**

(72) Inventor: **WÜNSCH, Bernhard**
**48149 Münster (DE)**

(74) Representative: **Peters, Hajo et al**
**ZACCO GmbH**
**Bayerstrasse 83**
**80335 München (DE)**

(56) References cited:

| | |
|---|---|
| **WO-A-02/48152** | **WO-A1-00/06545** |
| **WO-A1-01/07050** | **US-A- 3 686 186** |
| **US-A- 3 959 475** | **US-A- 3 962 259** |

- **MARXER, ADRIAN ET AL: "Spiro piperidines. I. Synthesis of spiro[isobenzofuran-1(3H), 4'-piperidines] and spiro[isobenzofuran-1(3H), 3'-piperidines]" JOURNAL OF ORGANIC CHEMISTRY , 40(10), 1427-33 CODEN: JOCEAH; ISSN: 0022-3263, 1975, XP000568850**
- **MAIER, CHRISTOPH A. ET AL: "Novel .sigma. Receptor Ligands. Part 2. SAR of Spiro[[2] benzopyran-1,4'- piperidines] and Spiro[[2] benzofuran-1,4'-piperidines] with Carbon Substituents in Position 3" JOURNAL OF MEDICINAL CHEMISTRY , 45(22), 4923-4930 CODEN: JMCMAR; ISSN: 0022-2623, 2002, XP002388302**
- **MAIER, CHRISTOPH A. ET AL: "Novel Spiropiperidines as Highly Potent and Subtype Selective .sigma.-Receptor Ligands. Part 1" JOURNAL OF MEDICINAL CHEMISTRY , 45(2), 438-448 CODEN: JMCMAR; ISSN: 0022-2623, 2002, XP002388303**
- **DHAR, T.G. MURALI ET AL: "Design and Synthesis of Novel alpha1a Adrenoceptor-Selective Antagonists. 2. Approaches To Eliminate Opioid Agonist Metabolites via Modification of Linker and 4-Methoxycarbonyl-4-phenylpiperidine Moiety" JOURNAL OF MEDICINAL CHEMISTRY, vol. 42, no. 23, 1999, pages 4778-4793, XP002388304**
- **WUENSCH, BERNARD ET AL: "Synthese und ZNS-Aktivitàt spirocyclischer Pethid- und Prodin-Analoga" ARCHIV PHARM., vol. 326, 1993, pages 513-518, XP009068721 Weinheim**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

The present invention relates to compounds having pharmacological activity towards the sigma (σ) receptor, and more particularly to some spiro[benzopyran] or spiro[benzofuran] derivatives, to processes of preparation of such compounds, to pharmaceutical compositions comprising them, and to their use in therapy and prophylaxis, in particular for the treatment of psychosis.

### BACKGROUND OF THE INVENTION

The search for new therapeutic agents has been greatly aided in recent years by better understanding of the structure of proteins and other biomolecules associated with target diseases. One important class of these proteins is the sigma (σ) receptor, a cell surface receptor of the central nervous system (CNS) which may be related to the dysphoric, hallucinogenic and cardiac stimulant effects of opioids. From studies of the biology and function of sigma receptors, evidence has been presented that sigma receptor ligands may be useful in the treatment of psychosis and movement disorders such as dystonia and tardive dyskinesia, and motor disturbances associated with Huntington's chorea or Tourette's syndrome and in Parkinson's disease (Walker, J.M. et al, Pharmacological Reviews, 1990, 42, 355). It has been reported that the known sigma receptor ligand rimcazole clinically shows effects in the treatment of psychosis (Snyder, S.H., Largent, B.L. J. Neuropsychiatry 1989, 1, 7). The sigma binding sites have preferential affinity for the dextrorotatory isomers of certain opiate benzomorphans, such as (+)SKF 10047, (+)cyclazocine, and (+)pentazocine and also for some narcoleptics such as haloperidol.

The sigma receptor has at least two subtypes, which may be discriminated by stereoselective isomers of these pharmacoactive drugs. SKF 10047 has nanomolar affinity for the sigma 1 (σ-1) site, and has micromolar affinity for the sigma (σ-2) site. Haloperidol has similar affinities for both subtypes. Endogenous sigma ligands are not known, although progesterone has been suggested to be one of them. Possible sigma-site-mediated drug effects include modulation of glutamate receptor function, neurotransmitter response, neuroprotection, behavior, and cognition (Quirion, R. et al. Trends Pharmacol. Sci., 1992, 13:85-86). Most studies have implied that sigma binding sites (receptors) are plasmalemmal elements of the signal transduction cascade. Drugs reported to be selective sigma ligands have been evaluated as antipsychotics (Hanner, M. et al. Proc. Natl. Acad. Sci., 1996, 93:8072-8077). The existence of sigma receptors in the CNS, immune and endocrine systems have suggested a likelihood that it may serve as link between the three systems.

Spiro-compounds and their synthesis are known in the art (US Patent no. 3686186; US Patent no. 3962259; J. Org. Chem., 1975, p. 1427 ff; US Patent no. 3959475). Analgesic properties of spiro-compounds have been described (Archiv. Pharm., 1993, p. 513 ff.) as well as the role of spiro-compounds as adreno receptor- or nocireceptor antagonists (J. Med. Chem., 1999, p. 4478 ff.; WO 01/07050; WO 00/06545). Some spiro-compounds have been shown to act as ligands of neuropeptide Y5-receptor and of sigma receptors (WO 02/48152, J. Med. Chem., 2002, p. 4923 ff.; J. Med. Chem., 2002, p. 438 ff.).

There is still a need to find compounds that have pharmacological activity towards the sigma receptor, being both effective and selective, and having good "drugability" properties, i.e. good pharmaceutical properties related to administration, distribution, metabolism and excretion.

### SUMMARY OF THE INVENTION

We have now found a family of structurally distinct spiro[benzopyran] or spiro[benzofuran] derivatives which are particularly selective inhibitors of the sigma receptor.

The invention is directed to compounds of general formula Ia,

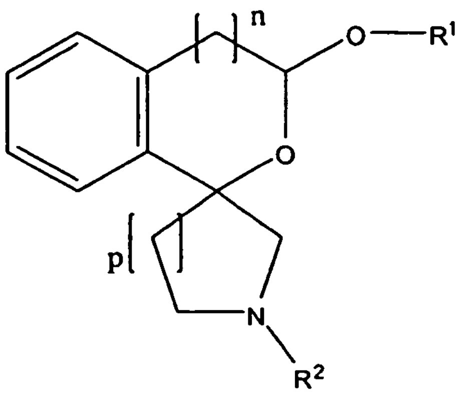

**Ia**

wherein

n is selected from 0 or 1;

p is selected from 1 or 2

$R^1$ is selected from hydrogen, $C_{1-6}$-alkyl

$R^2$ is selected from hydrogen; optionally at least mono-substituted $C_{1-18}$-aliphatic-group, saturated or unsaturated, linear or branched; optionally at least mono-substituted $(CH_2)_{1-6}$-aryl, $(CH_2)_{1-6}$-heteroaryl, $(CH_2)_{1-6}$-cycloalkyl, cycloalkyl, aryl or heterocyclyl;

or $R^2$ together with the bonded nitrogen is binding the nitrogen in a cycloalkyl-spiro-complex thus forming a quarternary ammonium according to formula X (with q being 1 or 2):

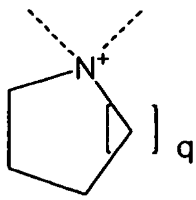

All compounds according to formula Ia as depicted above also fall under general formula I as depcited below. Therefore any mentioning of compunds of formula I also applies to the compounds of formula Ia.

A non-claimed embodiment of the invention is directed to compounds according to general formula I:

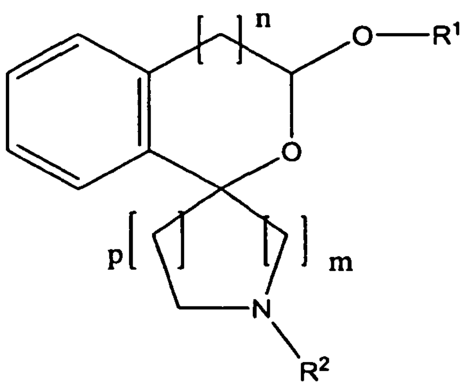

**(I)**

wherein

m is selected from 1 or 2, p is selected from 1 or 2, and m + p is either 2 or 3;

n is selected from 0 or 1;

$R^1$ is selected from hydrogen, $C_{1-6}$-aliphatic-group, saturated or unsaturated, linear or branched, substituted or unsubstituted; alkyl-aryl, substituted or unsubstituted;

$R^2$ is selected from hydrogen; $C_{1-18}$-aliphatic-group, saturated or unsaturated, linear or branched, substituted or unsubstituted; optionally at least mono-substituted aryl or alkyl-aryl; optionally at least mono-substituted heteroaryl or alkyl-heteroaryl; optionally at least mono-substituted cycloalkyl or alkyl-cycloalkyl, or $R^2$ together with the bonded nitrogen is binding the nitrogen in a cycloalkyl-spiro-complex thus forming a quarternary ammonium according to formula X (with q being 1 or 2):

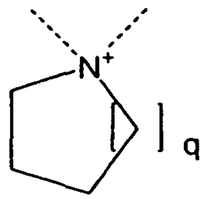

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

Specifically if $R^2$ together with the bonded Nitrogen is binding the nitrogen in a cycloalkyl-spiro-complex thus forming a quarternary ammonium according to formula X (with q being 1 or 2):

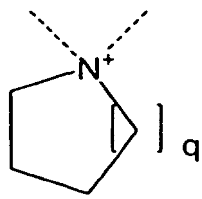

;

this means that the compound of general formula I turns into a compound according to general formula Xa (with q being 1 or 2):

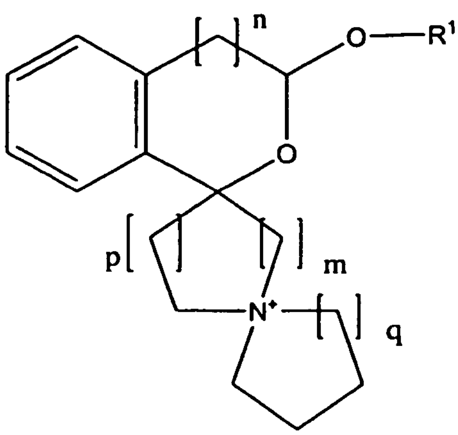

A non-claimed embodiment of the invention is directed to compounds according to general formula (I)

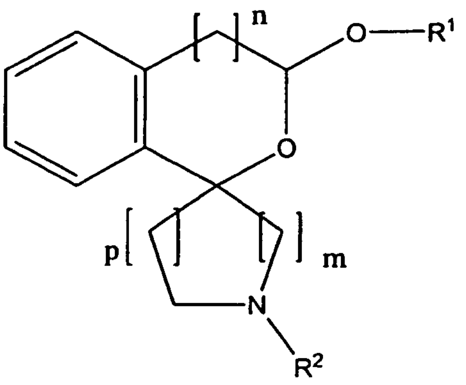

(I) ,

wherein

m is selected from 1 or 2, p is selected from 1 or 2, and m + p is either 2 or 3;

n is selected from 0 or 1;

$R^1$ is selected from hydrogen, $C_{1-6}$-alkyl, saturated or unsaturated, linear or branched, substituted or unsubstituted; alkyl-aryl, substituted or unsubstituted; $R^2$ is selected from hydrogen, $C_{1-18}$-alkyl, saturated or unsaturated, linear or branched, substituted or unsubstituted; aryl or alkyl-aryl, substituted or unsubstituted; heteroaryl or alkyl-heteroaryl, substituted or unsubstituted; cycloalkyl or alkyl-cycloalkyl, substituted or unsubstituted,

or $R^2$ together with the bonded Nitrogen is binding the nitrogen in a cycloalkyl-spiro-complex thus forming a quarternary ammonium according to formula X (with q being 1 or 2):

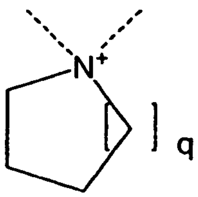

;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In the context of this invention aliphatic group or aliphatic radical includes alkyl, alkenyl and alkinyl.

In the context of this invention, alkyl radical or group is understood as meaning saturated, linear or branched hydrocarbons, which can be unsubstituted or mono- or polysubstituted. Alkenyl and alkinyl groups, on the other hand include groups like e.g. $-CH=CH-CH_3$ or $-C\equiv C-CH_3$, while the saturated alkyl encompasses e.g. $-CH_3$ and - $CH_2-CH_3$. In these radicals, $C_{1-2}$-alkyl represents C1- or C2-alkyl, $C_{1-3}$-alkyl represents $C_1$-, C2- or C3-alkyl, $C_{1-4}$-alkyl represents C1-, C2-, C3- or C4-alkyl, $C_{1-5}$-alkyl represents C1-, C2-, C3-, C4-, or C5-alkyl, $C_{1-6}$-alkyl represents C1-, C2-, C3-, C4-, C5- or C6-alkyl, $C_{1-7}$-alkyl represents $C_1$-, C2-, C3-, C4-, C5-, C6- or C7-alkyl, $C_{1-8}$-alkyl represents C1-, C2-, C3-, C4-, C5-, C6-, C7- or $C_8$-alkyl, $C_{1-10}$-alkyl represents $C_1$-, C2-, C3-, C4-, C5-, C6-, C7-, C8-, C9- or C10-alkyl and $C_{1-18}$-alkyl represents C1-, C2-, C3-, C4-, C5-, C6-, C7-, C8-, C9-, C10-, C11-, C12-, C13-, C14-, C15-, C16-, C17- or C18-alkyl. The alkyl radicals are preferably methyl, ethyl, propyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also $CHF_2$. $CF_3$ or $CH_2OH$ etc.

In the context of this invention cycloalkyl radical or group is understood as meaning saturated and unsaturated (but not aromatic) cyclic hydrocarbons (without a heteroatom in the ring), which can be unsubstituted or mono- or polysubstituted. Furthermore, $C_{3-4}$-cycloalkyl represents C3- or C4-cycloalkyl, $C_{3-5}$-cycloalkyl represents C3-, C4- or

C5-cycloalkyl, $C_{3-6}$-cycloalkyl represents C3-, C4-, C5- or C6-cycloalkyl, $C_{3-7}$-cycloalkyl represents C3-, C4-, C5-, C6- or $C_7$-cycloalkyl, $C_{3-8}$-cycloalkyl represents C3-, C4-, C5-, C6-, C7- or C8-cycloalkyl, $C_{4-5}$-cycloalkyl represents C4- or C5-cycloalkyl, $C_{4-6}$-cycloalkyl represents C4-, C5- or C6-cycloalkyl, $C_{4-7}$-cycloalkyl represents C4-, C5-, C6- or C7-cycloalkyl, $C_{5-6}$-cycloalkyl represents C5- or C6-cycloalkyl and $C_{5-7}$-cycloalkyl represents C5-, C6- or C7-cycloalkyl. However, mono- or polyunsaturated, preferably monounsaturated, cycloalkyls also in particular fall under the term cycloalkyl as long as the cycloalkyl is not an aromatic system. The cycloalkyl radicals are preferably cyclopropyl, 2-methylcyclopropyl, cyclopropylmethyl, cyclobutyl, cyclopentyl, cyclopentylmethyl, cyclohexyl, cycloheptyl, cyclooctyl, and also adamantyl.

In the context of this invention alkyl-cycloalkyl is understood as meaning a cycloalkyl group (see above) being connected to another atom through a $C_{1-6}$-alkyl group (see above), whereas the $C_{1-6}$-alkyl-group is always saturated and unsubstituted, and linear or branched. More preferably in the context of this invention alkyl-cycloalkyl is understood as meaning one cycloalkyl group - optionally at least mono-substituted - being connected to the scaffold of a given general formula by $(CH_2)_n$ e.g. $(CH_2)_{1-6}$; i.e. an alkylene-group. Thus it can also be expressed as $(CH_2)_{1-6}$-cyloalkyl. Examples include $(CH_2)_{12}$-cyclopropyl, $(CH_2)_{1-6}$-2-methylcyclopropyl, $(CH_2)_{1-6}$-cyclopropylmethyl, $(CH_2)_{1-6}$-cyclobutyl, $(CH_2)_{1-6}$-cyclopentyl, $(CH_2)_{1-6}$-cyclopentylmethyl, $(CH_2)_{1-6}$-cyclohexyl, $(CH_2)_{1-6}$-cycloheptyl, $(CH_2)_{1-6}$-cyclooctyl, and $(CH_2)_{1-6}$-adamantyl.

In connection with alkyl or aliphatic group - unless defined otherwise - the term substituted in the context of this invention is understood as meaning replacement of at least one hydrogen radical by F, Cl, Br, I, $NH_2$. SH or OH, and does include "mono-" (once substituted) or "polysubstituted" (more than once substituted) radicals. "Polysubstituted" being understood as meaning that the replacement takes effect both on different and on the same atoms several times with the same or different substituents, for example three times on the same C atom, as in the case of $CF_3$, or at different places, as in the case of e.g. $-CH(OH)-CH_2-CH_2-CHCl_2-$. "Optionally at least monosubstituted" means either "monosubstituted", "polysubstituted" or - if the option is not fulfilled - "unsubstituted".

The term $(CH_2)_{3-6}$ (an alkylen) is to be understood as meaning $-CH_2-CH_2-CH_2-$, $-CH_2-CH_2-CH_2-CH_2-$, $-CH_2-CH_2-CH_2-CH_2-CH_2-$ and $-CH_2-CH_2-CH_2-CH_2-CH_2-CH_2-$; $(CH_2)_{1-6}$ is to be understood as meaning $-CH_2-$, $-CH_2-CH_2-$, $-CH_2-CH_2-CH_2-$, $-CH_2-CH_2-CH_2-CH_2-$, $-CH_2-CH_2-CH_2-CH_2-CH_2-$, and $-CH_2-CH_2-CH_2-CH_2-CH_2-CH_2-$; $(CH_2)_{1-4}$ is to be understood as meaning $-CH_2-$, $-CH_2-CH_2-$, $-CH_2-CH_2-CH_2$ and $-CH_2-CH_2-CH_2-CH_2-$, $(CH_2)_{4-5}$ is to be understood as meaning $-CH_2-CH_2-CH_2-CH_2-$ and $-CH_2-CH_2-CH_2-CH_2-CH_2-$, etc.

An aryl radical or group is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

In the context of this invention alkyl-aryl is understood as meaning an aryl group (see above) being connected to another atom through a $C_{1-6}$-alkyl-group (see above), whereas the $C_{1-6}$-alkyl-group is always saturated and unsubstituted, and linear or branched. More preferably in the context of this invention alkyl-aryl is understood as meaning one aryl group - optionally at least mono-substituted - being connected to the scaffold of a given general formula by $(CH_2)_n$ e.g. $(CH_2)_{1-6}$; i.e. an alkylene-group. Thus it can also be expressed as $(CH_2)_{1-6}$-aryl. Examples include $(CH_2)_{1-6}$-phenyl, e,g, benzyl, or $(CH_2)_{1-6}$-naphthyl.

A heterocyclyl radical or group is understood as meaning heterocyclic ring systems, saturated or unsaturated ring which contains one or more heteroatoms from the group consisting of nitrogen, oxygen and/or sulfur in the ring and can also be mono- or polysubstituted. Examples which may be mentioned from the group of heteroaryls are furan, benzofuran, thiophene, benzothiophene, pyrrole, pyridine, pyrimidine, pyrazine, quinoline, isoquinoline, phthalazine, benzo-1,2,5-thiadiazole, benzothiazole, indole, benzotriazole, benzodioxolane, benzodioxane, carbazole and quinazoline.

In the context of this invention alkyl-heterocylyl is understood as meaning a heterocyclyl group (see above) being connected to another atom through a $C_{1-6}$-alkyl group (see above), whereas the $C_{1-6}$-alkyl-group is always saturated and unsubstituted, and linear or branched. More preferably in the context of this invention alkyl-heteriocyclyl is understood as meaning one heterocyclyl group - optionally at least mono-substituted - being connected to the scaffold of a given general formula by $(CH_2)_n$ e.g. $(CH_2)_{1-6}$; i.e. an alkylene-group. Thus it can also be expressed as $(CH_2)_{1-6}$-heterocyclyl. Examples include $(CH_2)_{1-6}$-furan, $(CH_2)_{1-6}$-benzofuran, $(CH_2)_{1-6}$-thiophene, $(CH_2)_{1-6}$-benzothiophene, $(CH_2)_{1-6}$-pyrrole, $(CH_2)_{1-6}$-pyridine, $(CH_2)_{1-6}$-pyrimidine, $(CH_2)_{1-6}$-pyrazine, $(CH_2)_{1-6}$-quinoline, $(CH_2)_{1-6}$-isoquinoline, $(CH_2)_{1-6}$-phthalazine, $(CH_2)_{1-6}$-benzo-1,2,5-thiadiazole, $(CH_2)_{1-6}$-benzothiazole, $(CH_2)_{1-6}$-indole, $(CH_2)_{1-6}$-benzotriazole, $(CH_2)_{1-6}$-benzodioxolane, $(CH_2)_{1-6}$-benzodioxane, $(CH_2)_{1-6}$-carbazole and $(CH_2)_{1-6}$-quinazoline.

In connection with aryl or alkyl-aryl, cycloalkyl or alkyl-cycloalkyl, heterocyclyl or alkyl-heterocyclyl, substituted is understood - unless defined otherwise - as meaning substitution of the ring-system of the aryl or alkyl-aryl, cycloalkyl or alkyl-cycloalkyl; heterocyclyl or alkyl-heterocyclyl by OH, SH, =O, halogen (F, Cl, Br, I), CN, $NO_2$, COOH; $NR_xR_y$, with $R_x$ and $R_y$ independently being either H or a saturated or unsaturated, linear or branched, substituted or unsubstituted $C_{1-6}$-alkyl; a saturated or unsaturated, linear or branched, substituted or unsubstituted $C_{1-6}$-alkyl; a saturated or unsaturated, linear or branched, substituted or unsubstituted $-O-C_{1-6}$-alkyl (alkoxy); a saturated or unsaturated, linear or

branched, substituted or unsubstituted -S-$C_{1-6}$-alkyl; a saturated or unsaturated, linear or branched, substituted or unsubstituted - C(O)-$C_{1-6}$-alkyl-group; a saturated or unsaturated, linear or branched, substituted or unsubstituted -C(O)-O-$C_{1-6}$-alkyl-group; a substituted or unsubstituted aryl or alkyl-aryl; a substituted or unsubstituted cycloalkyl or alkyl-cycloalkyl; a substituted or unsubstituted heterocyclyl or alkyl-heterocyclyl. "Substituted" does include "mono-" (once substituted) or "polysubstituted" (more than once substituted) radicals. "Polysubstituted" being understood as meaning that the replacement takes effect both on different and on the same atoms several times with the same or different substituents. "Optionally at least monosubstituted" means either "monosubstituted", "polysubstituted" or - if the option is not fulfilled - "unsubstituted".

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic-especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids and in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The compounds of the invention may be in crystalline form or either as free compounds or as solvates and it is intended that those forms are within the scope of the present invention. Methods of solvation are generally known within the art. Suitable solvates are pharmaceutically acceptable solvates. The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

Any compound that is a prodrug of a compound of formula (I) is within the scope of the invention. The term "prodrug is used in its broadest sense and encompasses those derivatives that are converted in vivo to the compounds of the invention. Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (April 2002).

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by $^{13}C$- or $^{14}C$-enriched carbon or $^{15}N$-enriched nitrogen are within the scope of this invention.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I) or, or of its salts, solvates or prodrugs.

The term "pharmacological tool" refers to the property of compounds of the invention through which they are particularly selective ligands for Sigma receptors which implies that compound of formula (I), described in this invention, can be used as a model for testing other compounds as sigma ligands, ex. a radiactive ligands being replaced, and can also be used for modeling physiological actions related to sigma receptors.

The compounds of the present invention represented by the above described formula (I) may include enantiomers depending on the presence of chiral centres or isomers depending on the presence of multiple bonds (e.g. Z, E).

The single isomers, enantiomers or diastereoisomers and mixtures thereof fall within the scope of the present invention.

In another preferred embodiment of the invention the compound according to the invention is having a general formula Ia,

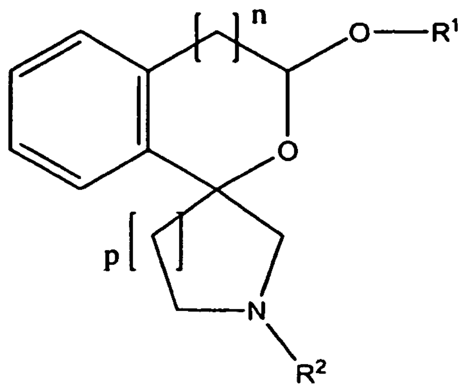

Ia

wherein

n is selected from 0 or 1;

p is selected from 1 or 2;

$R^1$ is selected from H, $C_{1-6}$-alkyl

$R^2$ is selected from hydrogen, $C_{1-18}$-alkyl, alkyl-aryl, alkyl-heteroaryl, alkyl-cycloalkyl, cycloalkyl, aryl, heterocyclyl;

or $R^2$ together with the bonded Nitrogen is binding the nitrogen in a cycloalkyl-spiro-complex thus forming a quarternary ammonium according to formula X (with q being 1 or 2):

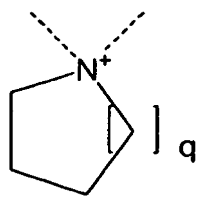

.

In another preferred embodiment of the invention the compound according to the invention is having a general formula Ia,

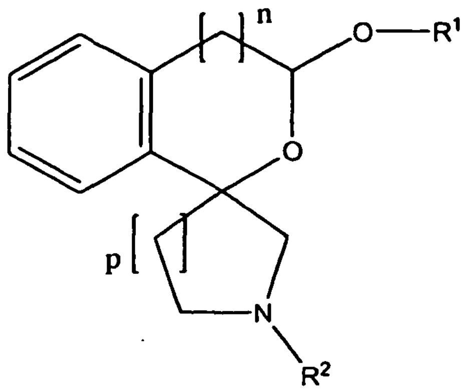

Ia

wherein

n is selected from 0 or 1;

p is selected from 1 or 2;

$R^1$ is selected from H, $C_{1-6}$-alkyl;

$R^2$ is selected from hydrogen; optionally at least mono-substituted $C_{1-18}$-aliphatic group, saturated or unsaturated, linear or branched; optionally at least mono-substituted alkyl-aryl, alkyl-heteroaryl, alkyl-cycloalkyl, cycloalkyl, aryl, or heterocyclyl;

or $R^2$ together with the bonded Nitrogen is binding the nitrogen in a cycloalkyl-spiro-complex thus forming a quaternary ammonium according to formula X (with q being 1 or 2):

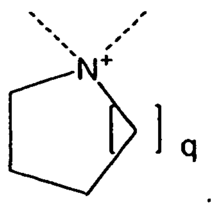

In another preferred embodiment of the invention the compound according to the invention is having a general formula II,

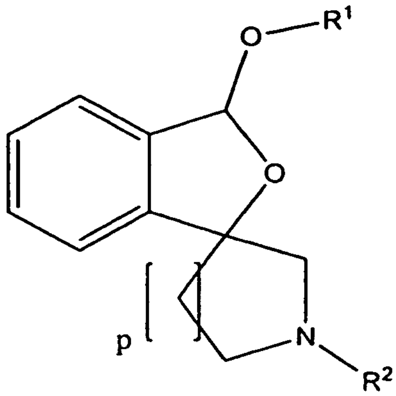

II

wherein

p is selected from 1 or 2;

$R^1$ is selected from H, $C_{1-6}$-alkyl;

$R^2$ is selected from hydrogen; optionally at least mono-substituted $C_{1-18}$-aliphatic group, saturated or unsaturated, linear or branched; optionally at least mono-substituted alkyl-aryl, alkyl-heteroaryl, alkyl-cycloalkyl, cycloalkyl, aryl, or heterocyclyl;

or $R^2$ together with the bonded Nitrogen is binding the nitrogen in a cycloalkyl-spiro-complex thus forming a quaternary ammonium according to formula X (with q being 1 or 2):

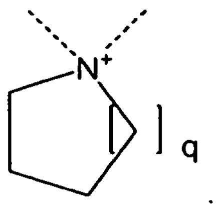

In another preferred embodiment of the invention the compound according to the invention is having a general formula III,

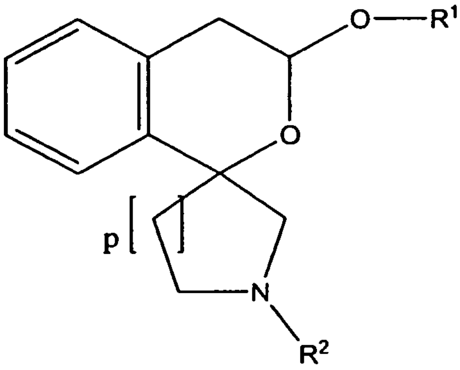

III

wherein

p is selected from 1 or 2;

$R^1$ is selected from H, $C_{1-6}$-alkyl;

$R^2$ is selected from hydrogen; optionally at least mono-substituted $C_{1-18}$-aliphatic group, saturated or unsaturated, linear or branched; optionally at least mono-substituted alkyl-aryl, alkyl-heteroaryl, alkyl-cycloalkyl, cycloalkyl, aryl, or heterocyclyl;

or $R^2$ together with the bonded Nitrogen is binding the nitrogen in a cycloalkyl-spiro-complex thus forming a quarternary ammonium according to formula X (with q being 1 or 2):

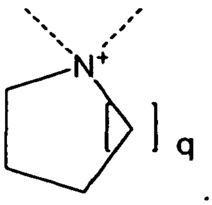

In another preferred embodiment of the invention the compound according to the invention is having any one of general formulas IIa or IIb,

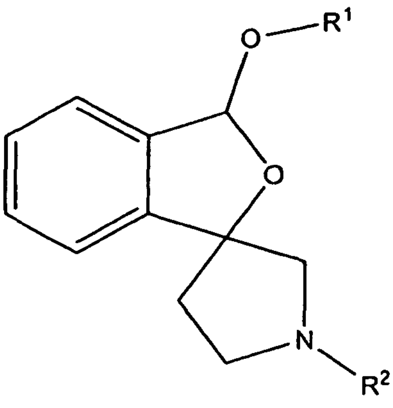

IIa

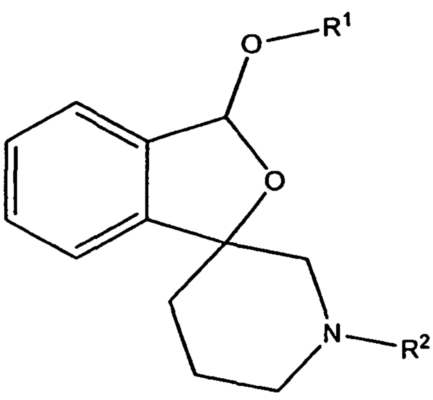

IIb

wherein

$R^1$ is selected from H, $C_{1-6}$-alkyl;

$R^2$ is selected from hydrogen; optionally at least mono-substituted $C_{1-18}$-aliphatic group, saturated or unsaturated, linear or branched; optionally at least mono-substituted alkyl-aryl, alkyl-heteroaryl, alkyl-cycloalkyl, cycloalkyl, aryl, or heterocyclyl;

or $R^2$ together with the bonded Nitrogen is binding the nitrogen in a cycloalkyl-spiro-complex thus forming a quaternary ammonium according to formula X (with q being 1 or 2):

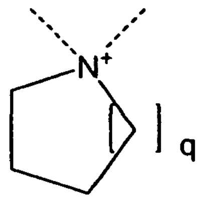

.

In another preferred embodiment of the invention the compound according to the invention is having any one of general formulas IIIa or IIIb,

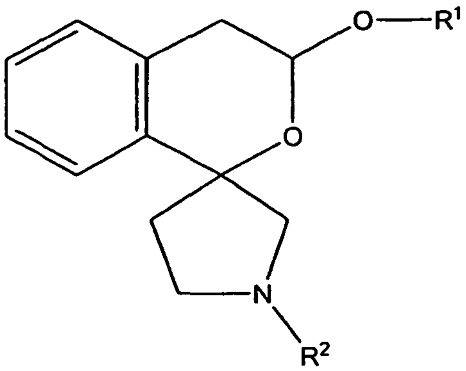

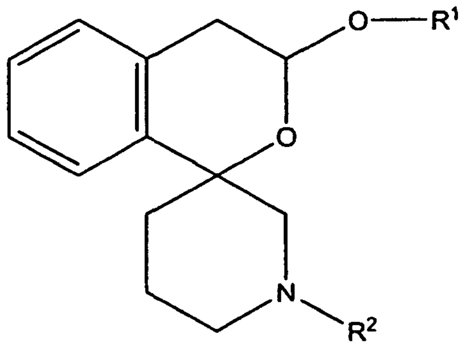

IIIa IIIb

wherein

$R^1$ is selected from H, $C_{1-6}$-alkyl;

$R^2$ is selected from hydrogen; optionally at least mono-substituted $C_{1-18}$-aliphatic group, saturated or unsaturated, linear or branched; optionally at least mono-substituted alkyl-aryl, alkyl-heteroaryl, alkyl-cycloalkyl, cycloalkyl, aryl, or heterocyclyl;

or $R^2$ together with the bonded Nitrogen is binding the nitrogen in a cycloalkyl-spiro-complex thus forming a quaternary ammonium according to formula X (with q being 1 or 2):

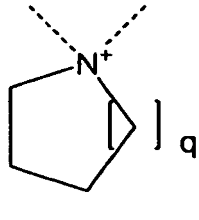

.

In another preferred embodiment of the invention the compound according to the invention is characterized in that $R^1$ is selected from H, $CH_3$ or $C_2H_5$, especially $R^1$ is selected from $CH_3$.

In another preferred embodiment of the invention the compound according to the invention is characterized in that $R^2$ is selected from

hydrogen; optionally at least mono-substituted $C_{1-18}$-aliphatic-group, saturated or unsaturated, linear or branched; optionally at least mono-substituted aryl or alkyl-aryl; optionally at least mono-substituted heteroaryl or alkyl-heteroaryl;

preferably $R^2$ is selected from hydrogen, optionally at least mono-substituted $C_{1-18}$-aliphatic-group, saturated or unsaturated, linear or branched, substituted or unsubstituted; optionally at least mono-substituted alkyl-aryl; optionally at least mono-substituted alkyl-heteroaryl.

In another preferred embodiment of the invention the compound according to the invention is characterized in that $R^2$ is selected from

hydrogen; optionally at least mono-substituted $C_{4-12}$-aliphatic-group, saturated or unsaturated, linear or branched; optionally at least mono-substituted $(CH_2)_{1-6}$-aryl, optionally at least mono-substituted $(CH_2)_{1-6}$-alkyl-heteroaryl;

preferably $R^2$ is selected from hydrogen; optionally at least mono-substituted $C_{4-10}$-aliphatic group, saturated or unsaturated, linear or branched, substituted or unsubstituted; $(CH_2)_{1-4}$-aryl, substituted or unsubstituted; $(CH_2)_{1-4}$-heteroaryl, substituted or unsubstituted;

more preferably $R^2$ is selected from hydrogen; optionally at least mono-substituted, linear $C_{4-10}$-alkyl; optionally at least mono-substituted $(CH_2)_{1-4}$-aryl; optionally at least mono-substituted $(CH_2)_{1-4}$-alkyl-heteroaryl.

In another preferred embodiment of the invention the compound according to the invention is characterized in that $R^2$ forms together with the bonded Nitrogen a cycloalkyl-spiro-complex thus forming a quaternary ammonium according to formula X (with q being 1 or 2):

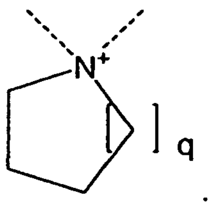

In another preferred embodiment of the invention the compound according to the invention is characterized in that the compound is selected from

1'-Benzyl-3-methoxy-3,4-dihydrospiro[[2]benzopyran-1,3'-piperidin] (1) (2)

3-Methoxy-1'-(2-phenylethyl)-3,4-dihydrospiro[[2]benzopyran-1,3'-piperidin] (4) (5)

3-Methoxy-1'-(3-phenylpropyl)-3,4-dihydrospiro[[2]benzopyran-1,3'-piperidin] (6) (7)

3-Methoxy-1'-(4-phenylbutyl)-3,4-dihydrospiro[[2]benzopyran-1,3'-piperidin] (8) (9)

1'-Butyl-3-methoxy-3,4-dihydrospiro[[2]benzopyran-1,3'-piperidin] (10) (11)

1'-Hexyl-3-methoxy-3,4-dihydrospiro[[2]benzopyran-1,3'-piperidin] (12) (13)

3-Methoxy-1'-octyl-3,4-dihydrospiro[[2]benzopyran-1,3'-piperidin] (14) (15)

1'-Decyl-3-methoxy-3,4-dihydrospiro[[2]benzopyran-1,3'-piperidin] (16) (17)

1'-Benzyl-3-methoxy-3,4-dihydrospiro[[2]benzopyran-1,3'-pyrrolidin] (18)

3-Methoxy-1'-(2-phenylethyl)-3,4-dihydrospiro[[2]benzopyran-1,3'-pyrrolidin] (20)

3-Methoxy-1'-(4-phenylbutyl)-3,4-dihydrospiro[[2]benzopyran-1,3'-pyrrolidin] (21)

1'-Buty1-3-methoxy-3,4-dihydrospiro[[2]benzopyran-1,3'-pyrrolidin] (22)

3-Methoxy-1'-octyl-3,4-dihydrospiro[[2]benzopyran-1,3'-pyrrolidin] (23)

1'-Benzyl-3-methoxy-3*H*-spiro[[2]benzofuran-1,3'-pyrrolidin] (25)

3-Methoxy-1'-(2-Phenylethyl)-3*H*-spiro [[2]benzofuran-1,3'-pyrrolidin] (27)

3-Methoxy-1'-(4-Phenylbutyl)-3*H*-spiro [[2]benzofuran-1,3'-pyrrolidin] (28)

1'-Butyl-3-methoxy-3*H*-spiro[[2]benzofuran-1,3'-pyrrolidin] (29)

3-Methoxy-1'-octyl-3*H*-spiro[[2]benzofuran-1,3'-pyrrolidin] (30)

N,N-Cyclobutyl-3-methoxy-3*H*-spiro[[2]benzofuran-1,3'-pyrrolidiniumbromid] (31) or

1'-(4-(1*H*-Imidazol-1-yl)-butyl)-3-methoxy-3*H*-spiro[[2]benzofuran-1,3'-pyrrolidin] (34);

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another non-claimed embodiment of the invention the compound according to the invention is having a general formula IV.

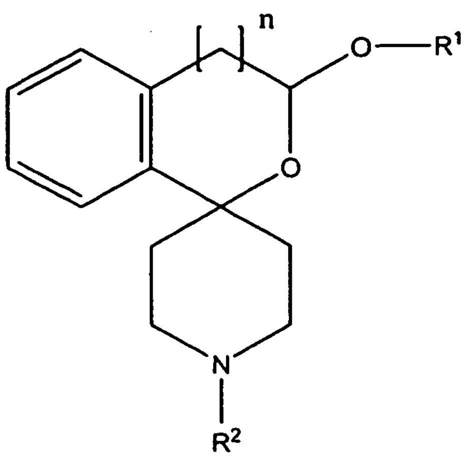

IV

wherein

n is selected from 0 or 1;

$R^1$ is selected from H, $C_{1-6}$-alkyl;

$R^2$ is selected from hydrogen; optionally at least mono-substituted $C_{1-18}$-aliphatic group; optionally at least mono-substituted alkyl-aryl, alkyl-heteroaryl, alkyl-cycloalkyl, cycloalkyl, aryl, or heterocyclyl;

or

$R^2$ together with the bonded Nitrogen is binding the nitrogen in a cycloalkyl-spiro-complex thus forming a quarternary ammonium according to formula X (with q being 1 or 2):

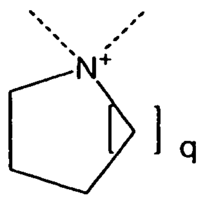

.

In another non-claimed embodiment of the invention according to general formula IV the compound according to the invention is having any one of general formulas IVa or IVb,

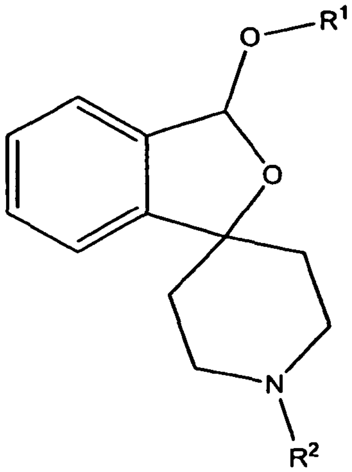

IVa

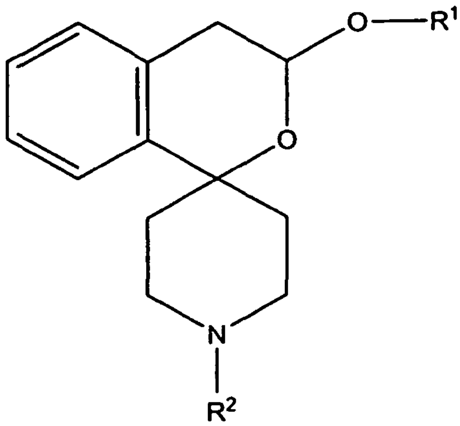

IVb

wherein

$R^1$ is selected from H, $C_{1-6}$-alkyl;

$R^2$ is selected from hydrogen; optionally at least mono-substituted $C_{1-18}$-aliphatic group; optionally at least mono-substituted alkyl-aryl, alkyl-heteroaryl, alkyl-cycloalkyl, cycloalkyl, aryl, or heterocyclyl.

In another non-claimed embodiment of the invention the compound according to the invention according to general formula IV is characterized in that $R^1$ is selected from H, $CH_3$ or $C_2H_5$, especially $R^1$ is selected from $CH_3$.

In another non-claimed embodiment of the invention according to general formula IV the compound according to the invention is characterized in that $R^2$ is selected from

hydrogen; optionally at least monosubstituted $C_{1-18}$-aliphatic group saturated or unsaturated, linear or branched; optionally at least monosubstituted aryl or alkyl-aryl; optionally at least monosubstituted heteroaryl or alkyl-heteroaryl;

preferably $R^2$ is selected from optionally at least monosubstituted $C_{4-12}$-aliphatic group, saturated or unsaturated, linear or branched.

In another non-claimed embodiment of the invention according to general formula IV the compound according to the invention is characterized in that $R^2$ is selected from

hydrogen; optionally at least monosubstituted $C_{1-18}$-aliphatic group, saturated or unsaturated, linear or branched; optionally at least monosubstituted aryl or alkyl-aryl; optionally at least monosubstituted heteroaryl or alkyl-heteroaryl.

In another non-claimed embodiment of the invention according to general formula IV the compound according to the invention is characterized in that $R^2$ is selected selected from $C_{4-12}$-alkyl, saturated or unsaturated, linear or branched, substituted or unsubstituted, preferably $C_{4-10}$-alkyl, saturated or unsaturated, linear or branched, substituted or unsubstituted, most preferably $C_{4-10}$-alkyl, saturated, linear, substituted or unsubstituted.

In another non-claimed embodiment of the invention according to general formula IV the compound according to the invention is characterized in that the compound is selected from

N,N-Cyclobutyl-3-methoxy-3*H*-spiro[[2]benzofuran-1,4'-piperidiniumbromid] (32),

1'-(4-(1H-imidazol-1-yl)-butyl)-3-methoxy-3H-spiro[[2]benzofuran-1,4'-piperidin] (35); or

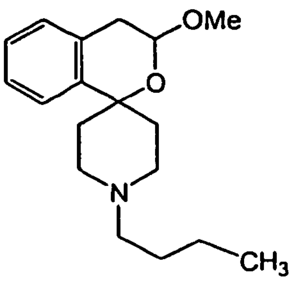 or 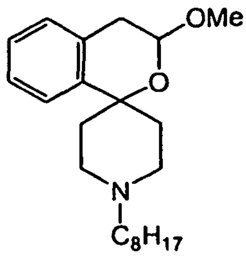 ;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In general the processes are described below in the experimental part. The starting materials are commercially available or can be prepared by conventional methods.

The obtained reaction products may, if desired, be purified by conventional methods, such as crystallisation and chromatography. Where the above described processes for the preparation of compounds of the invention give rise to mixtures of stereoisomers, these isomers may be separated by conventional techniques such as preparative chromatography. If there are chiral centers the compounds may be prepared in racemic form, or individual enantiomers may be prepared either by enantiospecific synthesis or by resolution.

One preferred pharmaceutically acceptable form is the crystalline form, including such form in pharmaceutical composition. In the case of salts and solvates the additional ionic and solvent moieties must also be non-toxic. The compounds of the invention may present different polymorphic forms, it is intended that the invention encompasses all such forms.

Another non-claimed embodiment of the invention refers to a group of compounds according to general formula V (encompassing the compounds of formula I)

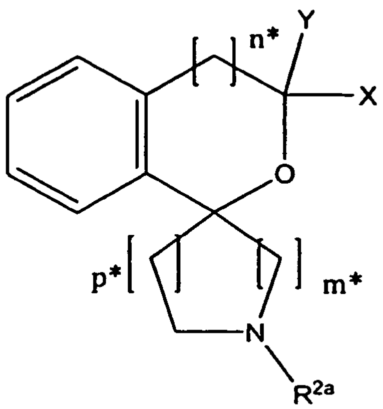

V

wherein

m* is selected from 1 or 2, p* is selected from 1 or 2, and m* + p* is either 2 or 3;

n* is selected from 0 or 1;

X is H, while Y is selected from CN; H; $C_{1-6}$-alkyl, linear or branched, substituted or unsubstituted; alkyl-aryl, substituted or unsubstituted; $C_{1-6}$-alkyl-C(O)-OH, $C_{1-6}$-alkyl-C(O)H, C(O)-O-$C_{1-6}$-alkyl or $C_{1-6}$-alkyl-C(O)-O-$C_{1-6}$-alkyl, linear or branched, substituted or unsubstituted; $C_{1-6}$-alkyl-C(O)-$NH_2$ or $C_{1-6}$-alkyl-C(O)-NH-$C_{1-6}$-alkyl, linear or branched, substituted or unsubstituted; $C_{1-6}$-alkyl-$NH_2$ or $C_{1-6}$-alkyl-NH-$C_{1-6}$-alkyl, linear or branched, substituted or unsubstituted; $C_{1-6}$-alkyl-CN, linear or branched, substituted or unsubstituted; or O-$R^{1a}$,

with $R^{1a}$ being selected from H; $C_{1-6}$-alkyl, linear or branched, substituted or unsubstituted; alkyl-aryl, substituted or unsubstituted;

or

X and Y together form =O;

$R^{2a}$ is selected from hydrogen, $C_{1-18}$-alkyl, linear or branched, substituted or unsubstituted; aryl or alkyl-aryl, substituted or unsubstituted; heteroaryl or alkyl-heteroaryl, substituted or unsubstituted; cycloalkyl or alkyl-cycloalkyl, substituted or unsubstituted;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

The production process of compounds according to general formula V that go beyond formula I, especially in regards to X and Y, can be derived analogously from Maier et Wünsch; J.Med.Chem. 2002, 45, 438-448 or Maier et Wünsch; J.Med.Chem. 2002, 45, 4923-4930, included here completely by reference.

Another non-claimed embodimentof the invention are compounds according to general formula V)

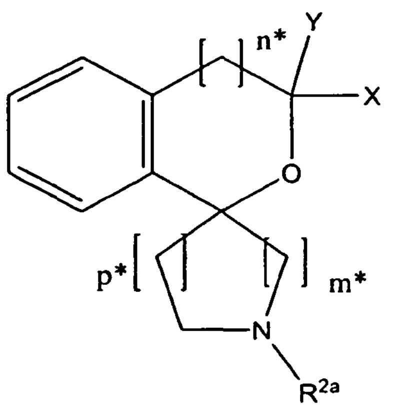

V

wherein

m* is selected from 1 or 2, p* is selected from 1 or 2, and m* + p* is either 2 or 3;

n* is selected from 0 or 1;

X is H, while Y is selected from CN; H; $C_{1-6}$-aliphatic-group, saturated or unsaturated, linear or branched, substituted or unsubstituted; optionally at least mono-substituted alkyl-aryl; $C_{1-6}$-aliphatic-group-C(O)-OH, $C_{1-6}$-aliphatic-group-C(O)H, C(O)-O-$C_{1-6}$-aliphatic-group or $C_{1-6}$-aliphatic-group-C(O)-O-$C_{1-6}$-aliphatic-group, saturated or unsaturated, linear or branched, substituted or unsubstituted; $C_{1-6}$-aliphatic-group-C(O)-$NH_2$ or $C_{1-6}$-aliphatic-group-C(O)-NH-$C_{1-6}$-aliphatic-group, saturated or unsaturated, linear or branched, substituted or unsubstituted; $C_{1-6}$-aliphatic-group-$NH_2$ or $C_{1-6}$-aliphatic-group-NH-$C_{1-6}$-aliphatic-group, saturated or unsaturated, linear or branched, substituted or

unsubstituted; $C_{1-6}$-aliphatic-group-CN, saturated or unsaturated, linear or branched, substituted or unsubstituted; or O-$R^{1a}$,

with $R^{1a}$ being selected from H; $C_{1-6}$-aliphatic-group, saturated or unsaturated, linear or branched, substituted or unsubstituted; optionally at least monosubstituted alkyl-aryl;

or

X and Y together form =O;

$R^{2a}$ is selected from hydrogen, $C_{1-18}$-aliphatic-group, saturated or unsaturated, linear or branched, substituted or unsubstituted; optionally at least mono-substituted alkyl-aryl aryl or alkyl-aryl; optionally at least mono-substituted heteroaryl or alkyl-heteroaryl; optionally at least mono-substituted cycloalkyl or alkyl-cycloalkyl, substituted or unsubstituted;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

A non-claimed embodiment of the invention are compounds according to general formula V

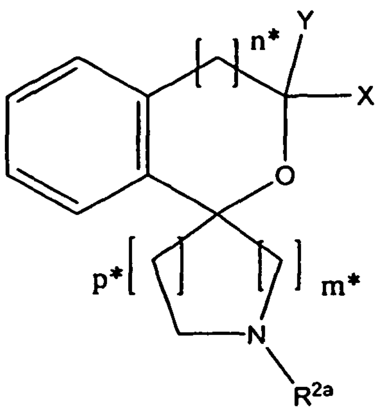

V

wherein

m* is 1, p* is selected from 1 or 2, and m* + p* is either 2 or 3;

n* is selected from 0 or 1;

X is H, while Y is selected from CN; H; $C_{1-6}$-aliphatic-group, saturated or unsaturated, linear or branched, substituted or unsubstituted; optionally at least mono-substituted alkyl-aryl; $C_{1-6}$-aliphatic-group-C(O)-OH, $C_{1-6}$-aliphatic-group-C(O)H, C(O)-O-$C_{1-6}$-aliphatic-group or $C_{1-6}$-aliphatic-group-C(O)-O-$C_{1-6}$-aliphatic-group, saturated or unsaturated, linear or branched, substituted or unsubstituted; $C_{1-6}$-aliphatic-group-C(O)-$NH_2$ or $C_{1-6}$-aliphatic-group-C(O)-NH-$C_{1-6}$-aliphatic-group, saturated or unsaturated, linear or branched, substituted or unsubstituted; $C_{1-6}$-aliphatic-group-$NH_2$ or $C_{1-6}$-aliphatic-group-NH-$C_{1-6}$-aliphatic-group, saturated or unsaturated, linear or branched, substituted or unsubstituted; $C_{1-6}$-aliphatic-group-CN, saturated or unsaturated, linear or branched, substituted or unsubstituted; or O-$R^{1a}$,

with $R^{1a}$ being selected from H; $C_{1-6}$-aliphatic-group, saturated or unsaturated, linear or branched, substituted or unsubstituted; optionally at least monosubstituted alkyl-aryl;

or

X and Y together form =O;

$R^{2a}$ is selected from hydrogen, $C_{1-18}$-aliphatic-group, saturated or unsaturated, linear or branched, substituted or unsubstituted; optionally at least mono-substituted alkyl-aryl aryl or alkyl-aryl; optionally at least mono-substituted heteroaryl or alkyl-heteroaryl; optionally at least mono-substituted cycloalkyl or alkyl-cycloalkyl, substituted or unsubstituted;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

Preferably these non-claimed compounds according to formula V are described by formula VI

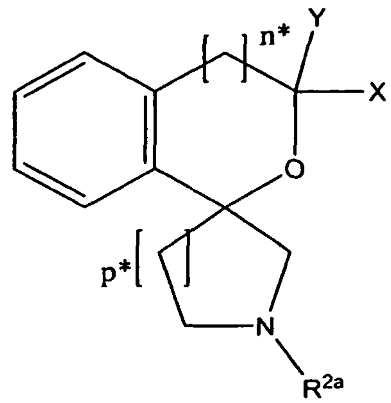

VI,

wherein n*, p*, X, Y and $R^{2a}$ have the same meaning as described above.

Another aspect of the invention refers to a pharmaceutical composition which comprises a compound according to the invention or a pharmaceutically acceptable salt, prodrug, isomer or solvate thereof, and a pharmaceutically acceptable carrier, adjuvant or vehicle. The present invention thus provides pharmaceutical compositions comprising a compound of this invention, or a pharmaceutically acceptable salt, derivative, prodrug or stereoisomers thereof together with a pharmaceutically acceptable carrier, adjuvant, or vehicle, for administration to a patient.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form, either solid or liquid. Suitable dose forms for oral administration may be tablets, capsules, syrops or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the apropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of the diseases to be treated.

Generally an effective administered amount of a compound of the invention will depend on the relative efficacy of the compound chosen, the severity of the disorder being treated and the weight of the sufferer. However, active

compounds will typically be administered once or more times a day for example 1, 2, 3 or 4 times daily, with typical total daily doses in the range of from 0.1 to 1000 mg/kg/day.

The compounds and compositions of this invention may be used with other drugs to provide a combination therapy. The other drugs may form part of the same composition, or be provided as a separate composition for administration at the same time or at different time.

Another aspect of the invention refers to the use of a compound according to the invention in the manufacture of a medicament.

Another aspect of the invention refers to the use of a compound according to the invention in the manufacture of a medicament for the treatment or prophylaxis of a sigma receptor mediated disease or condition. A preferred embodiment of this is this use wherein the disease is diarrhoea, lipoprotein disorders, metabolic syndrome, treatment of elevated triglyceride levels, chylomicronemia, hyperlipoproteinemia; hyperlipidemia, especially mixed hyperlipidemia; hypercholesterolemia, dysbetalipoproteinemia, hypertriglyceridemia including both the sporadic and familial disorder (inherited hypertriglyceridemia), migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine, tardive diskinesia, ischemic stroke, epilepsy, stroke, depression, stress, psychotic condition, schizophrenia; inflammation, autoimmune diseases or cancer.

A preferred embodiment of this is this use wherein the disease is pain, especially neuropathic pain, inflammatory pain or other pain conditions, allodynia and/or hyperalgesia, especially mechanical allodynia.

Another aspect of the invention refers to the use of a compound according o the invention as pharmacological tool or as anxiolytic or immunosuppressant.

The term "pharmacological tool" refers to the property of compounds of the invention through which they are particularly selective ligands for Sigma receptors which implies that compound of formula I, described in this invention, can be used as a model for testing other compounds as Sigma ligands, ex. a radiactive ligands being replaced, and can also be used for modeling physiological actions related to Sigma receptors.

Another aspect of this invention relates to a method of treating or preventing a sigma receptor mediated disease which method comprises administering to a patient in need of such a treatment a therapeutically effective amount of a compound as above defined or a pharmaceutical composition thereof. Among the sigma mediated diseases that can be treated are diarrhoea, lipoprotein disorders, metabolic syndrome, treatment of elevated triglyceride levels, chylomicronemia, hyperlipoproteinemia; hyperlipidemia, especially mixed hyperlipidemia; hypercholesterolemia, dysbetalipoproteinemia, hypertriglyceridemia including both the sporadic and familial disorder (inherited hypertriglyceridemia), migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine, tardive diskinesia, ischemic stroke, epilepsy, stroke, depression, stress, pain, especially neuropathic pain, inflammatory pain or other pain conditions, allodynia and/or hyperalgesia, especially mechanical allodynia, psychotic condition, schizophrenia; inflammation, autoimmune diseases or cancer; disorders of food ingestion, the regulation of appetite, for the reduction, increase or maintenance of body weight, for the prophylaxis and/or treatment of obesity, bulimia, anorexia, cachexia or type II diabetes, preferably type II diabetes caused by obesity. The compounds of the invention can also be employed as pharmacological tool or as anxiolytic or immunosuppressant.

## EXAMPLES:

### General Experimental Part (Methods and Equipment of the synthesis and analysis

All solvents used for synthesis were p. a. quality.

The separation of mixtures of substances using thin-layer chromatography was done on glass plates layered by silica gel. Analysis of the plates was done after treatment with iodine gas or incubation with Dragendorff's reagent under UV light.

As a rule synthesized products were purified using flash-chromatography.

Melting points were measured using capillaries. As sometimes the products were mixtures of diastereoisomers, the melting point had to be expressed as a range.

IR-spectra were measured using the FT-IR-480 Plus Fourier Transform Spectrometer with ATR (Fa. Jasco). All substances were either measured directly as solids or in oil.

NMR-Spectra were measured using Mercury-400BB (Fa. Varian) at a temperature of 21 °C. $\sigma$, measured in ppm, is based on the signal TMS measured in comparison to the residue signal ($CHCl_3$) of the solvent ($CDCl_3$):

$^1$H-NMR-Spectroscopy:

$$\delta\ (TMS) = \delta\ (CHCl_3) - 7.26$$

$^{13}$C-NMR-Spectroscopy:

$$\delta\ (TMS) = \delta\ (CHCl_3) - 77.0$$

Mass-spectra (MS) were measured using the GCQ Finnigan MAT (Fa. Finnigan) with Xcalibur Version 1.1. The method of ionisation is shown in brackets: EI = electronic ionisation (70 eV); CI = Chemical ionisation (Isobutane or $NH_3$, 170 eV).

Elementary analysis was done with VarioEL (Fa. Elementar).

Before using HPLC the maximum absorption of the substances was measured using the UV/Vis-Spectra done with a 50Bio Cary Spektrophotometer (Fa. Varian).

For determination of the purity and for the separation of the diastereomers a HPLC Hitachi L6200A Intelligent Pump with a UV-Detector (Merck) was used.

Synthesis was done in the synthetic microwave Discover (Fa. CEM).

Some reactions were done using protective gas.

Reactions at -78°C were done in an acetone bath in a Dewar.

**Example 1:**

**Synthesis of *cis*-1'-Benzyl-3-methoxy-3,4-dihydrospiro[[2]benzopyran-1,3'-piperidin]**

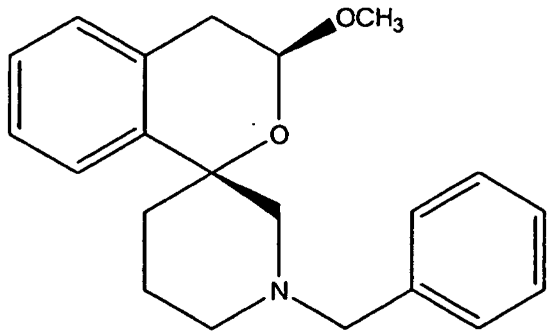

Certrichloride (138 mg, 0.56 mmol) was suspended in 3 mL THF under nitrogen and stirred for 2 h at -78°C. 2-(2-Bromphenyl)acetaldehyddimethylacetal (130 mg, 0.53 mmol) was dissolved in THF (3 mL) and cooled to -78°C. To this solution 0.34 mL n-butyl-lithium, 1.6 M in hexane (0.55 mmol) was slowly added and stirred 1 h at -78°C. The acetal solution together with 2ml THF to remove any residues was added to the Cer-suspension. After 1 h stirring at -78°C 1-benzylpiperidine-3-one (94.6 mg, 0.50 mmol) dissolved in THF (2 mL) was added an stirred for further 2 h at -78°C. After finishing the reaction saturated ammonium chloride was added and the reaction mixture was extracted for 6 times using diethyl ether. The ether phases were washed 2 times with $H_2O$. Then, the organic phase was extracted six times with 5%-HCl-solution and the HCl-phase washed twice with ether. To the $H_2O$-Phase solid KOH was slowly added until a pH>9 was reached. Following that, the $H_2O$-Phase was extracted six times with diethyl ether. The organic phase was washed twice with $H_2O$. Following that the phase was dried over $Na_2SO_4$, filtered and reduced under vacuum. The product was purified by flash-chromatography whereby the diastereomeres were also seperated (2 cm, 5 mL, Cyclohexan: Ethylacetat = 7:3, **(1)**: $R_f$ = 0.38, (2): $R_f$ = 0.20). Yield 46.9 mg (29 %).

(**Example 1**): slightly yellow oil

$C_{21}H_{25}NO_2$ (323.4)

E: Ber.: C 77.99; H 7.79; N 4.33
Gef.: C 77.89; H 8.06; N 4,04.

MS (EI): m/z (%) = 323 [$M^+$], 292 [$M^+$ -$OCH_3$], 263 [$M^+$ -$OCHOCH_3$].

IR: $\tilde{v}$ ($cm^{-1}$) = 2923 (ν C-H); 1451 (δ -$CH_2$-); 1385 (δ -$CH_3$); 1076, 1034 (ν CO); 753, 697 (δ C-H monosubst. Benzene).

$^1$H-NMR ($CDCl_3$): δ (ppm) = 1.60 - 1.66 (m, 2 H, $CH_2CH_2CH_2N$), 1.81 - 1.87 (m, 1 H, $CH_2CH_2CH_2N$), 1.92 - 1.98 (m, 1 H, $CH_2CH_2CH_2N$), 2.00 - 2.04 (m, 1 H, $CH_2CH_2CH_2N$), 2.10 - 2.15 (m, 1 H, $(CH_2)_3NCH_2$), 2.85 - 3.06 (m, 4 H, $CH_2CH_2CH_2N$ (1 H), $(CH_2)_3NCH_2$ (1 H), $ArCH_2CH$ (2 H)), 3.99 - 3.72 (dd, J = 112.1/13.3 Hz, 2 H, $NCH_2Ph$), 4.79 (t, J = 3.9 Hz, $ArCH_2CH$), 7.05 - 7.33 (m, 9 H, aromat. H).

**<u>Example 2:</u>**

**Synthesis of *trans*-1'-Benzyl-3-methoxy-3,4-dihydrospiro[[2]benzopyran-1,3'-piperidin]**

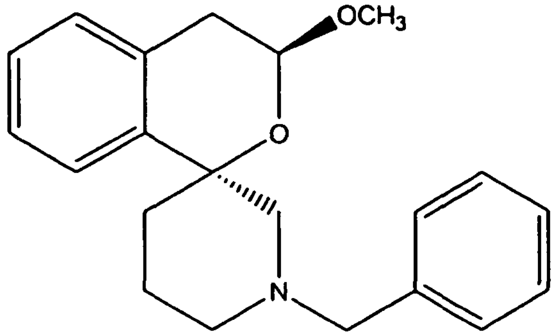

Done as in example 1 with this diastereomer being separated in the HPLC.

Example 2: slightly yellow oil

E: Ber.: C 77.99; H 7.79; N 4.33
Gef.: C 77.73; H 8.04; N 4.10.

MS (EI): m/z (%) = 323 [$M^+$], 292 [$M^+$ -$OCH_3$], 263 [$M^+$ -$OCHOCH_3$].

IR: $\tilde{v}$ ($cm^{-1}$) = 2921 (ν C-H); 1452 (δ -$CH_2$-); 1385 (δ -$CH_3$); 1076 (ν C-O); 754, 690 (δ C-H monosubst. Benzene).

$^1$H-NMR ($CDCl_3$): δ (ppm) = 1.55 - 1.67 (m, 2 H, $CH_2CH_2CH_2N$), 1.91 - 2.18 (m, 3 H, $CH_2CH_2CH_2N$ (2 H), $(CH_2)_2CH_2N$ (1 H)), 2.44 - 2.48 (m, 1 H, $(CH_2)_3NCH_2$), 2.83 - 3.04 (m, 4 H, $(CH_2)_2CH_2N$ (1 H), $(CH_2)_3NCH_2$ (1 H), $ArCH_2CH$ (2 H)), 3.44 (s, 3 H, $CHOCH_3$), 3.58 (dd, J = 32.3/13.2 Hz, 2 H, $NCH_2Ph$), 4.98 (t, J = 3.5 Hz, 1 H, $ArCH_2CH$), 7.07 - 7.39 (m, 9 H, aromat. H).

**<u>Example 3:</u>**

**Synthesis of *cis- and trans*-3-Methoxy-3,4-dihydrospiro[[2]benzopyran-1,3'-piperidin]**

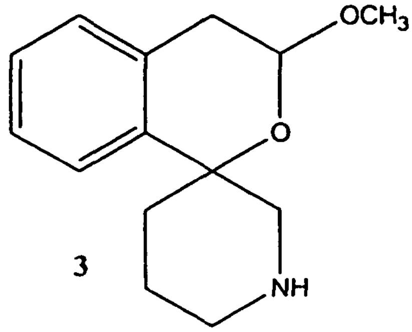

3

The mixture of coumpounds **(1/2)** (734 mg, 2.27 mmol) was dissolved in 12 mL methanol under nitrogen atmosphere. Dried ammonium formiate (716 mg, 11.3 mmol) and palladium on charcoal (145 mg, 10 %) were added in dry form. The nixture was heated under reflux for 2 h and filtered. The solvent was removed in vacuuo and the crude product purified with Flash-Chromatography (3 cm, 20 mL, methanol:ammonia = 98:2, $R_f$ = 0.69).

White solid, Mp. 54 - 58°C, yield 480 mg (91 %).

$C_{14}H_{19}NO_2$ (233.3)

| | |
|---|---|
| MS (EI): | m/z (%) = 233 [$M^+$], 202 [$M^+$ -$OCH_3$], 190 [$M^+$ -$NH(CH_2)_2$]. |
| IR: | $\tilde{\nu}$ ($cm^{-1}$) = 2933 (ν C-H); 1442 (δ -$CH_2$-); 1386 (δ -$CH_3$); 1075, 1055 (ν CO); 752 (δ C-H). |
| $^1$H-NMR ($CDCl_3$): | δ (ppm) = 1.54 - 1.58 (m, 1 H, $CH_2CH_2CH_2N$), 1.79 - 2.04 (m, 4 H, $CH_2CH_2CH_2N$ (1 H), $CH_2(CH_2)_2N$ (2 H), $(CH_2)_2CH_2N$ (1 H)), 2.15 - 2.19 (m, 1 H, $(CH_2)_2CH_2N$), 2.66 - 2.78 (m, 1 H, $(CH_2)_2CH_2N$), 2.86 - 2.98 (m, 2 H, $(CH_2)_2CH_2N$, $(CH_2)_2CH_2N$), 3.05 - 3.16 (m, 1 H, $(CH_2)_2CH_2N$), 3.55/3.60 (2 s, together 3 H, $CHOCH_3$ (cis, trans)), 4.87 - 4.96 (2 t, J = 3.3 Hz, together 1 H, $ArCH_2CH$), 7.09 - 7.26 (m, 4 H, aromat. H). |

__Example 4:__

**Synthesis of *cis*-3-Methoxy-1'-(2-phenylethyl)-3,4-dihydrospiro[[2]benzopyran-1,3'-piperidin]**

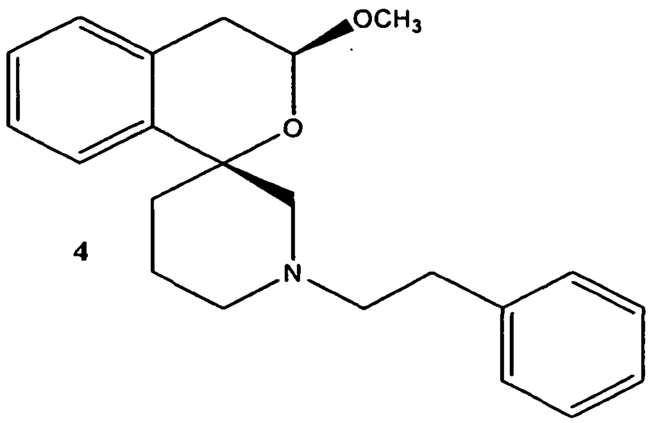

4

**Method 1**

1-Chloro-2-phenylethane (93.2 mg, 0.66 mmol) and $K_2CO_3$(334 mg, 2.42 mmol) were added to a solution of compound **(3)** (96.2 mg, 0.41 mmol) in THF (5 mL). The mixture was heated for 19 h under reflux and then filtered. The solvent was removed in vacuo and the crude product purified with Flash-Chromatography (1 cm, 5 mL, Cyclohexane:

Ethyl acetate = 7:3, **(4)**: $R_f$ = 0.36, **(5)**: $R_f$ = 0.30).

Slightly yellow oil, total yield 49 mg (35 %).

**Method 2**

Compound **(3)** (187 mg, 0.80 mmol), 1-Chloro-2-phenylethane (141 mg, 1.00 mmol) and $K_2CO_3$ (663 g, 4.80 mmol) were dissolved in acetonitril (3 mL) and treated with microwave (40 min; 220 Watt, 4 bar, 100°C). Then the mixture was filtered, the solvent was removed in vacuo and the crude product purified with Flash-Chromatography seperating the diastereomers (2 cm, 10 mL, Cyclohexane:Ethyl acetate = 7:3, **(4)**: $R_f$ = 0.36, **(5)**: $R_f$ = 0.30). Yield 125 mg (46 %).
**(4)**: slightly yellow oil

$C_2H_{27}/NO_2$ (337.5)

| | |
|---|---|
| E: | Ber.: C 78.30; H 8.06; N 4.15<br>Gef.: C 78.18; H 8.37; N 4.08. |
| MS (EI): | m/z (%) = 306 [$M^+$ -$OCH_3$], 246 [$M^+$ -$CH_2$Ph]. |
| IR: | $\tilde{v}$ ($cm^{-1}$) = 2924 (ν C-H); 1452 (δ -$CH_2$-); 1385 (δ -$CH_3$); 1076,1034 (ν CO); 751, 697 (δ C-H monosubst. Benzene). |
| $^1$H-NMR ($CDCl_3$): | δ (ppm) = 1.68 - 1.75 (d, J = 13.3 Hz, 1 H, $CH_2CH_2CH_2N$), 1.80 - 1.89 (m, 1 H, $CH_2CH_2CH_2N$), 1.97 - 2.06 (t, J = 13.3 Hz, 1 H, $CH_2CH_2CH_2N$), 2.22 (m, 3 H, $CH_2CH_2CH_2N$ (1 H), $CH_2CH_2CH_2N$ (2 H)), 2.52 - 2.63 (m, 1 H, $NCH_2CH_2Ph$), 2.70 - 2.79 (m, 1 H, $NCH_2CH_2Ph$), 2.80 - 2.87 (m, 2 H, $NCH_2CH_2Ph$), 2.86 - 2.93 (m, 1 H, $ArCH_2CH$), 3.05 - 3.10 (m, 3 H, $ArCH_2CH$ (1 H), $(CH_2)_3NCH_2$ (2 H)), 3.46 (s, 3 H, $OCH_3$), 5.12 (t, J = 3.52 Hz, 1 H, $ArCH_2CH$), 7.13 - 7.29 (m, 9 H, aromat. H). |

__Example 5:__

Synthesis of *trans*-3-Methoxy-1'-(2-phenylethyl)-3,4- dihydrospiro [[2]benzopyran-1,3'-piperidin]

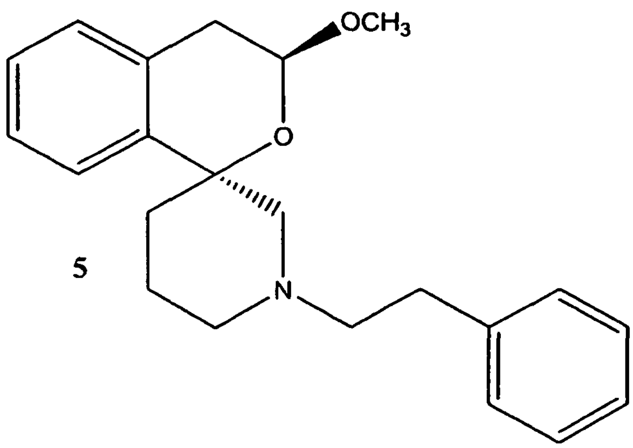

5

See example 4 above.
**(5)**: slightly yellow oil

$C_{22}H_{27}NO_2$ (337.5)

E: Ber.: C 78.30; H 8.06; N 4.15
Gef.: C 78.55; H 8.00; N 3.84.

MS (EI): m/z (%) = 306 [$M^+$ -$OCH_3$], 246 [$M^+$ -$CH_2Ph$].

IR: $\tilde{v}$ ($cm^{-1}$) = 2923 (v C-H); 1452 (δ -$CH_2$-); 1077 (v C-O); 753, 698 (δ C-H monosubst. Benzene).

$^1$H-NMR ($CDCl_3$): δ (ppm) = 1.53 - 1.74 (m, 4 H, $CH_2CH_2CH_2N$), 1.90 - 2.17 (m, 2 H, $CH_2CH_2CH_2N$), 2.21 - 2.26 (m, 1 H, $NCH_2CH_2Ph$), 2.43 - 2.52 (m, 1 H, $NCH_2CH_2Ph$), 2.60 - 2.69 (m, 1 H, $NCH_2CH_2Ph$), 2.77 - 2.99 (m, 3 H, $ArCH_2CH$ (2 H), $NCH_2CH_2Ph$ (1 H)), 3.00 - 3.13 (m, 2 H, $(CH_2)_3NCH_2$), 3.50 (s, 3 H, $OCH_3$), 4.93 (s, 1 H, $ArCH_2CH$), 7.01 - 7.27 (m, 9 H, aromat. H).

**Example 6:**

**Synthesis of *cis* -3-Methoxy-1'-(3-phenylpropyl)-3,4-dihydrospiro[[2]benzopyran-1,3'-piperidin]**

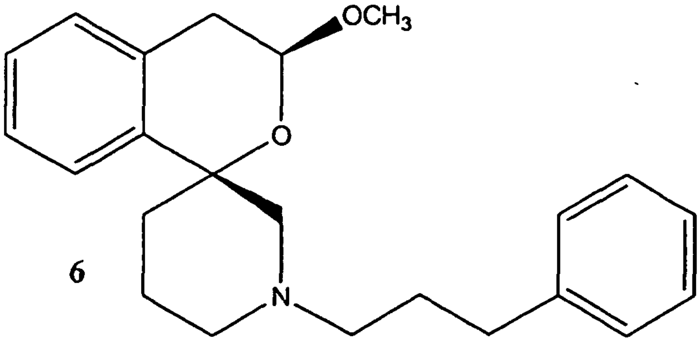

6

Compound **(3)** (135 mg, 0.58 mmol) was dissolved in acetonitril (12 mL). 1-Bromo-3-phenylpropane (126 mg, 0.63 mmol) and $K_2CO_3$ (554 mg, 4.01 mmol) were added, and the mixture was heated under reflux for 113 h. The nixture was filtered and the solvent completely removed. The crude product was purified with Flash-Chromatography seperating the diastereomers (2 cm, 10 mL, Petrolether:Ethylacetat = 8:2, **(6)**: $R_f$ = 0.31, **(7)**: $R_f$ = 0.23). Total yield 166 mg (82 %). **(6)**: slightly yellow oil

$C_{23}H_{29}NO_2$ (351.5)

E: Ber.: C 78.59; H 8.32; N 3.99
Gef.: C 78.10; H 8.39; N 3.75.

MS (EI): m/z (%) = 351 [$M^+$], 320 [$M^+$ -$OCH_3$], 246 [$M^+$ -$(CH_2)_2Ph$].

IR: $\tilde{v}$ ($cm^{-1}$) = 2943 (v C-H); 1450 (δ -$CH_2$-); 1382 (δ -$CH_3$); 1077, 1033 (v CO); 747, 700 (δ C-H monosubst. Benzene).

$^1$H-NMR ($CDCl_3$): δ (ppm) = 1.52 - 1.63 (m, 1 H, $CH_2CH_2CH_2N$), 1.72 - 1.76 (m, 3 H, $CH_2CH_2CH_2N$ (1 H), $CH_2CH_2Ph$ (2 H)), 1.85 - 2.2 (m, 3 H, $CH_2CH_2CH_2N$), 2.08 - 2.17 (m, 1 H, $CH_2CH_2CH_2N$), 2.21 - 2.28 (m, 1 H, $NCH_2(CH_2)_2Ph$), 2.37 - 2.45 (m, 1 H, $NCH_2(CH_2)_2Ph$), 2.53 (t, J = 7.82 Hz, 2 H, $CH_2CH_2Ph$), 2.78 - 2.98 (m, 4 H, $ArCH_2CH$, $(CH_2)_3NCH_2$), 3.42 (s, 3 H, $OCH_3$), 5.00 (t, J = 4.14 Hz, 1 H, $ArCH_2CH$), 7.02 - 7.19 (m, 9 H, aromat. H).

**Example 7:**

**Synthesis of *trans*-3-Methoxy-1'-(3-phenylpropyl)-3,4. dihydrospiro[[2]benzopyran-1,3'-piperidin]**

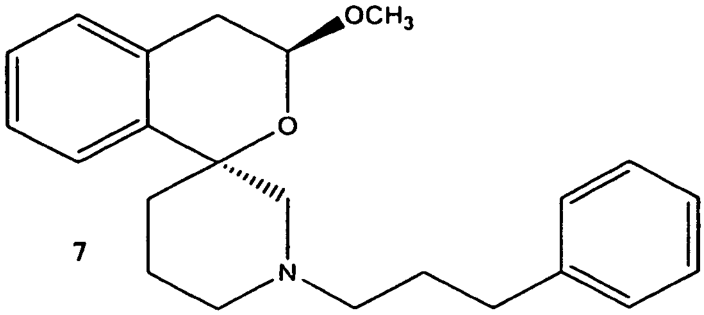

7

See example 6 above
**(7)**: slightly yellow oil

$C_{23}H_{29}NO_2$ (351.5)

| | |
|---|---|
| E: | Ber.: C 78.59; H 8.32; N 3.99<br>Gef.: C 78.83; H 8.38; N 3.71. |
| MS (EI): | m/z (%) = 351 [$M^+$], 320 [$M^+$ -$OCH_3$], 246 [$M^+$ -$(CH_2)_2Ph$]. |
| IR: | $\tilde{v}$ ($cm^{-1}$) = 2923 (v C-H); 1452 (δ -$CH_2$-); 1384 (δ -$CH_3$); 1077, (v C-O); 750, 698 (δ C-H monosubst. Benzene). |
| $^1$H-NMR ($CDCl_3$): | δ (ppm) = 1.53 - 1.58 (m, 2 H, $CH_2CH_2CH_2Ph$), 1.66 - 1.70 (m, 2 H, $CH_2CH_2CH_2N$), 1.78 - 1.83 (m, 2 H, $CH_2CH_2CH_2N$), 1.90 - 2.03 (m, 2 H, $CH_2CH_2CH_2N$). 2.27 - 2.80 (m, 2 H, $NCH_2(CH_2)_2Ph$), 2.52 (t, J = 7.23 Hz, 2 H, $(CH_2)_2CH_2Ph$), 2.80 - 2.96 (m, 4 H, $ArCH_2CH$, $(CH_2)_3NCH_2$), 3.43 (s, 3 H, $OCH_3$), 4.88 (t, J = 3.88 Hz, 1 H, $ArCH_2CH$), 6.98 - 7.21 (m, 9 H, aromat. H). |

**Example 8:**

**Synthesis of *cis* -3-Methoxy-1'-(4-phenylbutyl)-3,4-dihydrospiro[[2]benzopyran-1,3'-piperidin]**

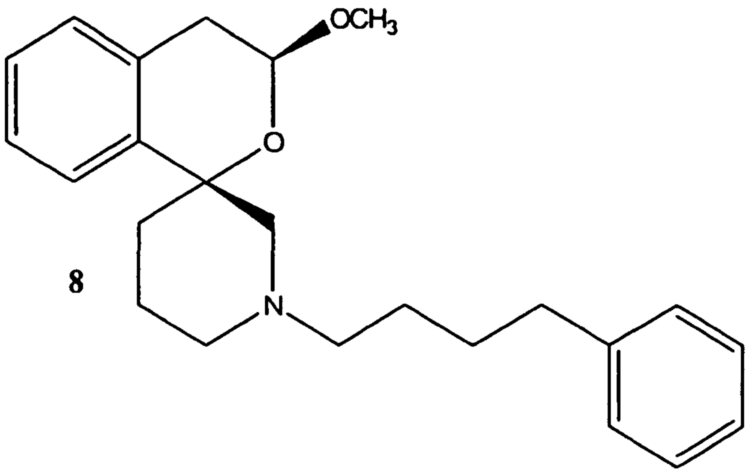

**8**

**Method 1**

3-Methoxy-3,4-dihydrospiro[[2]benzopyran-1,3'-piperidin] **(3)** (59.3 mg, 0.25 mmol) was dissolved in 8 mL acetonitril. 1-Chlo-4-phenylbutan (53.8 mg, 0.32 mmol) and $K_2$-$CO_3$ (243 mg, 1.76 mmol) were added. The mixture was heated under reflux for 40 h, filtered, the solvent removed in vacuo and then purified with flash-chromatography (2 cm, 10 mL, Petrolether:Ethyl acetate = 7:3, **(8)**: $R_f$ = 0.18, **(9)**: $R_f$ = 0.13). Total yield 33 mg (36 %).

**Method 2**

The cis-Diastereomer of compound **(3)** (51.3 mg, 0.22 mmol), 1-Chloro-4-phenylbutane (50.4 mg, 0.30 mmol) and $K_2CO_3$ (240 mg, 1.74 mmol) were dissolved in 3 mL acetonitril and treated with microwave (40 min; 220 Watt, 4 bar, 100°C)

The mixture was filtered and the solvent removed in vacuo. The crude product was purified with flash-chromatography (1 cm, 5 mL, Petrolether: Ethyl acetate = 7:3, $R_f$ = 0.18).
**(8)**: slightly yellow oil, Yield 20 mg (25 %).

$C_{24}H_{31}NO_2$ (365.5)

| | |
|---|---|
| E: | Ber.: C 78.86; H 8.55; N 3.83<br>Gef.: C 78.54; H 8.94; N 3.64. |
| MS (EI): | m/z (%) = 365 [$M^+$]. 334 [$M^+$ -$OCH_3$], 246 [$M^+$ -$(CH_2)_3Ph$], 176 [$M^+$ - $CH_2(CH_2)_2N(CH_2)_4Ph$]. |
| IR: | $\tilde{v}$ ($cm^{-1}$) = 2927 (v C-H); 1452 ($\delta$ -$CH_2$-); 1385 ($\delta$ -$CH_3$); 1076, 1033 (v CO); 750, 697 ($\delta$ C-H monosubst. Benzene). |
| $^1$H-NMR ($CDCl_3$): | $\delta$ (ppm) = 1.45 - 1.63 (m, 6 H, $CH_2(CH_2)_2CH_2Ph$, $CH_2CH_2CH_2N$), 1.69 - 1.78 (m, 1 H, $CH_2CH_2CH_2N$), 1.89 - 1.97 (m, 3 H, $CH_2CH_2CH_2N$ (1 H), $CH_2CH_2CH_2N$ (2 H), 2.22 - 2.40 (m, 2 H, $CH_2(CH_2)_3Ph$), 2.50 - 2.58 (m, 2 H, $(CH_2)_3CH_2Ph$), 2.78 - 2.98 (m, 4 H, $ArCH_2CH$, $(CH_2)_3NCH_2$), 3.40 (s, 3 H, $OCH_3$), 5.00 (s, 1 H, $ArCH_2CH$), 7.04 - 7.20 (m, 9 H, aromat. H). |

The trans-Diastereomer of compound **(3)** (70.0 mg, 0.30 mmol) was dissolved in 3 mL Acetonitrile. 1-Chloro-4-phenylbutane (69.0 mg, 0.41 mmol) and $K_2C0_3$ (331 mg, 2.40 mmol) were added and the mixture was treated with microwave (220 Watt, 40 min, 100°C, 4 bar). The mixture was filtered, washed with $CH_2Cl_2$ and the solvent removed in vacuo. The crude product was purified with flash-chromatography (1 cm, 5 mL, Petrolether:Ethylacetat = 7:3, $R_f$= 0.13).

**Example 9:**

**Synthesis of trans-3-Methoxy-1'-(4-phenylbutyl)-3,4-dihydrospiro[[2]benzopyran-1,3'-piperidin]**

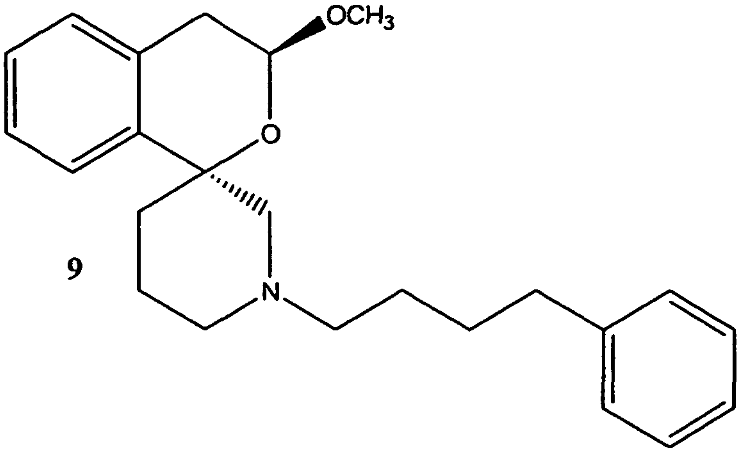

9

**See example 8 above:**
(9): slightly yello oil, Yield 28 mg (26 %).

$C_{24}H_{31}NO_2$ (365.5)

| | |
|---|---|
| E: | Ber.: C 78.86; H 8.55; N 3.83<br>Gef.: C 78.20; H 8.72; N 3.86. |
| MS (EI): | m/z (%) = 365 [$M^+$], 334 [$M^+$ -$OCH_3$]. 246 [$M^+$ -$(CH_2)_3Ph$], 176 [$M^+$ - $CH_2(CH_2)_2N(CH_2)_4Ph$]. |
| IR: | $\tilde{v}$ ($cm^{-1}$) = 2925 (v C-H); 1452 ($\delta$ -$CH_2$-); 1385 ($\delta$ -$CH_3$); 1077 (v C-O); 753, 698 ($\delta$ C-H monosubst. Benzene). |
| $^1$H-NMR ($CDCl_3$): | $\delta$ (ppm) = 1.64 - 1.70 (m, 6 H, $CH_2(CH_2)_2CH_2Ph$, $CH_2CH_2CH_2N$), 1.88 - 1.91 (m, 1 H, $CH_2CH_2CH_2N$), 2.07 - 2.19 (m, 3 H, $CH_2CH_2CH_2N$ (1 H), $CH_2CH_2CH_2N$ (2 H), 2.42 - 2.58 (m, 2 H, $CH_2(CH_2)_3Ph$), 2.71 - 2.75 (m, 2 H, $(CH_2)_3CH_2Ph$), 2.99 - 3.15 (m, 4 H, $ArCH_2CH$, $(CH_2)_3NCH_2$), 3.64 (s, 3 H, $OCH_3$), 5.10 (s, 1 H, $ArCH_2CH$, 7.23 - 7.44 (m, 9 H, aromat. H). |

**Example 10:**

**Synthesis of *cis*-1'-Butyl-3-methoxy-3,4-dihydrospiro[[2]benzopyran-1,3'-piperidin]**

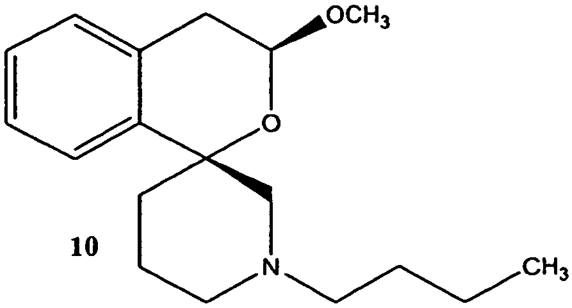

10

1-Bromobutane (108 mg, 0.79 mmol) and $K_2CO_3$ (482 mg, 3.49 mmol) were added to a solution of compound **(3)** (119 mg, 0.51 mmol) in acetonitrile (13 mL) and heated under relux for 30 h. Then the mixture was filtered, the solvent was removed in vacuo and the crude product purified with Flash-Chromatography seperating the diastereomers (2 cm, 10 mL, Petrolether:Ethylacetat = 7:3, **(10):** $R_f$ = 0.40, **(11):** $R_f$=_ 0.30). Total yield: 129 mg (77 %).
(10): Slightly yellow oil

$C_{18}H_{27}NO_2$ (289.4)

| | |
|---|---|
| E: | Ber.: C 74.70; H 9.40; N 4.84<br>Gef.: C 74.48; H 9.47; N 4.71. |
| MS (EI): | m/z (%) = 289 [$M^+$], 258 [$M^+$ -$OCH_3$], 246 [$M^+$ -$(CH_2)_2CH_3$]. |
| IR: | $\tilde{\nu}$ ($cm^{-1}$) = 2928 (ν C-H), 1445 (δ -$CH_2$-), 1385 (δ -$CH_3$), 1077 (ν C-O), 752 (δ C-H). |
| $^1$H-NMR ($CDCl_3$): | δ (ppm) = 0.88 (t, J = 7.4 Hz, 3 H, $(CH_2)_3CH_3$), 1.30 (sext, J = 7.4 Hz, 2 H, $(CH_2)_2CH_2CH_3$), 1.42 - 1.50 (m, 2 H, $CH_2CH_2CH_2CH_3$), 1.61 - 1.68 (m, 1 H, $CH_2CH_2CH_2N$), 1.77 - 1.83 (m, 1 H, $CH_2CH_2CH_2N$), 1.95 - 2.08 (m, 3 H, $CH_2CH_2CH_2N$ (2 H), $CH_2CH_2CH_2N$ (1 H)), 2.12 - 2.30 (m, 2 H, $CH_2CH_2CH_2N$, $NCH_2(CH_2)_2CH_3$), 2.38 - 2.46 (m, 1 H, $NCH_2(CH_2)_2CH_3$), 2.85 - 3.06 (m, 4 H, $(CH_2)_3NCH_2$, $ArCH_2CH$), 3.50 (s, 3 H, $OCH_3$), 5.08 (t, J = 3.9 Hz, 1 H, $ArCH_2CH$), 7.07 - 7.19 (m, 4 H, aromat. H). |

__Example 11:__

**Synthesis of *trans*-1'-Butyl-3-methoxy-3,4-dihydrospiro[[2]benzopyran-1,3'-piperidin]**

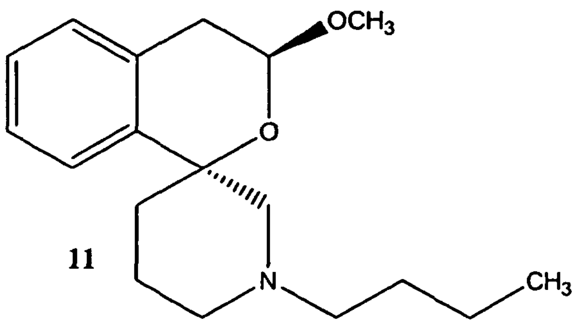

11

See example 10 above.
**(11):** Slightly yellow oil

$C_{18}H_{27}NO_2$ (289.4)

| | |
|---|---|
| E: | Ber.: C 74.70; H 9.40; N 4.84<br>Gef.: C 75.10; H 9.60; N 4.60. |
| MS: | m/z = 289 [$M^+$], 258 [$M^+$ -$OCH_3$], 246 [$M^+$ -$(CH_2)_2CH_3$]. |
| IR: | $\tilde{\nu}$ ($cm^{-1}$) = 2930 (ν C-H), 1443 (δ -$CH_2$-), 1365 (δ -$CH_3$), 1078 (ν C-O), 755 (δ C-H). |
| $^1$H-NMR ($CDCl_3$): | δ (ppm) = 0.89 (t, J = 7.4 Hz, 3 H, $(CH_2)_3CH_3$), 1.29 (sext, J = 7.4 Hz, 2 H, $(CH_2)_2CH_2CH_3$), 1.50 (quint, J = 7.4 Hz, 2 H, $CH_2CH_2CH_2CH_3$), 1.57 - 1.66 (m, 2 H, $CH_2CH_2CH_2N$), 1.94 - 1.97 (m, 1 |

H, $CH_2CH_2CH_2N$), 1.97 - 2.10 (m, 2 H, $CH_2CH_2CH_2N$, $CH_2CH_2CH_2N$), 2.31 - 2.45 (m, 3 H, $CH_2CH_2CH_2N$ (1 H), $NCH_2(CH_2)_2CH_3$ (2 H)), 2.91 - 3.01 (m, 4 H, $(CH_2)_3NCH_2$, $ArCH_2CH$), 3.54 (s, 3 H, $OCH_3$), 4.98 (t, J = 3.9 Hz, 1 H, $ArCH_2CH$), 7.09 - 7.31 (m, 4 H, aromat. H).

**Example 12:**

**Synthesis of *cis*-1'-Hexyl-3-methoxy-3,4-dihydrospiro[[2]benzopyran-1,3'-piperidin]**

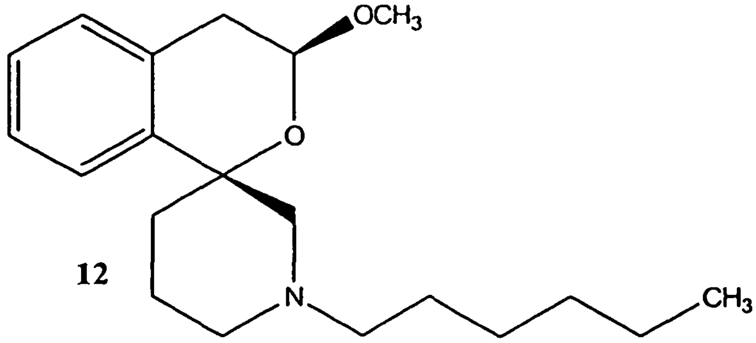

12

Methode 1

Compound **(3)** (91.3 mg, 0.39 mmol), 1-Chlorohexane (63.7 mg, 0.53 mmol) and $K_2$-$CO_3$ (334 mg, 2.42 mmol) were dissolved in acetonitril (9 mL) and heated under reflux for 30 h. After filtration the solvent was removed completely and the product prorified with Flash-Chromatography (2 cm, 10 mL. Petrolether:Ethylacetat = 8:2, **(12):** $R_f$= 0.28, (13): $R_f$ = 0.23). Total yield 28 mg (23 %).

**Method 2**

1-Chlorohexane (183 mg, 1.52 mmol) was dissolved together with Sodium iodide (271 mg, 1.81 mmol) in acetone (3 mL) and treated with microwave (180 Watt, 2 bar, 80° C, 10 min).

The Sodium-chloride was removed by filtration and to the clear solution containing 1-lodo-hexane was added compound **(3)** (230 mg, 0.99 mmol) and $K_2CO_3$ (1.10 g, 8.0 mmol) and treated with 1 mL acetone and then with microwave (180 Watt, max. 5 bar, 100°C, 40 min)

Following the reaction the mixture was filtrated, washed with $CH_2Cl_2$ and the solvent removed in vacuo. The crude product was purified by flash-chromatography seperating the diastereomers (2 cm, 10 mL, Petrolether:Ethylacetat = 8:2, **(12):** $R_f$= 0.28, **(13):** $R_f$ = 0.23). Total yield 276 mg (88 %).
**(12):** Slightly yellow oil

$C_{20}H_{31}NO_2$ (317.5)

| | |
|---|---|
| E: | Ber.: C 75.67; H 9.84; N 4.41<br>Gef.: C 75.79; H 10.13; N 4.37. |
| MS (EI): | m/z (%) =317 [$M^+$], 286 [$M^+$ -$OCH_3$], 246 [$M^+$ -$(CH_2)_4$ $CH_3$]. |
| IR: | $\tilde{\nu}$ ($cm^{-1}$) = 2925 (ν C-H), 1445 (δ -$CH_2$-), 1386 (δ -$CH_3$), 1077 (ν C-O), 752 (δ C-H). |
| $^1$H-NMR ($CDCl_3$): | δ (ppm) = 0.84 - 0.87 (m, 3 H, $(CH_2)_5CH_3$), 1.20 - 1.32 (m, 6 H, $CH_2CH_2(CH_2)_3CH_3$), 1.42 - 1.51 (m, 2 H, $CH_2CH_2(CH_2)_3CH_3$), 1.63 - 1.66 (m, 1H, $CH_2CH_2CH_2N$), 1.76 -1.84 (m, 1 H, $CH_2CH_2CH_2N$), 1.95 - 2.10 (m, 3 H, $CH_2CH_2CH_2N$ (2 H), $CH_2CH_2CH_2N$ (1 H)), 2.15 - 2.29 (m, 2 H, $CH_2CH_2CH_2N$, $NCH_2(CH_2)_4CH_3$), 2.39 - 2.47 (m, 1 H, $NCH_2(CH_2)_4CH_3$), 2.85 - 3.06 (m, 4 H, $(CH_2)_3NCH_2$, $ArCH_2CH$), 3.49 (s, 3 H, $OCH_3$), 5.08 (t, J = 3.9 Hz, 1 H, $ArCH_2CH$), 7.09 - 7.21 (m, |

4 H, aromat. H).

**Example 13:**

**Synthesis of *trans*-1'-Hexyl-3-methoxy-3,4-dihydrospiro[[2]benzopyran-1,3'-piperidin]**

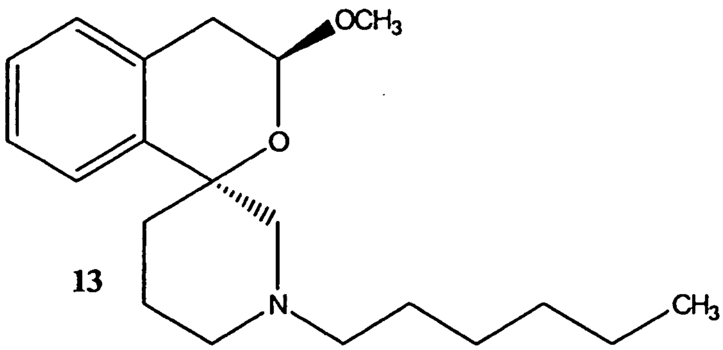

**13**

See example 12 above.
**(13):** Slightly yellow oil

$C_{20}H_{31}NO_2$ (317.5)

| | |
|---|---|
| E: | ber.: C 75.67, H 9.84, N 4.41<br>gef.: C 75.41, H 10.00, N 4.41. |
| MS (EI): | m/z (%) = 317 [$M^+$], 286 [$M^+$ -$OCH_3$], 246 [$M^+$ -$(CH_2)_4CH_3$]. |
| IR: | $\tilde{v}$ ($cm^{-1}$) = 2926 (v C-H), 1444 ($\delta$ -$CH_2$-), 1385 ($\delta$ -$CH_3$), 1078 (v C-O), 755 ($\delta$ C-H). |
| $^1$H-NMR ($CDCl_3$): | $\delta$ (ppm) = 0.78 - 0.84 (m, 3 H, $(CH_2)_5CH_3$), 1.15 - 1.24 (m, 6 H, $CH_2CH_2(CH_2)_3CH_3$). 1.39 - 1.47 (m, 2 H, $CH_2CH_2(CH_2)_3CH_3$). 1.50 - 1.59 (m, 2 H, $CH_2CH_2CH_2N$), 1.84 - 2.05 (m, 3 H, $CH_2CH_2CH_2N$ (2 H), $CH_2CH_2CH_2N$ (1 H)), 2.21 - 2.35 (m, 3 H, $CH_2CH_2CH_2N$ (1 H), $NCH_2(CH_2)_4CH_3$ (2 H)), 2.84 - 2.92 (m, 4 H, $(CH_2)_3NCH_2$. $ArCH_2CH$), 3.46 (s, 3 H, $OCH_3$), 4.93 (t, J = 3.5 Hz, 1 H, $ArCH_2CH$), 7.02 - 7.23 (m, 4 H, aromat. H). |

**Example 14:**

**Synthesis of *cis*-3-Methoxy-1'-octyl-3,4-dihydrospiro[[2]benzopyran-1,3'-piperidin]**

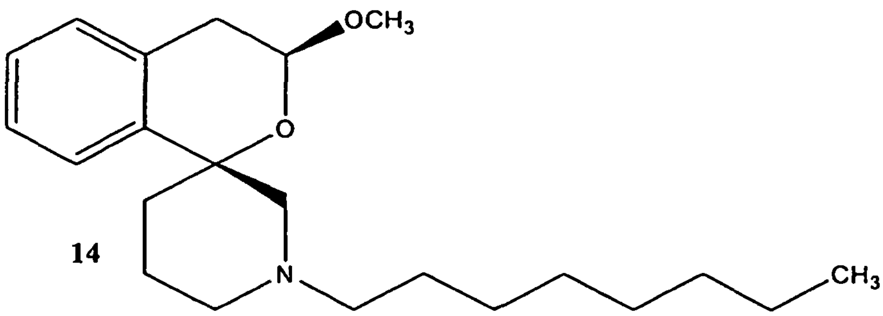

**14**

To a solution of compound (3) (136 mg, 0.56 mmol) in acetonitril (14 mL) 1-Bromo-octane (137 mg, 0.71 mmol) and $K_2CO_3$ (481 mg, 3.48 mmol) were added and the mixture heated under reflux for 25.5 h. Then the base was removed by filtration, the slvent removed in vacuo and the product purified by Flash-Chromatography seperating the diastereomers (3 cm, 20 mL, Petrolether:Ethylacetat = 8:2, **(14):** $R_f$ = 0.24, **(15):** $R_f$ = 0.14). Total yield 183 mg (95 %).

**(14):** Slightly yellow oil

$C_{22}H_{35}NO_2$ (345.5)

| | |
|---|---|
| E: | Ber.: C 76.48; H 10.21; N 4.05<br>Gef.: C 76.55; H 10.17; N 3.88. |
| MS (EI): | m/z (%) = 345 [$M^+$], 314 [$M^+$-$OCH_3$], 246 [$M^+$ -$(CH_2)_6$ $CH_3$]. |
| IR: | $\tilde{\nu}$ ($cm^{-1}$) = 2924 (ν C-H), 1445 (δ -$CH_2$-), 1386 (δ -$CH_3$), 1078 (ν C-O), 752 (δ C-H). |
| $^1$H-NMR ($CDCl_3$): | δ (ppm) = 0.84 - 0.88 (m, 3 H, $(CH_2)_7CH_3$), 1.21 - 1.30 (m, 10 H, $CH_2CH_2(CH_2)_5CH_3$), 1.42 - 1.53 (m, 2 H, $CH_2CH_2(CH_2)_5CH_3$), 1.62 - 1.68 (m, 1 H, $CH_2CH_2CH_2N$), 1.76 - 1.84 (m, 1 H, $CH_2CH_2CH_2N$), 1.95 - 2.10 (m, 3 H, $CH_2CH_2CH_2N$ (2 H), $CH_2CH_2CH_2N$ (1 H)), 2.16 - 2.30 (m, 2 H, $CH_2CH_2CH_2N$, $NCH_2(CH_2)_6CH_3$), 2.39 - 2.48 (m, 1 H, $NCH_2(CH_2)_6CH_3$). 2.85 - 3.06 (m, 4 H, $(CH_2)_3NCH_2$, $ArCH_2CH$), 3.49 (s, 3 H, $OCH_3$), 5.08 (t, J = 3.9 Hz, 1 H, $ArCH_2CH$), 7.10 - 7.26 (m, 4 H, aromat. H). |

**Example 15:**

**Synthesis of *trans*-3-Methoxy-1'-octyl-3,4-dihydrospiro[[2]benzopyran-1,3'-piperidin]**

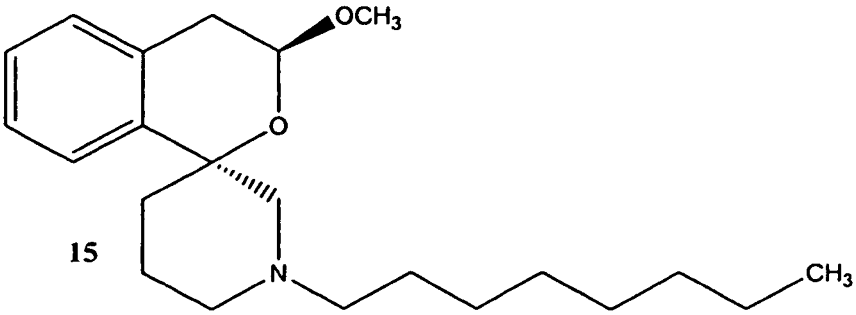

15

See example 14 above

**(15):** Slightly yellow oil

$C_{22}H_{35}NO_2$ (345.5)

| | |
|---|---|
| E: | Ber.: C 76.48; H 10.21; N 4.05<br>Gef.: C 76.52; H 10.48; N 4.24. |
| MS (EI): | m/z (%) = 345 [$M^+$], 314 [$M^+$ -$OCH_3$], 246 [$M^+$ -$(CH_2)_6CH_3$]. |
| IR: | $\tilde{\nu}$ ($cm^{-1}$) = 2923 (ν C-H), 1452 (δ -$CH_2$-), 1079 (ν C-O), 754 (δ C-H). |
| $^1$H-NMR ($CDCl_3$): | δ (ppm) = 0.85 - 0.89 (m, 3 H, $(CH_2)_7CH_3$), 1.21 - 1.30 (m, 10 H, $CH_2CH_2(CH_2)_5CH_3$), 1.45 - 1.53 (m, 2 H, $CH_2CH_2(CH_2)_5CH_3$), 1.57 - 1.63 (m, 2 H, $CH_2CH_2CH_2N$), 1.92 - 2.12 (m, 3 H, $CH_2CH_2CH_2N$ |

(2 H), $CH_2CH_2CH_2N$ (1 H)), 2.32 - 2.42 (m, 3 H, $CH_2CH_2CH_2N$ (1 H), $NCH_2(CH_2)_6CH_3$ (2 H)), 2.87 - 3.02 (m, 4 H, $(CH_2)_3NCH_2$, $ArCH_2CH$), 3.54 (s, 3 H, $OCH_3$), 4.98 (t, J = 3.5 Hz, 1 H, $ArCH_2CH$), 7.08 - 7.31 (m, 4 H, aromat. H).

**Example 16:**

**Synthesis of *cis*-1'-Decyl-3-methoxy-3,4-dihydrospiro[[2]benzopyran-1,3'-piperidin]**

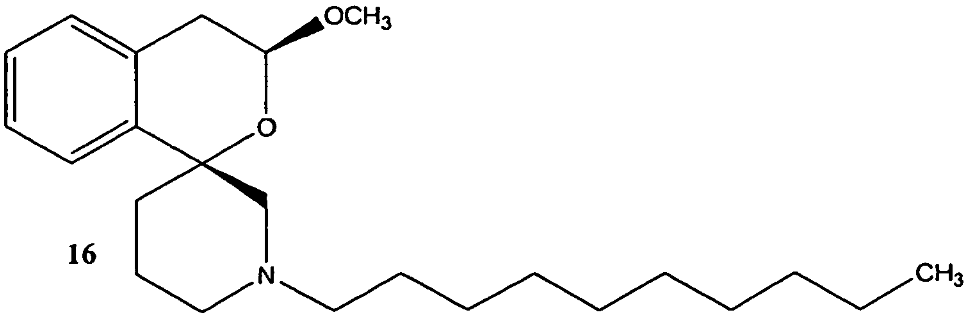

16

Compound **(3)** (127 mg, 0.55 mmol) was dissolved in acetonitrile (14 mL) and 1-Bromo-decane (156 mg, 0.71 mmol) and $K_2CO_3$ (486 mg, 3.52 mmol) were added. The mixture was heated under reflux for 25.5 h, then filtrated and the solvent completely removed in vacuo. The crude product was purified by Flash-Chromatography (3 cm, 20 mL, Petrolether: Ethyl acetate = 9:1, **(16):** $R_f$ = 0.21, **(17):** $R_f$= 0.14) seperating the diastereomers. Total yield 173 mg (85 %). **(16):** Slightly yellow oil

$C_{24}H_{39}NO_2$ (373.6)

E: Ber.: C 77.16; H 10.52; N 3.75
Gef.: C 76.70; H 10.63; N 3.50.

MS (EI): m/z (%) = 373 [$M^+$], 342 [$M^+$ -$OCH_3$], 246 ($M^+$ -$(CH_2)_8CH_3$].

IR: $\tilde{\nu}$ ($cm^{-1}$) = 2923 (ν C-H), 1450 (δ -$CH_2$-), 1386 (δ -$CH_3$), 1078 (ν C-O), 752 (δ C-H).

$^1$H-NMR ($CDCl_3$): δ (ppm) = 0.85 - 0.89 (t, J = 7.0 Hz, 3 H, $(CH_2)_9CH_3$), 1.19 - 1.25 (m, 14 H, $CH_2CH_2(CH_2)_7CH_3$), 1.42 - 1.51 (m, 2 H, $CH_2CH_2(CH_2)_7CH_3$), 1.76 - 1.84 (m, 1 H, $CH_2CH_2CH_2N$), 1.76 - 1.84 (m, 1 H, $CH_2CH_2CH_2N$), 1.94 - 2.07 (m, 3 H, $CH_2CH_2CH_2N$ (2 H), $CH_2CH_2CH_2N$ (1 H)), 2.15 - 2.28 (m, 2 H, $CH_2CH_2CH_2N$, $NCH_2(CH_2)_8CH_3$), 2.37 - 2.45 (m, 1 H, $NCH_2(CH_2)_8CH_3$), 2.85 - 3.06 (m, 4 H, $(CH_2)_3NCH_2$, $ArCH_2CH$), 3.49 (s, 3 H, $OCH_3$), 5.08 (t, J = 3.9 Hz, 1 H, $ArCH_2CH$), 7.09 - 7.22 (m, 4 H, aromat. H).

**Example 17:**

**Synthesis of *trans*-1'-Decyl-3-methoxy-3,4-dihydrospiro[[2]benzopyran-1,3'-piperidin]**

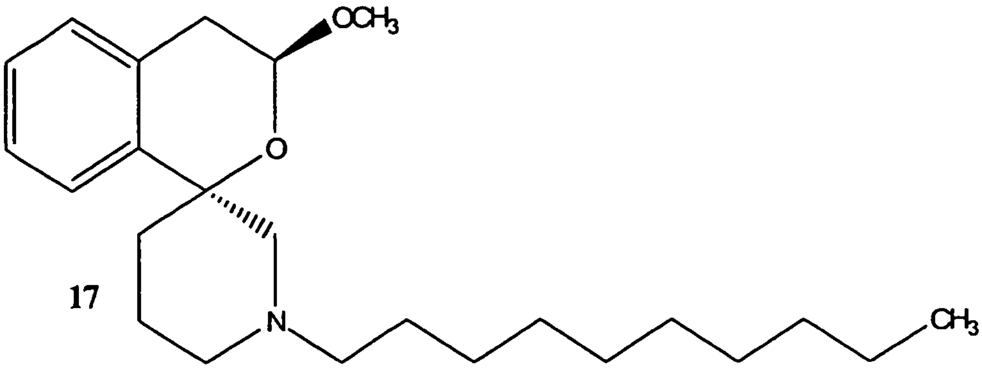

17

See example 16 above.
**(17):** Slightly yellow oil

$C_{24}H_{39}NO_2$ (373.6)

| | |
|---|---|
| E: | Ber.: C 77.16; H 10.52; N 3.75<br>Gef.: C 76.75; H 10.80; N 3.83. |
| MS (EI): | m/z (%) = 373 [$M^+$], 342 [$M^+$ -$OCH_3$], 246 [$M^+$ -$(CH_2)_8CH_3$]. |
| IR: | $\tilde{v}$ ($cm^{-1}$) = 2922 (v C-H), 1453 (δ -$CH_2$-), 1079 (v C-O), 754 (δ C-H). |
| $^1$H-NMR ($CDCl_3$): | δ (ppm) = 0.85 - 0.89 (t, J = 7.0 Hz, 3 H, $(CH_2)_9CH_3$), 1.20 - 1.33 (m, 14 H, $CH_2CH_2(CH_2)_7CH_3$), 1.46 - 1.55 (m, 2 H, $CH_2CH_2(CH_2)_7CH_3$), 1.55 - 1.65 (m, 2 H, $CH_2CH_2CH_2N$), 1.94 - 2.12 (m, 3 H, $CH_2CH_2CH_2N$ (2 H), $CH_2CH_2CH_2N$ (1 H)), 2.30 - 2.42 (m, 3 H, $CH_2CH_2CH_2N$ (1 H), $NCH_2(CH_2)_8CH_3$ (2 H)), 2.87 - 3.02 (m, 4 H, $(CH_2)_3NCH_2$, $ArCH_2CH$), 3.54 (s, 3 H, $OCH_3$), 4.98 (t, J = 3.5 Hz, 1 H, $ArCH_2CH$), 7.09 - 7.31 (m, 4 H, aromat. H). |

__Example 18:__

**Synthesis of *cis*/*trans*-1'-Benzyl-3-methoxy-3,4-dihydrospiro[[2]benzopyran-1,3'-pyrrolidin]**

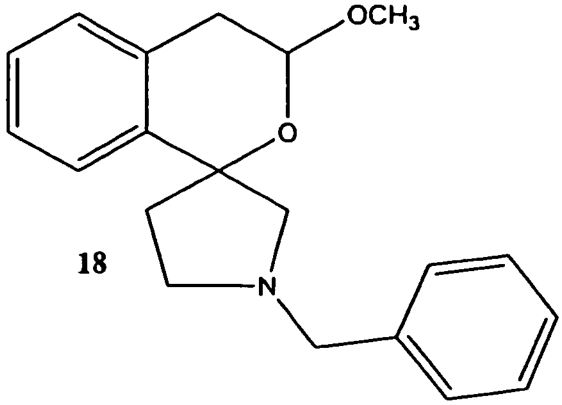

18

2-(2-Bromphenyl)acetaldehyddimethylacetal (1.18 g, 4.83 mmol) was dissolved under nitrogen atmosphere in THF (15 mL) and cooled to -78°C. 3.7 mL n-Butyllithium, 1.6 M in Hexan (5.92 mmol), were slowly added. After stirring for 20 min 1-Benzylpyrrolidin-3-one (846 mg, 4.84 mmol), dissolved in THF (10 mL), was slowly added. The continously stirred mixture was slowly (over 12 h) heated up to room temperature with the solution turning yellow. Then, 20 mL of a solution of saturated Ammonium-chloride was added and extracted six times with diethylether. The Ether phases were

washed twice withy $H_2O$. Then, the organic phase was extracted six times with a solution of 5% HCl and the HCl phase washed twice with ether. To the $H_2O$-Phase was slowly added solid KOH until a pH>9 was reached. Then, the $H_2O$-Phase was extracted six times with diethylether. The organic phase was washed twice with $H_2O$ and twice with a solution of 10%-$NaHSO_3$. It was dried over $Na_2SO_4$, filtered and the solvent removed in vacuo. The crude product was purified by flash-chromatography (5 cm, 30 mL, Dichlormethan: Methanol = 98:2, $R_f$ = 0.41).
Slightly yellow oil, yield 617 mg (41 %).
$C_{20}H_{23}NO_2$ (309.4)

| | |
|---|---|
| E: | Ber.: C 77.64; H 7.49; N 4.53<br>Gef.: C 77.34; H 7.57; N 4.37. |
| MS (EI): | m/z (%) = 309 [$M^+$278 [$M^+$ -$OCH_3$], 249 [$M^+$ -$OCHOCH_3$]. |
| IR: | $\tilde{v}$ ($cm^{-1}$) = 2910 (ν C-H), 1452 (δ -$CH_2$-), 1385 (δ -$CH_3$), 1075 (ν C-O). 754/ 697 (δ C-H monosubst. Benzene). |
| $^1$H-NMR ($CDCl_3$): | δ (ppm) = 2.13 - 2.24 (m, 0.5 H, C$H_2$$CH_2$N (cis or trans)), 2.32 - 2.45 (m, 1.5 H, C$H_2$$CH_2$N (cis, trans)), 2.71 - 2.80 (q, J = 7.2 Hz, 0.5 H, $CH_2$C$H_2$N$CH_2$ (cis or trans)), 2.87 - 3.05 (m, 5 H, $CH_2$C$H_2$N$CH_2$ (1.5 H; cis, trans), $CH_2$$CH_2$NC$H_2$ (2 H), ArC$H_2$CHO$CH_3$ (1.5 H; cis, trans)), 3.10 - 3.18 (m, 0.5 H, ArC$H_2$CHO$CH_3$ (cis or trans)), 3.50/3.54 (2 s, together 3 H, OC$H_3$ (cis, trans)), 3.69 - 3.80 (m, 2 H, NC$H_2$Ph), 4.73/4.82 (2 t, J = 5.2 Hz, J = 3.3 Hz, together 1 H, $CH_2$C*H*O$CH_3$(cis, trans)), 7.03 (d, J = 7.2 Hz, 1 H, aromat. H), 7.13 - 7.48 (m, 8 H, aromat. H).<br>Relation of diastereomers 52:48. |

**Example 19:**

**Synthesis of *cis*/*trans*-3-Methoxy-3,4-dihydrospiro[[2]benzopyran-1,3'-pyrrolidin]**

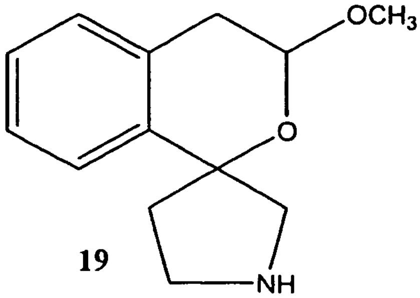

**19**

Compound **(18)** (1.04 g, 3.38 mmol) was dissolved under nitrogen in methanol (20 mL). Ammonium formiate (1.09 g, 17.3 mmol) and Pd/C (213 mg, 10 %) were added as solids and der mixture heated under reflux for 2h. Then, the catalyst was removed by filtration and the solvent removed in vacuo. The crude product was purified by Flash-Chromatography (4 cm, 30 mL, Methanol: Ammonia = 98:2, $R_f$= 0.48).
Clear Oil, Yield 604 mg (82 %).

$C_{13}H_{17}NO_2$ (219.3)

| | |
|---|---|
| MS (EI): | m/z (%) = 219 [$M^+$], 188 [$M^+$ -$OCH_3$], 159 [$M^+$ -$OCHOCH_3$], 145 [$M^+$ -$OCH_3$, - NH$(CH_2)_2$]. |
| IR: | $\tilde{v}$ ($cm^{-1}$) = 2939/ 2873 (ν C-H), 1444 (δ -$CH_2$-), 1387 (δ -$CH_3$), 1073 (ν CO), 754 (δ C-H). |
| $^1$H-NMR ($CDCl_3$): | δ (ppm) = 2.03 - 2.41 (m, 2 H, C$H_2$$CH_2$N), 2.59 (br s, 1 H, $CH_2$N*H*$CH_2$), 2.88 - 3.01 (m, 2.5 H, $CH_2$C$H_2$N (2 H), ArC$H_2$CHO$CH_3$ (0.5 H; cis or trans), 3.13 (d, J = 12.5 Hz, 0.5 H, ArC$H_2$CHO$CH_3$ (cis or trans)), 3.20 - 3.30 (m, 1 H, $CH_2$$CH_2$NC$H_2$), 3.32 - 3.43 (m, 2 H, ArC$H_2$CHO$CH_3$ (1 H; cis or trans), $CH_2$$CH_2$NC$H_2$ (1 H)), 3.52/3.53 (2 s, together 3 H, OC$H_3$ (cis, trans)), 4.77 - 4.85 (m, 1 H, Ar$CH_2$C*H*O$CH_3$), 7.09 - 7.26 (m, 4 H, aromat. H). Relation of diastereomers 57:43. |

**Example 20:**

**Synthesis of *cis*/*trans*-3-Methoxy-1'-(2-phenylethyl)-3,4-dihydrospiro[[2]benzopyran-1,3'-pyrrolidin]**

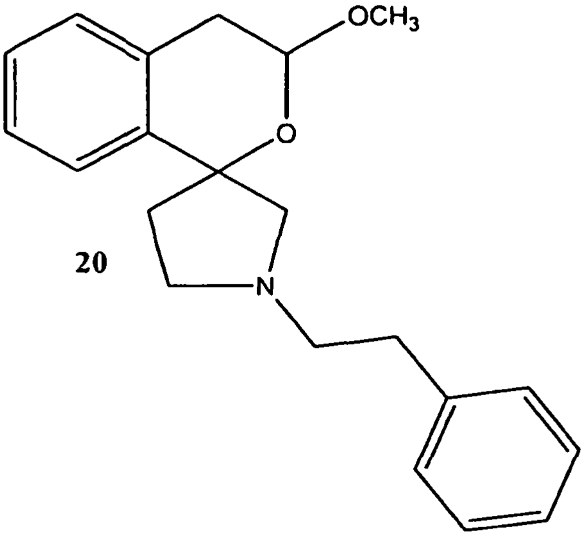

20

Compound **(19)** (58.6 mg, 0.27 mmol), 1-Chloro-2-phenylethane (57.0 mg, 0.41 mmol) and $K_2CO_3$ (298 mg, 2.16 mmol) were dissolved in 2 mL Acetonitril and treated with microwave (180 Watt, max. 5 bar, 140°C, 25 min hold time).
The mixture was filtered and washed with $CH_2Cl_2$. Then, the solvent was removed in vacuo and the crude product purified by flash-chromatography (1 cm, 5 mL, Petrolether:Ethyl acetate = 7:3, $R_f$ = 0.22).
Slightly yellow oil, Yield 40 mg (45 %).

$C_{21}H_{25}NO_2$ (323.4)

| | |
|---|---|
| E: | Ber.: C 77.99; H 7.79; N 4.33<br>Gef.: C 77.73; H 7.85; N 4.24. |
| MS (EI): | m/z (%) = 292 [$M^+$ -$OCH_3$], 232 [$M^+$ -$CH_2$Ph]. |
| IR: | $\tilde{v}$ ($cm^{-1}$) = 2939 (v C-H), 1452 ($\delta$ -$CH_2$-), 1384 ($\delta$ -$CH_3$), 1076 (v C-O), 752/ 698 ($\delta$ C-H monosubst. Benzene). |
| $^1$H-NMR ($CDCl_3$): | $\delta$ (ppm) = 2.11 - 2.20 (m, 0.5 H, C$H_2$$CH_2$N (cis or trans)), 2.29 - 2.46 (m, 1.5 H, C$H_2$$CH_2$N (cis, trans)), 2.77 - 3.06 (m, 9 H, N(C$H_2$)$_2$Ph (4 H), $CH_2$C$H_2$N$CH_2$ (4 H), ArC$H_2$CHO$CH_3$ (1.5 H; cis, trans)), 3.19 - 3.24 (m, 0.5 H, ArC$H_2$CHO$CH_3$), 3.53/3.54 (2 s, together 3 H, OC$H_3$ (cis, trans)), 4.80 - 4.85 (m, 1 H, Ar$CH_2$C*H*O$CH_3$), 7.03 - 7.08 (m, 1 H, aromat. H), 7.15 - 7.39 (m, 8 H, aromat. H). Relation of Diastereomers 52:48. |

**Example 21:**

**Synthesis of *cis*/*trans*-3-Methoxy-1'-(4-phenylbutyl)-3,4-dihydrospiro[(2]benzopyran-1,3'-pyrrolidin]**

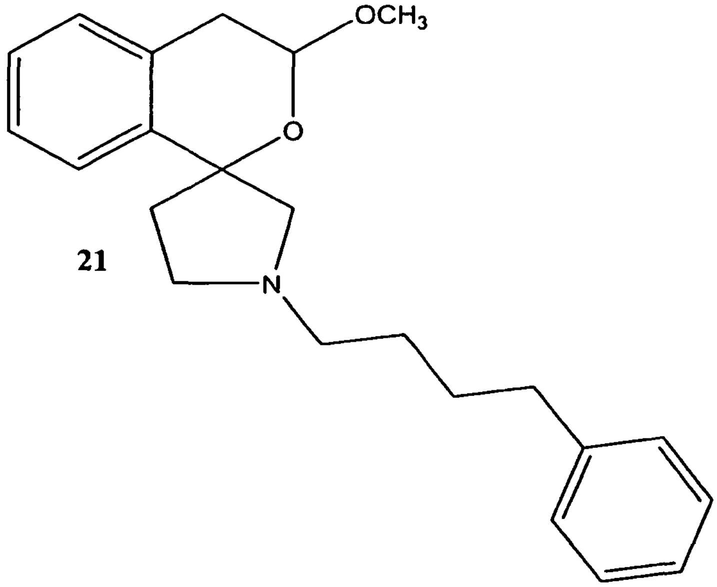

21

Compound **(19)** (58.0 mg, 0.26 mmol), 1-Chlor-4-phenylbutan ( 60.7 mg, 0.36 mmol) and $K_2CO_3$ (298 mg, 2.16 mmol) were dissolved in 2 mL acetonitril and treated with microwave (180 Watt, max.: 5 bar, 140°C, 25 min).
The mixture was filtered and washed with $CH_2Cl_2$. Then, the solvent was removed in vacuo and the crude product purified by flash-chromatography (1 cm, 5 mL, Petrolether:Ethyl acetate = 6:4, $R_f$= 0.38).
Slightly yellow oil, yield 60 mg (56 %).

$C_{23}H_{29}NO_2$ (351.5)

| | |
|---|---|
| E: | Ber.: C 78.60; H 8.32; N 3.98<br>Gef.: C 78.13; H 8.36; N 3.95. |
| MS: | m/z (%) = 351 [$M^+$], 320 [$M^+$ -$OCH_3$], 232 [$M^+$ -$(CH_2)_3$Ph], 201 [$M^+$ -$OCH_3$, - $(CH_2)_3$Ph]. |
| IR: | $\tilde{v}$ ($cm^{-1}$) = 2933 (ν C-H), 1452 (δ -$CH_2$-), 1385 (δ -$CH_3$), 1076 (ν C-O), 748/ 698 (δ C-H monosubst. Benzene). |
| $^1$H-NMR ($CDCl_3$): | δ (ppm) = 1.49 - 1.53 (m, 2 H, $NCH_2CH_2CH_2CH_2$Ph (2 H)), 1.62 - 1.66 (m, 2 H, $NCH_2CH_2CH_2CH_2$Ph (2 H)), 2.03 - 2.10 (m, 0.5 H, $CH_2CH_2$N (cis or trans)), 2.20 - 2.38 (m, 1.5 H, $CH_2CH_2$N (cis, trans)), 2.47 - 2.51 (m, 2 H, $N(CH_2)_3CH_2$Ph), 2.55 - 2.59 (m, 2 H, $CH_2CH_2$N), 2.61 - 2.67 (m, 0.5 H, $NCH_2$ $(CH_2)_3$Ph (cis or trans)), 2.75 - 2.92 (m, 5 H, $NCH_2(CH_2)_3$Ph (1.5 H; cis, trans), $ArCH_2CHOCH_3$ (2 H), $CH_2CH_2NCH_2$ (1.5 H; cis, trans)), 3.05 - 3.09 (m, 0.5 H, $CH_2CH_2NCH_2$ (cis or trans), 3.46 (s, 3 H, $OCH_3$), 4.70 - 4.78 (m, 1 H, $ArCH_2CHOCH_3$), 6.95 - 7.02 (m, 1 H, aromat. H), 7.08 - 7.38 (m, 8 H, aromat. H).<br>Relation of diastereomers 54:46. |

## Example 22:

**Synthesis of *cis*/*trans*-1'-Butyl-3-methoxy-3,4-dihydrospiro[[2]benzopyran-1,3'-pyrrolidin]**

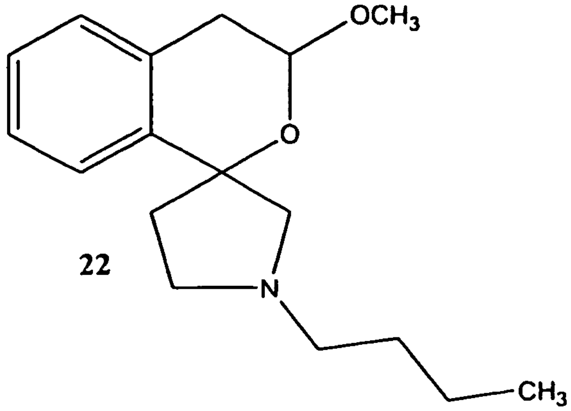

22

1-Bromo-butane (88.0 mg, 0.64 mmol), $K_2CO_3$ (483 mg, 3.50 mmol) and compound **(19)** (92.3 mg, 0.42 mmol) were dissolved in acetonitril (3 mL) gelöst and treated with microwave (180 Watt, max.: 5 bar, 140°C, 40 min).
Then the mixture was filtered, washed with $CH_2Cl_2$ and the solvent completely removed in vacuo. The crude product was purified by flash-chromatography (1 cm, 5 mL, Petrolether:Ethyl acetate = 7:3, $R_f$= 0.31).
Slightly yellow oil, yield 76 mg (69 %).

$C_{17}H_{25}NO_2$ (275.4)

| | |
|---|---|
| E: | Ber.: C 74.14; H 9.15; N 5.09<br>Gef.: C 74.10; H 9.60; N 4.60. |
| MS (EI): | m/z (%) = 275 [$M^+$], 244 [$M^+$ -$OCH_3$], 232 [$M^+$ -$(CH_2)_2CH_3$], 201 [$M^+$ -$OCH_3$, - $(CH_2)_2CH_3$]. |
| IR: | $\tilde{\nu}$ ($cm^{-1}$) = 2929 (ν C-H), 1452 (δ -$CH_2$-), 1385 (δ -$CH_3$), 1076 (ν C-O), 758 (δ C-H). |
| $^1$H-NMR ($CDCl_3$): | δ (ppm) = 0.84 - 0.88 (2 t, J = 7.2 Hz, together 3 H, $N(CH_2)_3CH_3$ (cis, trans)), 1.31 (m, 2 H, $N(CH_2)_2CH_2CH_3$), 1.43 - 1.47 (m, 2 H, $NCH_2CH_2CH_2CH_3$), 2.03 - 2.12 (m, 0.5 H, $CH_2CH_2N$ (cis or trans)), 2.21 - 2.36 (m, together 1.5 H, $CH_2CH_2N$ (cis, trans)), 2.44 - 2.51 (m, 2 H, $CH_2CH_2N$), 2.62 - 2.70 (m, 0.5 H, $NCH_2(CH_2)_2CH_3$), 2.78 - 2.95 (m, together 5 H, $NCH_2(CH_2)_2CH_3$ (1.5 H; cis, trans), $ArCH_2CHOCH_3$ (2 H), $CH_2CH_2NCH_2$ (1.5 H; cis, trans)), 3.08 - 3.13 (m, 0.5 H, $CH_2CH_2NCH_2$ (cis or trans), 3.47 (s, 3 H, $OCH_3$), 4.72 - 4.76 (m, 1 H, $ArCH_2CHOCH_3$), 6.98 - 7.00 (m, 1 H, aromat. H), 7.09- 7.35 (m, 3 H, aromat. H).<br>Relation of diastereomers 52:48. |

__Example 23:__

**Synthesis of *cis*/*trans*-3-Methoxy-1'-octyl-3,4-dihydrospiro[[2]benzopyran-1,3'-pyrrolidin]**

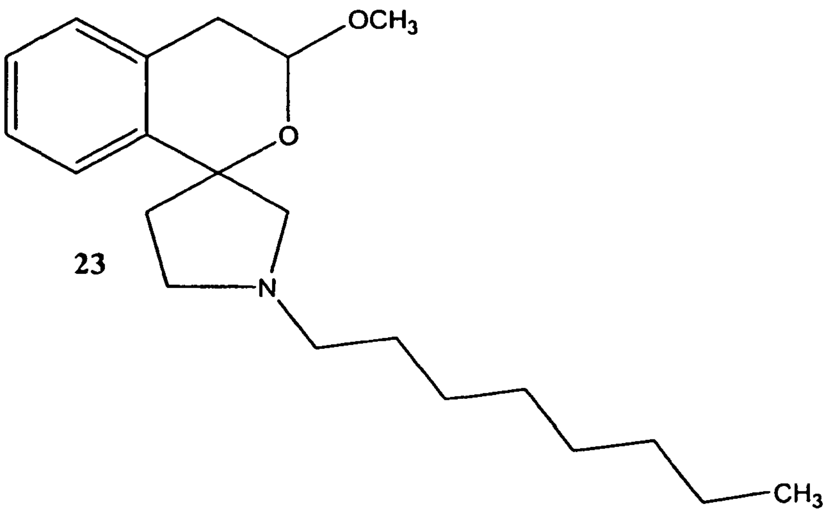

23

Compound **(19)** (91.0 mg, 0.42 mmol) was dissolved in Acetonitril (3 mL). 1-Bromoctan (111 mg, 0.57 mmol) and $K_2CO_3$ (452 mg, 3.28 mmol) were added and treated with microwave (180 Watt, max.: 5 bar, 140°C, 40 min).

Then the mixture was filtered, washed with $CH_2Cl_2$, the solvent completely removed in vacuo and the crude product purified by Flash-Chromatography (1 cm, 5 mL, Petrolether: Ethylacetat = 6:4, $R_f$ = 0.33).

Yellow Oil, Yield 85 mg (64 %).

$C_{21}H_{33}NO_2$ (331.5)

| | |
|---|---|
| E: | Ber.: C 76.09; H 10.03; N 4.23<br>Gef.: C 76.42; H 10.05; N 4.17. |
| MS (EI): | m/z (%) = 331 [$M^+$], 300 [$M^+$ -$OCH_3$]. 232 [$M^+$ -$(CH_2)_6CH_3$], 201 [$M^+$ -$OCH_3$,-$(CH_2)_6CH_3$]. |
| IR: | $\tilde{v}$ ($cm^{-1}$) = 2924 (v C-H), 1452 ($\delta$ -$CH_2$-), 1385 ($\delta$ -$CH_3$), 1077 (v C-O), 757 ($\delta$ C-H). |
| $^1$H-NMR ( $CDCl_3$) | $\delta$ (ppm) = 0.85 - 0.90 (2 t, J = 6.6 Hz, together 3 H, $CH_2CH_3$ (cis, trans)), 1.22 - 1.40 (m, 10 H, $CH_2(CH_2)_5CH_3$), 1.48 - 1.58 (m, 2 H, $CH_2(CH_2)_5CH_3$), 2.10 - 2.19 (m, 0.5 H, $CH_2CH_2N$ (cis or trans)), 2.28 - 2.43 (m, 1.5 H, $CH_2CH_2N$ (cis, trans)), 2.53 - 2.55 (m, 2 H, $CH_2CH_2N$), 2.69 - 2.77 (m, 0.5 H, $NCH_2(CH_2)_6CH_3$), 2.83 - 3.02 (m, 5 H, $NCH_2(CH_2)_6CH_3$ (1.5 H), $ArCH_2CHOCH_3$ (2 H), $CH_2CH_2NCH_2$ (1.5 H)), 3.14 - 3.19 (m, 0.5 H, $CH_2CH_2NCH_2$ (cis or trans), 3.54 (s, 3 H, $OCH_3$), 4.79 - 4.83 (m, 1 H, $ArCH_2CHOCH_3$), 7.03 - 7.08 (m, 1 H, aromat. H), 7.16 - 7.42 (m, 3 H, aromat. H). Relation of diastereomers 51:49. |

**Examples 24 to 35 were prepared using analogous or similar procedures as in the preceeding examples 1 to 23 with example 24 being an intermediate.**

**Example 24 (intermediate):**

**Synthesis of 2-(1-Benzyl-3-hydroxypyrrolidin-3-yl)benzaldehyd-dimethylacetal**

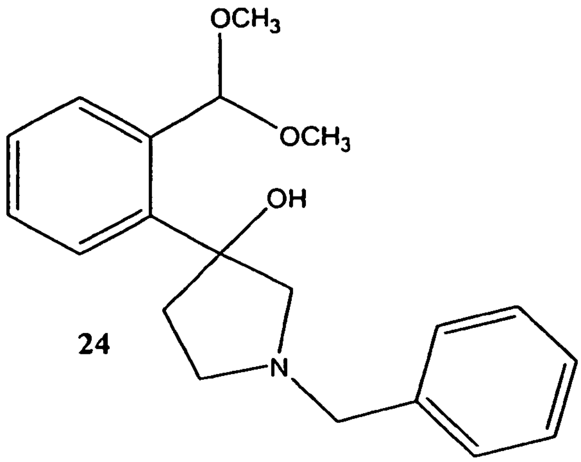

24

Then the crude product was purified by Flash-Chromatography (6 cm, 30 mL, Petrolether:Ethylacetat = 1:2, $R_f$ = 0.38).

Slightly yellow oil, Yield 1.65 g (51 %).

$C_{20}H_{25}NO_3$ (327.4)

| | |
|---|---|
| MS (EI): | m/z (%) = 327 [$M^+$], 312 [$M^+$ -$CH_3$], 296 [$M^+$ -$OCH_3$]. |
| IR: | $\tilde{\nu}$ ($cm^{-1}$) = 3437 (ν -OH), 2929/ 2799 (ν C-H), 1452 (δ -$CH_2$), 1070 (ν C-O), 752/ 698 (δ C-H monosubst. Benzene). |
| $^1$H-NMR ($CDCl_3$): | δ (ppm) = 2.28 - 2.42 (m 2 H, $CH_2CH_2N$), 2.60 - 2.68 (m, 1 H, $CH_2CH_2NCH_2$), 3.00 - 3.09 (m, 3 H, $CH_2CH_2NCH_2$ (2 H), $CH_2CH_2NCH_2$ (1 H)), 3.33 (s, 3 H, $CH(OCH_3)_2$), 3.37 (s, 3 H, $CH(OCH_3)_2$), 3.77 (s, 2 H, $NCH_2Ph$), 5.98 (s, 1 H, $ArCH(OCH_3)_2$), 7.26 - 7.39 (m, 8 H, aromat. H), 7.68 - 7.71 (m, 1 H, aromat. H). No Signal for proton of tert. Alcohol. |

**Example 25:**

**Synthesis of *cis*/*trans*-1'-Benzyl-3-methoxy-3*H*-spiro[[2]benzofuran-1,3'-pyrrolidin]**

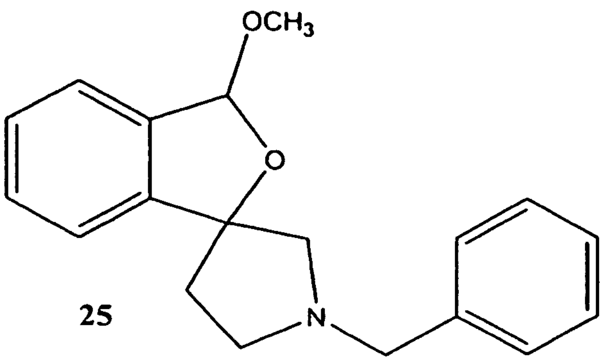

25

Then the crude product was purified by Flash-Chromatography (3 cm, 20 mL, Petrolether:Ethylacetat = 3:1, $R_f$= 0.31).

Slightly yellow oil, Yield 380 mg (92 %).

$C_{19}H_{21}NO_2$ (295.4)

| | |
|---|---|
| E: | Ber.: C 77.26; H 7.17; N 4.64<br>Gef.: C 77.03; H 7.11; N 4.74. |
| MS (EI): | m/z (%) = 295 [$M^+$], 264 [$M^+$ -$OCH_3$], 131 [$M^+$ -$OCH_3$. -$NBn(CH_2)_2$]. |
| IR: | $\tilde{v}$ ($cm^{-1}$) = 2905 (ν C-H), 1368 (δ -$CH_3$), 1081 (ν C-O), 750/ 697 (δ C-H monosubst. Benzene). |
| $^1$H-NMR ($CDCl_3$): | δ (ppm) = 2.24 - 2.35 (m, 2 H, $CH_2CH_2N$), 2.78 - 2.98 (m, 4 H, $CH_2CH_2NCH_2$), 3.43/3.46 (2 s, together 3 H, $OCH_3$ (cis, trans)), 3.66 - 3.77 (m, 2 H, $NCH_2Ph$), 6.06/6.07 (2 s, together 1 H, $ArCHOCH_3$ (cis, trans)), 7.23 - 7.40 (m, 9 H, aromat. H).<br>Relation of diastereomers 51:49. |

**Example 26:**

**Synthesis of *cis*/*trans*-3-Methoxy-3*H*-spiro[[2]benzofuran-1,3'-pyrrolidin) (26)**

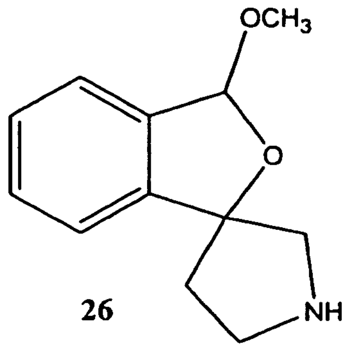

26

Then the mixture was filtered, the solvent completely removed in vacuo and the crude product purified by Flash-Chromatography (3 cm, 20 mL, Methanol:Ammonia = 98:2, $R_f$ = 0.48).

Clear oil, Yield 520 mg (87 %).

$C_{12}H_{15}NO_2$ (205.3)

| | |
|---|---|
| MS (EI): | m/z (%) = 205 [$M^+$], 174 [$M^+$ -$OCH_3$], 131 [$M^+$ -$OCH_3$, -$NBn(CH_2)_2$]. |
| IR: | $\tilde{v}$ ($cm^{-1}$) = 2932/ 2878 (ν C-H), 1368 (δ -$CH_3$), 1081 (ν C-O), 750 (δ C-H). |
| $^1$H-NMR ($CDCl_3$): | δ (ppm) = 2.12 - 2.33 (m, 2 H, $CH_2CH_2N$), 3.00 - 3.50 (m, 5 H, $CH_2NHCH_2$), 3.43/3.48 (2 s, together 3 H, $OCH_3$ (cis, trans)), 6.05/6.10 (2 s, together 1 H, $ArCHOCH_3$ (cis, trans)), 7.20 - 7.27 (2 d, J = 7.4 Hz, together 1 H, aromat. H (cis, trans)), 7.32 - 7.43 (m, 3 H, aromat. H).<br>Relation of diastereomers 52:48. |

**Example 27:**

**Synthesis of *cis*/*trans*-3-Methoxy-1'-(2-Phenylethyl)-3*H*-spiro [[2]benzofuran-1,3'-pyrrolidin]**

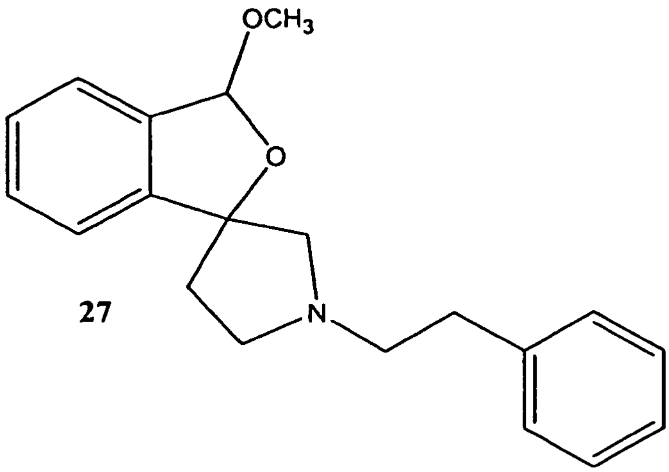

27

Method 1

Then the mixture was filtered, washed with $CH_2Cl_2$, the solvent completely removed in vacuo and the crude product purified by Flash-Chromatography (2 cm, 10 mL, Cyclohexan:Ethylacetat = 7:3, $R_f$= 0.28).
Slightly yellow oil, Yield 92.8 mg (30 %).

**Method 2 (Microwave)**

Then the mixture was filtered, washed with $CH_2Cl_2$, the solvent completely removed in vacuo and the crude product purified by Flash-Chromatography (3 cm, 20 mL, Cyclohexan:Ethylacetat = 7:3, $R_f$= 0.28).
Slightly yellow oil, Yield 171 mg (55 %).

$C_{20}H_{23}NO_2$ (309.4)

| | |
|---|---|
| E: | Ber.: C 77.24; H 7.56; N 4.47<br>Gef.: C 77.64; H 7.49; N 4.53. |
| MS (EI): | m/z (%) = 278 [$M^+$ -$OCH_3$], 218 [$M^+$ -$CH_2Ph$]. |
| IR: | $\tilde{\nu}$ ($cm^{-1}$) = 2927 (ν C-H), 1367 (δ -$CH_3$), 1081 (ν C-O), 749/ 698 (δ C-H monosubst. Benzene). |
| $^1$H-NMR ($CDCl_3$): | δ (ppm) = 2.29 - 2.36 (m, 2 H, C$H_2$C$H_2$N), 2.86 - 3.03 (m, 4 H, $CH_2$C$H_2$NC$H_2$), 3.03 - 3.12 (m, 4 H, NC$H_2$C$H_2$Ph), 3.48/3.49 (2 s, together 3 H, OC$H_3$ (cis, trans)), 6.07/6.09 (2 s, together 1 H, ArC*H*$OCH_3$ (cis, trans)), 7.20 - 7.40 (m, 9 H, aromat. H).<br>Relation cis:trans 53:47. |

**Example 28:**

**Synthesis of *cis*/*trans*-3-Methoxy-1'-(4-Phenylbutyl)-3*H*-spiro [[2]benzofuran-1,3'-pyrrolidin]**

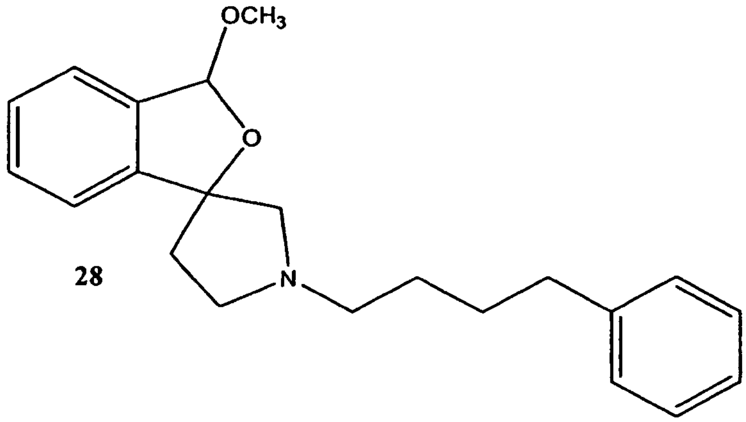

28

Method 1

Then the mixture was filtered, washed with $CH_2Cl_2$, the solvent completely removed in vacuo and the crude product purified by Flash-Chromatography (2 cm, 10 mL, Cyclohexan: Ethylacetat = 7:3, $R_f$ = 0.29).
Slightly yellow oil, Yield 230 mg (68 %).

**Method 2**

Then the mixture was filtered, washed with $CH_2Cl_2$, the solvent completely removed in vacuo and the crude product purified by Flash-Chromatography (3 cm, 20 mL, Cyclohexan:Ethylacetat = 7:3, $R_f$= 0.29).
Slightly yellow oil, Yield 253 mg (75 %).

$C_{22}H_{27}NO_2$ (337.5)

| | |
|---|---|
| E: | Ber.: C 78.30; H 8.06; N 4.15<br>Gef.: C 77.92; H 8.12; N 4.02. |
| MS (EI): | m/z (%) = 338 [$M^+$], 306 [$M^+$ -$OCH_3$], 218 [$M^+$ -$(CH_2)_3$Ph]. |
| IR: | $\tilde{\nu}$ ($cm^{-1}$) = 2932 (ν C-H), 1367 (δ -$CH_3$), 1082 (ν C-O), 747/ 698 (δ C-H monosubst. Benzene). |
| $^1$H-NMR ($CDCl_3$): | δ (ppm) = 1.51 - 1.53 (m, 2 H, $NCH_2CH_2(CH_2)_2$Ph), 1.60 - 1.63 (m, 2 H, $N(CH_2)_2CH_2CH_2$Ph), 2.15 - 2.30 (m, 2 H, $CH_2CH_2$N), 2.49 - 2.59 (m, 4 H, $CH_2CH_2NCH_2$), 2.60 - 2.91 (m, 4 H, $NCH_2(CH_2)_2CH_2$Ph), 3.39/3.40 (2 s, together 3 H, $OCH_3$ (cis, trans)), 5.98/6.00 (2 s, together 1 H, $ArCHOCH_3$ (cis, trans)), 7.10 - 7.32 (m, 9 H, aromat. H).<br>Relation of diastereomers 51:49. |

__Example 29:__

**Synthesis of *cis*/*trans*-1'-Butyl-3-methoxy-3*H*-spiro[[2]benzofuran-1,3'-pyrrolidin]**

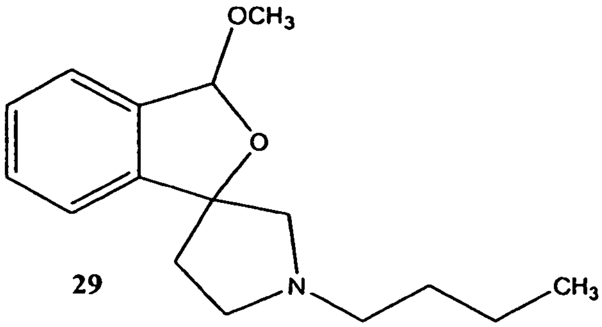

29

**Method 1**

Then the mixture was filtered, washed with $CH_2Cl_2$, the solvent completely removed in vacuo and the crude product purified by Flash-Chromatography (2 cm, 10 mL, Cyclohexan:Ethylacetat = 5:5, $R_f$= 0.19).
Slightly yellow oil, Yield 88.9 mg (34 %).

**Method 2**

Then the mixture was filtered, washed with $CH_2Cl_2$, the solvent completely removed in vacuo and the crude product purified by Flash-Chromatography (3 cm, 20 mL, Cyclohexan:Ethylacetat = 5:5, $R_f$= 0.19).
Slightly yellow oil, Yield 128 mg (49 %).

$C_{16}H_{23}NO_2$ (261.4)

| | |
|---|---|
| E: | Ber.: C 73.66; H 8.89; N 5.04<br>Gef.: C 73.53; H 8.87; N 5.36. |
| MS (EI): | m/z (%) = 261 [$M^+$], 230 [$M^+$ -$OCH_3$], 218 [$M^+$ -$(CH_2)_2CH_3$]. |
| IR: | $\tilde{v}$ ($cm^{-1}$) = 2929 (v C-H), 1367 (δ -$CH_3$), 1085 (v C-O), 754 (δ C-H). |
| $^1$H-NMR ($CDCl_3$): | δ (ppm) = 0.92/0.93 (2 t, J = 3.5 Hz, together 3 H, $CH_2CH_3$ (cis, trans)), 1.25 - 1.41 (2 sext, J = 7.2 Hz, together 2 H, $CH_2CH_2CH_3$ (cis, trans)), 1.44 - 1.60 (m, 2 H, $CH_2CH_2CH_3$), 2.27 (t, J = 7.0 Hz, 2 H, $CH_2CH_2N$), 2.50 - 2.60 (m, 2 H, $CH_2CH_2NCH_2$), 2.75 - 2.87 (m, 1 H, $NCH_2(CH_2)_2CH_3$), 2.93 - 3.03 (m, 3 H, $NCH_2(CH_2)_2CH_3$ (1 H), $CH_2CH_2NCH_2$ (2 H)), 3.46/3.48 (2 s, together 3 H, $OCH_3$ (cis, trans)), 6.05/6.07 (2 s, together 1 H, $ArCHOCH_3$ (cis, trans)), 7.32 - 7.39 (m, 4 H, aromat. H). Relation of diastereomers 52:48. |

**<u>Example 30:</u>**

**Synthesis of *cis*/*trans*-3-Methoxy-1'-octyl-3H-spiro[[2]benzofuran-1,3'-pyrrolidin]**

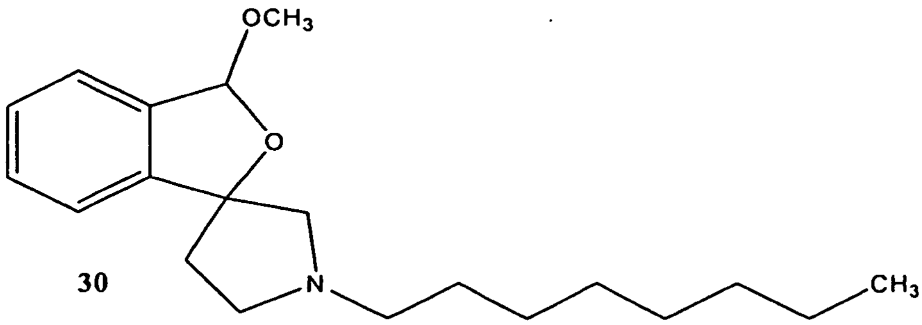

30

**Method 1**

Then the mixture was filtered, washed with $CH_2Cl_2$, the solvent completely removed in vacuo and the crude product purified by Flash-Chromatography (3 cm, 20 mL, Cyclohexan:Ethylacetat = 7:3, $R_f$= 0.36).
Slightly yellow oil, Yield 85.7 mg (27 %).

**Method 2**

Then the mixture was filtered, washed with $CH_2Cl_2$, the solvent completely removed in vacuo and the crude product purified by Flash-Chromatography (3 cm, 20 mL. Cyclohexan:Ethylacetat = 7:3, $R_f$= 0.36).
Slightly yellow oil, Yield 111 mg (35 %).

$C_{20}H_{31}NO_2$ (317.5)

| | |
|---|---|
| E: | Ber.: C 75.67; H 9.84; N 4.41<br>Gef.: C 75.03; H 10.01; N 4.26. |
| MS (EI) : | m/z (%) = 317 [$M^+$], 286 [$M^+$ -$OCH_3$], 218 [$M^+$ -$(CH_2)_6CH_3$]. |
| IR: | $\tilde{v}$ ($cm^{-1}$) = 2925 (v C-H), 1367 (δ -$CH_3$), 1082 (v C-O), 752 (δ C-H). |
| $^1$H-NMR ($CDCl_3$): | δ (ppm) = 0.85 - 0.89 (2 t, J = 6.7 Hz, together 3 H, $CH_2CH_3$ (cis, trans)), 1.22 - 1.38 (m, 10 H, $CH_2(CH_2)_5CH_3$), 1.50 - 1.60 (m, 2 H, $CH_2(CH_2)_5CH_3$), 2.22 - 2.28 (m, 2 H, $CH_2CH_2N$), 2.49 - 2.60 (m, 2 H, $CH_2CH_2NCH_2$), 2.72 - 2.83 (m, 1 H, $NCH_2(CH_2)_6CH_3$), 2.90 - 3.03 (m, 3 H, $NCH_2(CH_2)_6CH_3$ (1 H), $CH_2CH_2NCH_2$ (2 H)), 3.46/3.48 (2 s, together 3 H, $OCH_3$ (cis, trans)), 6.05/6.07 (2 s, together 1 H, $ArCHOCH_3$ (cis, trans)), 7.32 - 7.39 (m, 4 H, aromat. H).<br>Relation of diastereomers 57:43. |

<u>**Example 31:**</u>

**Synthesis of *cis/trans*-N,N-Cyclobutyl-3-methoxy-3*H*-spiro[[2]benzofuran-1,3'-pyrrolidiniumbromid]**

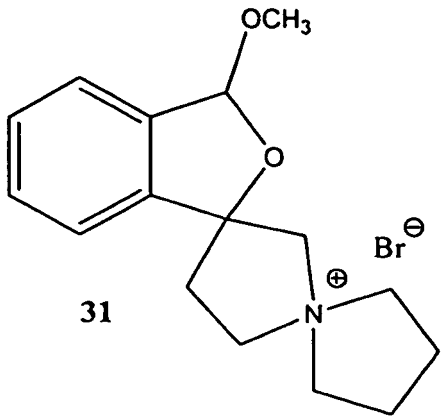

31

**Method 1**

White solid, Yield 280 mg (82 %).
$C_{16}H_{22}NO_2Br$ (340.3)

**Method 2**

White solid, Mp. 109°C, Yield 176 mg (91 %).
$C_{16}H_{22}NO_2I$ (387.3)

*Analysis of Bromide* $C_{16}H_{22}NO_2Br$ (340.3):

| | |
|---|---|
| MS (EI): | m/z (%) = 309 [$M^+$ -$OCH_3$], 260 [$M^+$ -$Br^-$], 186 [$M^+$ -$Br^-$, -$OCH_3$, -$(CH_2)_3$]. |
| IR: | $\tilde{\nu}$ ($cm^{-1}$) = 2977/ 2925 (ν C-H), 1367 (δ -$CH_3$), 1081 (ν C-O), 766 (δ C-H). |
| $^1$H-NMR ($CDCl_3$): | δ (ppm) = 2.20 - 2.35 (m, 4 H, $N^+CH_2(CH_2)_2CH_2$), 2.54 - 2.67 (m, 1 H, $CCH_2CH_2N$), 2.74 - 7.90 (m, 1 H, $CCH_2CH_2N$), 3.49 (s, 3 H, $OCH_3$ (cis or trans)), 3.55 (s, 3 H, $OCH_3$ (cis or trans)), 3.76 - 4.11 (m, 8 H, $N^+(CH_2)_4$), 6.16 (s, 1 H, $ArCHOCH_3$ (cis or trans)), 6.19 (s, 1 H, $ArCHOCH_3$ (cis or trans)), 7.44 - 7.62 (m, 4 H, aromat. H). Relation of diastereomers = 53:47. |

<u>**Example 32:**</u>

**Synthesis of N,N-Cyclobutyl-3-methoxy-3*H*-spiro[[2]benzofuran-1,4'-piperidiniumbromid]**

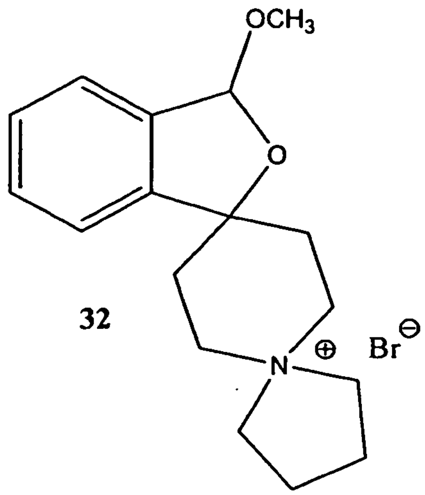

32

White solid, Mp. 124°C, Yield 173 mg (98 %).

$C_{17}H_{24}NO_2Br$ (354.3)

MS (EI): m/z (%) = 354 [$M^+$], 323 [$M^+$ -$OCH_3$].

IR: $\tilde{v}$ ($cm^{-1}$) = 2952 (ν C-H), 1373 (δ -$CH_3$), 1080 (ν C-O), 762 (δ C-H).

$^1$H-NMR ($CDCl_3$): δ (ppm) = 1.90 (dd, J = 54.4/15.3 Hz, 2 H, C(C$H_2$CH$_2$)$_2$N), 2.27 (s, 4 H, $N^+$(CH$_2$C$H_2$)$_2$), 2.74 - 7.55 (m, 2 H, C(C$H_2$CH$_2$)$_2$N), 3.53 (s, 3 H, OC$H_3$), 3.58 - 3.86 (m, 8 H, $N^+$(C$H_2$)$_4$), 6.13 (s, 1 H, ArC*H*OCH$_3$), 7.41 - 7.48 (m, 4 H, aromat. H).

**Example 33 (intermediate):**

**Synthesis of 1-(4-Chlorbutyl)-1*H*-imidazol (Intermediate)**

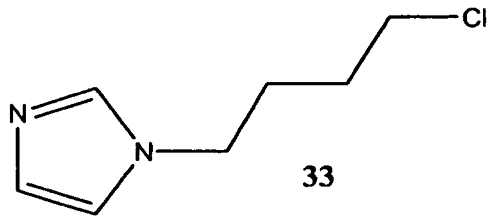

33

Then the crude product was purified by Flash-Chromatography (5 cm, 30 mL, Ethylacetat:Methanol = 9:1, $R_f$= 0.31).

Slightly yellow oil, Yield 1.31 g (83 %).

$C_7H_{11}N_2Cl$ (158.6)

MS (EI): m/z (%) = 159 [$M^+$], 131 [$M^+$ -CHN], 123 [$M^+$ -CHN, -Cl], 82 [$M^+$ -$(CH_2)_3Cl$].

IR: $\tilde{v}$ ($cm^{-1}$)) = 2945 (ν C-H), 1699 (ν C=N), 737 (ν C-Cl).

$^1$H-NMR ($CDCl_3$): δ (ppm) = 1.72 - 1.78 (m, 2 H, N(CH$_2$)$_2$C$H_2$), 1.91 - 1.96 (m, 2 H, NCH$_2$C$H_2$), 3.52 (t, J = 6.3 Hz, 2 H, C$H_2$Cl), 3.98 (t, J = 7.0 Hz, 2 H, NC$H_2$), 6.90 (s, 1 H, CH$_2$NC*H*CH), 7.05 (s, 1 H, CH$_2$NCHC*H*), 7.50 (s, 1 H, NC*H*N).

**Example 34:**

**Synthesis of *cis*/*trans*-1'-(4-(1*H*-Imidazol-1-yl)-butyl)-3-methoxy-3*H*-spiro[[2]benzofuran-1,3'-pyrrolidin]**

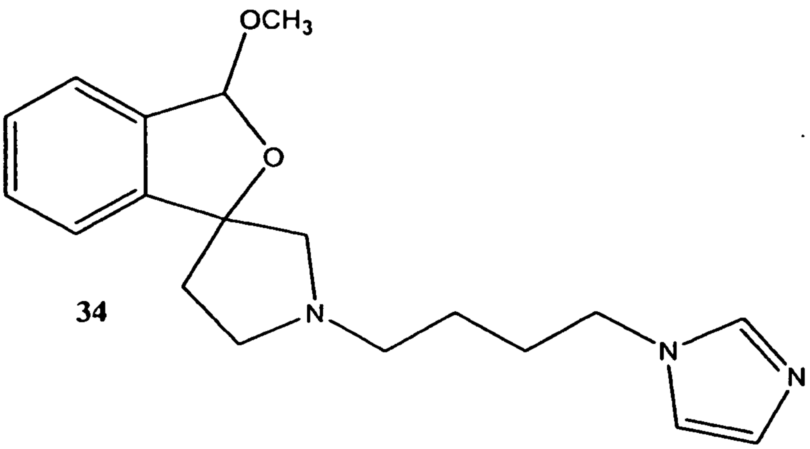

34

Clear oil, Yield 223 mg (65 %).

$C_{19}H_{25}N_3O_2$ (327.4)

| | |
|---|---|
| E: | Ber.: C 69.70; H 7.70; N 12.83<br>Gef.: C 69.60; H 7.82; N 11.79. |
| MS (EI): | m/z (%) = 327 [$M^+$], 296 [$M^+$ -$OCH_3$], 218 [$M^+$ -$(CH_2)_3$ -$C_3H_3N_2$]. |
| IR: | $\tilde{v}$ ($cm^{-1}$)) = 2935 (v C-H), 1367 ($\delta$ -$CH_3$), 1080 (v C-O), 756 ($\delta$ C-H). |
| $^1$H-NMR ($CDCl_3$): | $\delta$ (ppm) = 1.49 - 1.58 (m, 2 H, C$H_2$$(CH_2)_2$Imid), 1.81 - 1.92 (m, 2 H, C$H_2$$CH_2$Imid), 2.18 - 2.35 (m, 2 H, C$H_2$$CH_2$N), 2.53 - 2.57 (m, 2 H, $CH_2$C$H_2$N), 2.66 - 2.78 (m, 1 H, C$H_2$$(CH_2)_3$Imid), 2.82 - 2.95 (m, 3 H, C$H_2$$(CH_2)_3$Imid (1 H), $CH_2CH_2$NC$H_2$ (2 H)), 3.45/3.47 (2 s, together 3 H, $OCH_3$ (cis, trans)), 3.92 - 3.99 (m, 2 H, $(CH_2)_3$C$H_2$Imid), 6.04/6.07 (2 s, together 1 H, ArC*H*$OCH_3$ (cis, trans)), 6.91 (s, 1 H, $CH_2$NC*H*CH), 7.04 (s, 1 H, $CH_2$NCHC*H*), 7.25 - 7.46 (m, 4 H, aromat. H), 7.50 (s, 1 H, NC*H*N). |

**Example 35:**

**Synthesis of 1'-(4-(1*H*-Imidazol-1-yl)-butyl)-3-methoxy-3*H*-spiro[[2]benzofuran-1,4'-piperidin]**

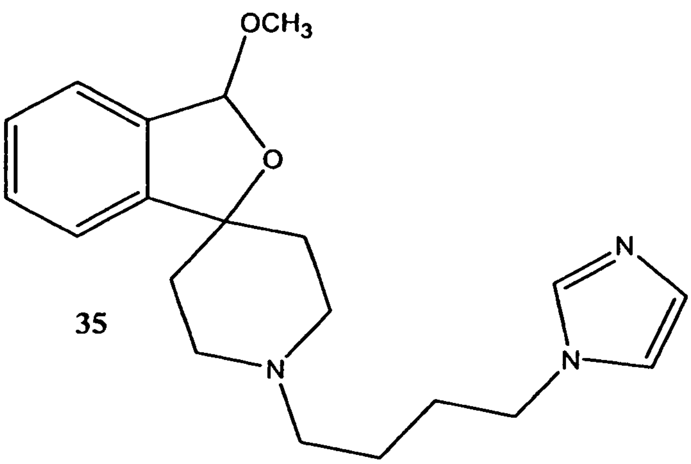

35

Clear oil, Yield 136 mg (43 %).

$C_{20}H_{27}N_3O_2$ (341.5)

| | |
|---|---|
| E: | Ber.: C 70.35; H 7.97; N 12.31<br>Gef.: C 69.60; H 7.82; N 11.79. |
| MS (EI): | m/z (%) = 310 [$M^+$ -$OCH_3$], 232 [$M^+$ -$(CH_2)_3C_3H_3N_2$]. |
| IR: | $\tilde{v}$ ($cm^{-1}$)) = 2943 (v C-H), 1376 (δ -$CH_3$), 1080 (v C-O), 758 (δ C-H). |
| $^1$H-NMR ($CDCl_3$): | δ (ppm) = 1.58 - 1.68 (m, 3 H, $CH_2(CH_2)_2$Imid (2 H), $C(CH_2CH_2)_2N$ (1 H)), 1.81 - 1.86 (m, 3 H, $CH_2CH_2$Imid (2 H), $C(CH_2CH_2)_2N$ (1 H)), 1.98 - 2.23 (m, 2 H, $C(CH_2CH_2)_2N$), 2.48 - 2.55 (m, 4 H, $C(CH_2CH_2)_2N$), 2.86 - 2.97 (m, 2 H, $CH_2(CH_2)_3$Imid), 3.46 (s, 3 H, $OCH_3$), 3.98 (t, J = 7.0 Hz, 2 H, $(CH_2)_3CH_2$Imid), 6.06 (s, 1 H, ArCHOCH₃), 6.92 (s, 1 H, $CH_2$NCHCH), 7.05 (s, 1 H, $CH_2$NCHCH), 7.17 - 7.38 (m, 4 H, aromat. H), 7.48 (s, 1 H, NCHN). |

**Example 36:**

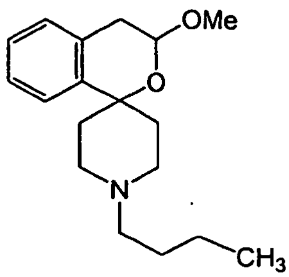

**Example 37:**

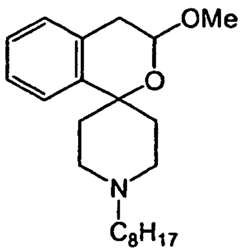

Examples 36 and 37 were prepared analogously to the processes described in Maier et Wünsch; J.Med.Chem. 2002, 45, 438-448 or Maier et Wünsch; J.Med.Chem. 2002, 45, 4923-4930.

**BIOLOGICAL ACTIVITY**

**A) In-Vitro**

Some representative compounds of the invention were tested for their activity as sigma (sigma-1 and sigma-2) inhibitors. The following protocols were followed:

**<u>Sigma-1 (Version A=</u>**

Brain membrane preparation and binding assays for the σ1-receptor were performed as described (DeHaven-Hudkins et al., 1992) with some modifications. In brief, guinea pig brains were homogenized in 10 vols. (w/v) of Tris-HCl 50 mM 0.32 M sucrose, pH 7.4, with a Kinematica Polytron PT 3000 at 15000 r.p.m. for 30 s. The homogenate was centrifuged at 1000g for 10 min at 4°C and the supernatants collected and centrifuged again at 48000g for 15 min at 4°C. The pellet was resuspended in 10 volumes of Tris-HCl buffer (50 mM, pH 7.4), incubated at 37°C for 30 min, and centrifuged at 48000g for 20 min at 4°C. Following this, the pellet was resuspended in fresh Tris-HCl buffer (50 mM, pH 7.4) and stored on ice until use.
Each assay tube contained 10 μL of [$^3$H](+)-pentazocine (final concentration of 0.5 nM), 900 μL of the tissue suspension to a final assay volume of 1 mL and a final tissue concentration of approximately 30 mg tissue net weight/mL. Non-specific binding was defined by addition of a final concentration of 1 μM haloperidol. All tubes were incubated at 37°C for 150 min before termination of the reaction by rapid filtration over Schleicher & Schuell GF 3362 glass fibre filters [previously soaked in a solution of 0,5% polyethylenimine for at least 1 h]. Filters were then washed with four times with 4 mL of cold Tris-HCl buffer (50 mM, pH 7.4). Following addition of scintillation cocktail, the samples were allowed to equilibrate overnight. The amount of bound radioactivity was determined by liquid scintillation spectrometry using a Wallac Winspectral 1414 liquid scintillation counter. Protein concentrations were determined by the method of Lowry et al. (1951).

**Sigma 1 (Version B)**

In brief the $\sigma_1$-receptor preparation was prepared from guinea pig brain. The brains were homogenized in 5 to 6 times of volume sucrose solution (0.32M) and homogenized. The homogenate was centrifuged at 2900 rpm, 4°C, 10 min). the supernatant was centrifuged again (23500 x g, 4°C, 20 min). The pellet was resuspended in Tris-buffer, incubated for 30 min at room temperature and centrifuged f (23500 x g, 4°C, 20 min). The pellet was resuspended in cold TRIS-buffer and homogenized. Then the protein content was measured (approx. 1.5 mg/mL) and the homogenate frozen at -80°C for later use.
The radioligand used was [$^3$H]-(+)-Pentazocin in TRIS-buffer. In a volume of 200 μL 50 μL TRIS-buffer, 50 μL compound solution of varying concentration, 50 μL radioligand- solution (8 nM; resulting in 2 nM in the assay) and finally 50 μL of receptor preparation (approx. 1.5 mg /mL) were given into a well of a microplate equipped with a filter. The plate was closed and stirred for 2.5 h at 37 °C and 500 rpm. Following that the solvents were removed by a harvester through the filter. After rinsing with $H_2O$ the filter was measured in a scintillation counter ([$^3$H]-protocol).

**Sigma-2 (Version A)**

Binding studies for σ 2-receptor were performed as described (Radesca et al., 1991) with some modifications. In brief, brains from sigma receptor type I (σ1) knockout mice were homogenized in a volume of 10 mUg tissue net weight of ice-cold 10 mM Tris-HCl, pH 7.4, containing 320 mM sucrose (Tris-sucrose buffer) with a Potter-Elvehjem homogenizer (10 strokes at 500 r.p.m.) The homogenates were then centrifuged at 1000g for 10 min at 4°C, and the supernatants were saved. The pellets were resuspended by vortexing in 2 mUg ice-cold Tris-sucrose buffer and centrifuged again at 1000g for 10 min. The combined 1000g supernatants were centrifuged at 31000g for 15 min at 4°C. The pellets were resuspended by vortexing in 3 mL/g 10 mM Tris-HCl, pH 7.4, and the suspension was kept at 25°C for 15 min. Following centrifugation at 31000g for 15 min, the pellets were resuspended by gentle Potter Elvehjem homogenization to a volume of 1.53 mUg in 10 mM Tris-HCl pH 7.4.
The assay tubes contained 10 μL of [$^3$H]-DTG (final concentration of 3 nM), 400 μL of the tissue suspension (5.3 mUg in 50 mM Tris-HCl, pH 8.0) to a final assay volume of 0.5 mL. Non-specific binding was defined by addition of a final concentration of 1 μM haloperidol. All tubes were incubated at 25°C for 120 min before termination of the reaction by rapid filtration over Schleicher & Schuell GF 3362 glass fibre filters [previously soaked in a solution of 0,5% polyethylenimine for at least 1 h]. Filters were washed with three times with 5 mL volumes of cold Tris-HCl buffer (10 mM, pH 8.0). Following addition of scintillation cocktail samples were allowed to equilibrate overnight. The amount of bound radioactivity was determined by liquid scintillation spectrometry using a Wallac Winspectral 1414 liquid scintillation counter. Protein concentrations were determined by the method of Lowry et al. (1951).

**References**

DeHaven-Hudkins, D. L., L.C. Fleissner, and F. Y. Ford-Rice, 1992, "Characterization of the binding of [3H](+) pentazocine to σ recognition sites in guinea pig brain", Eur. J. Pharmacol. 227, 371-378.
Radesca, L., W.D. Bowen, and L. Di Paolo, B.R. de Costa, 1991, Synthesis and Receptor Binding of Enantiomeric N-Substituted cis-N-[2-(3,4-Dichlorophenyl)ethyl]-2-(1-pyrrolidinyl)cyclohexylamines as High-Affinity σ Receptor Ligands, J. Med. Chem. 34, 3065-3074.
Langa, F., Codony X., Tovar V., Lavado A., Giménez E., Cozar P., Cantero M., Dordal A., Hernández E., Pérez

R., Monroy X., Zamanillo D., Guitart X., Montoliu L, 2003, Generation and phenotypic analysis of sigma receptor type I (Sigma1) knockout mice, European Journal of Neuroscience, Vol. 18, 2188-2196.
Lowry, O.H., N.J. Rosebrough, A.L. Farr, and R.J. Randall, 1951, Protein measurement with the Folin phenol reagent, J. Biol. Chem, 193, 265.

**SIGMA 2 (Version B)**

In brief the $\sigma_2$-Receptorpreparation was prepared from rat liver. The livers were homogenized in 5 to 6 times of volume sucrose solution (0.32M) and homogenized.

The homogenate was centrifuged at 2900 rpm, 4°C, 10 min). the supernatant was centrifuged again (31000 x g, 4°C, 20 min). The pellet was resuspended in TRIS-buffer, incubated for 30 min at room temperature while stirring and centrifuged f (31000 x g, 4°C, 20 min). The pellet was resuspended in cold TRIS-buffer pH 8 and homogenized. Then the protein content was measured (approx. 2 mg/mL) and the homogenate frozen at -80°C for later use.

The radioligand used was [$^3$H]-Ditolylguanidin in TRIS-buffer pH 8. The $\sigma_1$-receptor binding sites were masked through (+)-Pentazocin-solution in TRIS-Puffer pH 8.

In a volume of 200 µL 50 µL compound solution of varying concentration, 50 µL (+)-Pentazocin- solution (2 µM; resulting in 500 nM in the assay), 50 µL radioligand-solution (12 nM; resulting in 3 nM in the assay) and finally 50 µL of receptor preparation (approx. 2 mg /mL) were given into a well of a microplate equipped with a filter. The plate was closed and stirred for 2 h at room temperature and 500 rpm. Following that the solvents were removed by a harvester through the filter. After rinsing with $H_2O$ the filter was measured in a scintillation counter ([$^3$H]-protocol).

Some of the results obtained are shown in table (I).

**Table (I)**

| Example | % Binding σ1 $10^{-6}$ M (Ki nM) | % Binding σ1 $10^{-7}$ M (Ki nM) | % Binding σ1 $10^{-8}$ M (Ki nM) | % Binding σ2 $10^{-6}$M (Ki nM) |
|---|---|---|---|---|
| 18 | 96.2 (70) | | | |
| 1 | | 34 | | |
| 2 | 12.9 | | | 27.8 |
| 10 | 56 | | | 7.6 |
| 11 | 49 | | | 18.8 |
| 14 | 46.3 | | | 45.4 |
| 15 | 27.6 | | | 56.3 |
| 6 | 38.8 | | | 32.2 |
| 7 | 14 | | | 54.3 |
| 20 | | 89.5 | 32 | |
| 21 | | 67 | 32 | |
| 22 | | 52 | 29 | |
| 23 | | 70.4 | 0.8 | |
| 25 | | 88 | 22.2 | |
| 27 | | 92.2 | 40.4 | |
| 28 | | 80.4 | 27.4 | |
| 29 | | 68.9 | 45.6 | |
| 30 | | 91.5 | 41.8 | |
| 36 | 100.8 (2.3) | | | 39.7 |
| 37 | 103.9 | | | 72.6 |

**B) In-Vivo**

**EFFECT ON CAPSAICIN IN DEVELOPMENT OF MECHANICAL ALLODYNIA**

This model uses the von-Frey Filaments and is a model to test the effects or symptoms of neuropathic pain, allodynia etc.

Interest of the model:

- The injection of 1 μg of capsaicin to experimental animals produces acute pain followed by hyperalgesia/allodynia
- The mechanisms involved in capsaicin-induced acute pain and hyperalgesia are relatively well known (mainly activation of peripheral nociceptors and sensitization of spinal cord neurons, respectively)

The test protocol for all tests with von Frey filaments: After habituation mice were first treated with the test-compound (or solvent in controls). Then 1 μg capsaicin (1% DMSO) is injected into their paw resulting in developing pain in the effected paw. The effected paw is then treated with a mechanical stimulus and the latency time before the paw is withdrawn is measured.

Using this pharmacological test the compounds according to example 22 and example 25 showed a clear antiallodynic effect. They were tested at 16 mg/kg i.p. in 8 animals each and respectively achieved 45.67 ± 9.62 % and 44.28 ± 9.49 % of pain relief.

**Claims**

**1.** Compound of general formula Ia,

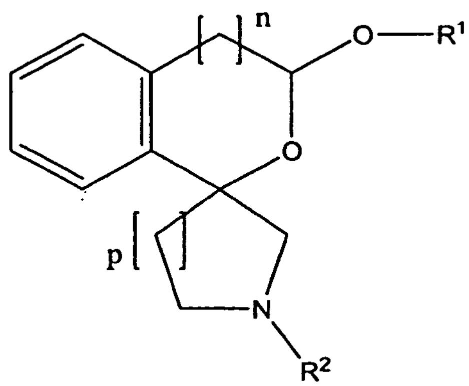

**Ia**

wherein

n is selected from 0 or 1;
p is selected from 1 or 2;
$R^1$ is selected from H, $C_{1-6}$-alkyl;
$R^2$ is selected from hydrogen; optionally at least mono-substituted $C_{1-18}$-aliphatic group, saturated or unsaturated, linear or branched; optionally at least mono-substituted $(CH_2)_{1-6}$-aryl, $(CH_2)_{1-6}$-heteroaryl, $(CH_2)_{1-6}$-cycloalkyl, cycloalkyl, aryl, or heterocyclyl;
or $R^2$ together with the bonded Nitrogen is binding the nitrogen in a cycloalkyl-spiro-complex thus forming a quarternary ammonium according to formula X (with q being 1 or 2):

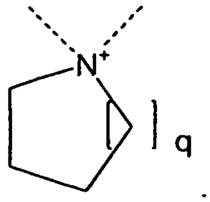

**2.** Compound according to claim 1, having a general formula II,

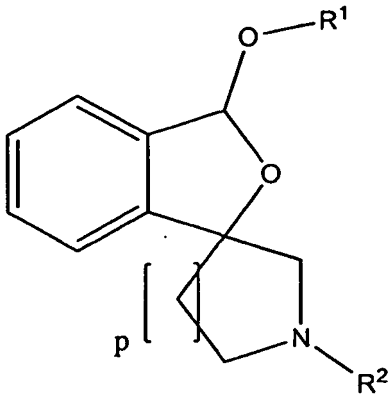

II

wherein

p is selected from 1 or 2;
$R^1$ is selected from H, $C_{1-6}$-alkyl;
$R^2$ is selected from hydrogen; optionally at least mono-substituted $C_{1-18}$-aliphatic group, saturated or unsaturated, linear or branched; optionally at least mono-substituted $(CH_2)_{1-6}$ -aryl, $(CH_2)_{1-6}$-heteroaryl, $(CH_2)_{1-6}$-cycloalkyl, cycloalkyl, aryl, or heterocyclyl;
or $R^2$ together with the bonded Nitrogen is binding the nitrogen in a cycloalkyl-spiro-complex thus forming a quarternary ammonium according to formula X (with q being 1 or 2):

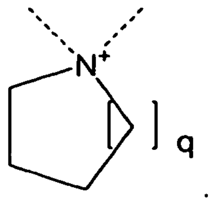

**3.** Compound according to claim 1, having a general formula III,

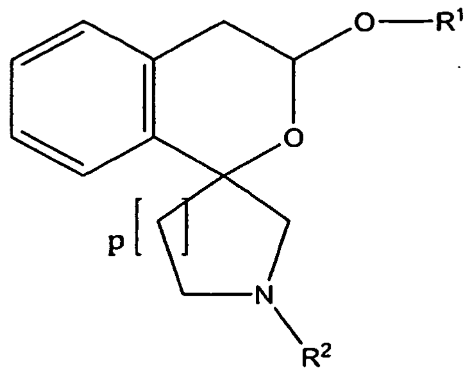

III

wherein

p is selected from 1 or 2;
$R^1$ is selected from H, $C_{1-6}$-alkyl;
$R^2$ is selected from hydrogen; optionally at least mono-substituted $C_{1-18}$-aliphatic group, saturated or unsaturated, linear or branched; optionally at least mono-substituted $(CH_2)_{1-6}$-aryl, $(CH_2)_{1-6}$-heteroaryl, $(CH_2)_{1-6}$-cycloalkyl, cycloalkyl, aryl, or heterocyclyl;
or $R^2$ together with the bonded Nitrogen is binding the nitrogen in a cycloalkyl-spiro-complex thus forming a quarternary ammonium according to formula X (with q being 1 or 2):

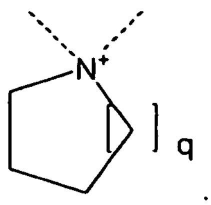

**4.** Compound according to claim 2, having any one of general formulas IIa or IIb,

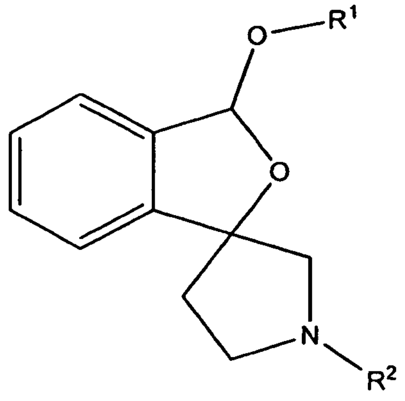

IIa

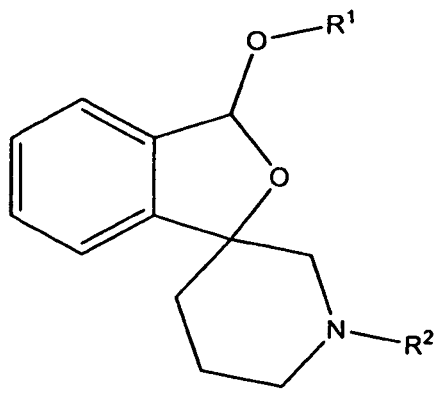

IIb

wherein

$R^1$ is selected from H, $C_{1-6}$-alkyl;
$R^2$ is selected from hydrogen; optionally at least mono-substituted $C_{1-18}$-aliphatic group, saturated or unsaturated, linear or branched; optionally at least mono-substituted $(CH_2)_{1-6}$-aryl, $(CH_2)_{1-6}$-heteroaryl, $(CH_2)_{1-6}$-cycloalkyl, cycloalkyl, aryl, or heterocyclyl;

or $R^2$ together with the bonded Nitrogen is binding the nitrogen in a cycloalkyl-spiro-complex thus forming a quarternary ammonium according to formula X (with q being 1 or 2):

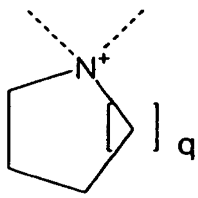

.

5. Compound according to claim 3, having any one of general formulas IIIa or IIIb,

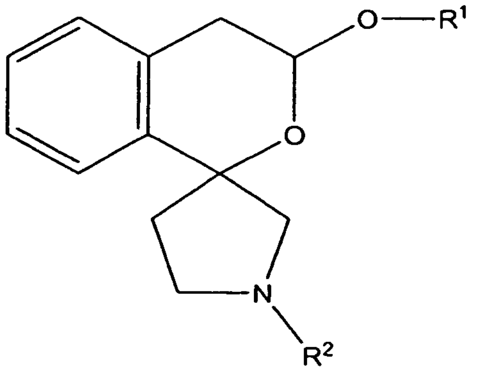

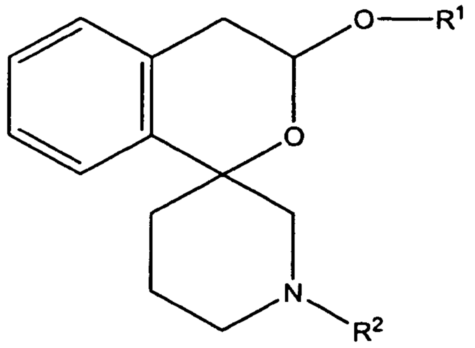

IIIa IIIb

wherein

$R^1$ is selected from H, $C_{1-6}$-alkyl;

$R^2$ is selected from hydrogen; optionally at least mono-substituted $C_{1-18}$-aliphatic group, saturated or unsaturated, linear or branched; optionally at least mono-substituted $(CH_2)_{1-6}$-aryl, $(CH_2)_{1-6}$-heteroaryl, $(CH_2)_{1-6}$-cycloalkyl, cycloalkyl, aryl, or heterocyclyl;

or $R^2$ together with the bonded Nitrogen is binding the nitrogen in a cycloalkyl-spiro-complex thus forming a quarternary ammonium according to formula X (with q being 1 or 2):

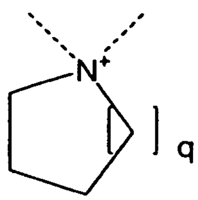

.

6. Compound according to any of claims 1 to 5, **characterized in that** $R^1$ is selected from H, $CH_3$ or $C_2H_5$.

7. Compound according to any of claims 1 to 6, **characterized in that** $R^2$ is selected from

hydrogen; optionally at least mono-substituted $C_{1-18}$-aliphatic-group, saturated or unsaturated, linear or branched; optionally at least mono-substituted aryl or $(CH_2)_{2-6}$-aryl; optionally at least mono-substituted heteroaryl or $(CH_2)_{1-6}$-heteroaryl;

or **characterized in that** $R^2$ is selected from

hydrogen; optionally at least mono-substituted $C_{4-12}$-aliphatic-group, saturated or unsaturated, linear or branched; optionally at least mono-substituted $(CH_2)_{1-6}$-aryl, optionally at least mono-substituted $(CH_2)_{1-6}$-alkyl-heteroaryl;

or **characterized in that** $R^2$ forms together with the bonded Nitrogen a cycloalkyl-spiro-complex thus forming a quarternary ammonium according to formula X (with q being 1 or 2):

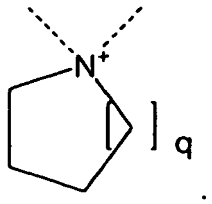

**8.** Compound according to claim 7, wherein $R^2$ is selected from hydrogen, optionally at least mono-substituted $C_{1-18}$-aliphatic-group, saturated or unsaturated, linear or branched, substituted or unsubstituted; optionally at least mono-substituted $(CH_2)_{1-6}$-aryl; optionally at least mono-substituted $(CH_2)_{1-6}$-heteroaryl.

**9.** Compound according to claim 7, wherein $R^2$ is selected from hydrogen; optionally at least mono-substituted $C_{4-10}$-aliphatic group, saturated or unsaturated, linear or branched, substituted or unsubstituted; $(CH_2)_{1-4}$-aryl, substituted or unsubstituted; $(CH_2)_{1-4}$-heteroaryl, substituted or unsubstituted, or $R^2$ is selected from optionally at least mono-substituted, linear $C_{4-10}$-alkyl or optionally at least mono-substituted $(CH_2)_{1-4}$-alkyl-heteroaryl.

**10.** Compound according to any of claims 1 to 9, **characterized in that** the compound is selected from

1'-Benzyl-3-methoxy-3,4-dihydrospiro[[2]benzopyran-1,3'-piperidin] (1) (2)
3-Methoxy-1'-(2-phenylethyl)-3,4-dihydrospiro[[2]benzopyran-1,3'-piperidin] (4) (5)
3-Methoxy-1'-(3-phenylpropyl)-3,4-dihydrospiro[[2]benzopyran-1,3'-piperidin] (6) (7)
3-Methoxy-1'-(4-phenylbutyl)-3,4-dihydrospiro[[2]benzopyran-1,3'-piperidin] (8) (9)
1'-Butyl-3-methoxy-3,4-dihydrospiro[[2]benzopyran-1,3'-piperidin] (10) (11)
1'-Hexyl-3-methoxy-3,4-dihydrospiro[[2]benzopyran-1,3'-piperidin] (12) (13)
3-Methoxy-1'-octyl-3,4-dihydrospiro[[2]benzopyran-1,3'-piperidin] (14) (15)
1'-Decyl-3-methoxy-3,4-dihydrospiro[[2]benzopyran-1,3'-piperidin] (16) (17)
1'-Benzyl-3-methoxy-3,4-dihydrospiro[[2]benzopyran-1,3'-pyrrolidin] (18)
3-Methoxy-1'-(2-phenylethyl)-3,4-dihydrospiro[[2]benzopyran-1,3'-pyrrolidin] (20)
3-Methoxy-1'-(4-phenylbutyl)-3,4-dihydrospiro[[2]benzopyran-1,3'-pyrrolidin] (21)
1'-Butyl-3-methoxy-3,4-dihydrospiro[[2]benzopyran-1,3'-pyrrolidin] (22)
3-Methoxy-1'-octyl-3,4-dihydrospiro[[2]benzopyran-1,3'-pyrrolidin] (23)
1'-Benzyl-3-methoxy-3*H*-spiro[[2]benzofuran-1,3'-pyrrolidin] (25)
3-Methoxy-1'-(2-Phenylethyl)-3*H*-spiro [[2]benzofuran-1,3'-pyrrolidin] (27)
3-Methoxy-1'-(4-Phenylbutyl)-3*H*-spiro [[2]benzofuran-1,3'-pyrrolidin] (28)
1'-Butyl-3-methoxy-3*H*-spiro[[2]benzofuran-1,3'-pyrrolidin] (29)
3-Methoxy-1'-octyl-3*H*-spiro[[2]benzofuran-1,3'-pyrrolidin] (30)
N,N-Cyclobutyl-3-methoxy-3*H*-spiro[[2]benzofuran-1,3'-pyrrolidiniumbromid] (31) or
1'-(4-(1*H*-Imidazol-1-yl)-butyl)-3-methoxy-3*H*-spiro[[2]benzofuran-1,3'-pyrrolidin] (34);

optionally in form of one of the stereoisomers, enantiomers or diastereomers, a racemate or in form of a mixture of at least two of the stereoisomers, enantiomers and/or diastereomers, in any mixing ratio, or a corresponding salt thereof, or a corresponding solvate thereof.

**11.** A pharmaceutical composition which comprises a compound as defined in any of claims 1 to 10 or a pharmaceutically acceptable salt, or solvate thereof, and a pharmaceutically acceptable carrier, adjuvant or vehicle.

**12.** Use of a compound as defined in any of claims 1 to 10 in the manufacture of a medicament for the treatment or prophylaxis of a sigma receptor mediated disease or condition.

**13.** Use according to claim 12, wherein the disease is diarrhoea, lipoprotein disorders, metabolic syndrome, treatment

of elevated triglyceride levels, chylomicronemia, hyperlipoproteinemia; hyperlipidemia, especially mixed hyperlipidemia; hypercholesterolemia, dysbetalipoproteinemia, hypertriglyceridemia including both the sporadic and familial disorder (inherited hypertriglyceridemia), migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine, tardive diskinesia, ischemic stroke, epilepsy, stroke, depression, stress, psychotic condition, schizophrenia; inflammation, autoimmune diseases or cancer; or
wherein the disease is pain, neuropathic pain, inflammatory pain or other pain conditions, allodynia and/or hyperalgesia, or mechanical allodynia.

**14.** A compound as defined in any of claims 1 to 10 for use in the treatment or for the prophylaxis of a sigma receptor mediated disease or condition.

**15.** A compound as defined in any of claims 1 to 10 for use in the treatment or for the prophylaxis of a sigma receptor mediated disease or condition wherein the disease is diarrhoea, lipoprotein disorders, metabolic syndrome, treatment of elevated triglyceride levels, chylomicronemia, hyperlipoproteinemia; hyperlipidemia, especially mixed hyperlipidemia; hypercholesterolemia, dysbetalipoproteinemia, hypertriglyceridemia including both the sporadic and familial disorder (inherited hypertriglyceridemia), migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine, tardive diskinesia, ischemic stroke, epilepsy, stroke, depression, stress, psychotic condition, schizophrenia; inflammation, autoimmune diseases or cancer; or
wherein the disease is pain, neuropathic pain, inflammatory pain or other pain conditions, allodynia and/or hyperalgesia, or mechanical allodynia.

## Patentansprüche

**1.** Verbindung der allgemeinen Formel Ia,

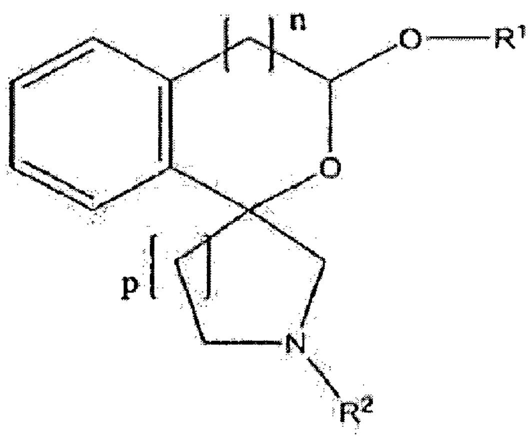

**Ia**

wobei

n ausgewählt ist aus 0 oder 1;
p ausgewählt ist aus 1 oder 2;
$R^1$ ausgewählt ist aus H, $C_{1-6}$-Alkyl;
$R^2$ ausgewählt ist aus Wasserstoff; wahlweise einer gesättigten oder ungesättigten, linearen oder verzweigten, mindestens monosubstituierten aliphatischen $C_{1-18}$-Gruppe; wahlweise mindestens monosubstituiertem $(CH_2)_{1-6}$-Aryl, $(CH_2)_{1-6}$-Heteroaryl, $(CH_2)_{1-6}$-Cycloalkyl, Cycloalkyl, Aryl oder Heterocyclyl;
oder $R^2$ zusammen mit dem gebundenen Stickstoff den Stickstoff in einem Cycloalkyl-Spirokomplex bindet und so ein quartäres Ammonium der Formel X (wobei q 1 oder 2 ist) bildet:

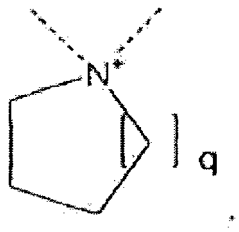

**2.** Verbindung nach Anspruch 1 mit der allgemeinen Formel II,

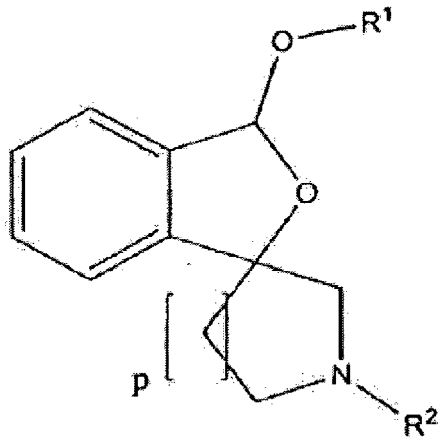

II

wobei

p ausgewählt ist aus 1 oder 2;
$R^1$ ausgewählt ist aus H, $C_{1-6}$-Alkyl;
$R^2$ ausgewählt ist aus Wasserstoff; wahlweise einer gesättigten oder ungesättigten, linearen oder verzweigten, mindestens monosubstituierten aliphatischen $C_{1-18}$-Gruppe; wahlweise mindestens monosubstituiertem $(CH_2)_{1-6}$-Aryl, $(CH_2)_{1-6}$-Heteroaryl, $(CH2)_{1-6}$-Cycloalkyl, Cycloalkyl, Aryl oder Heterocyclyl;
oder $R^2$ zusammen mit dem gebundenen Stickstoff den Stickstoff in einem Cycloalkyl-Spirokomplex bindet und so ein quartäres Ammonium der Formel X (wobei q 1 oder 2 ist) bildet:

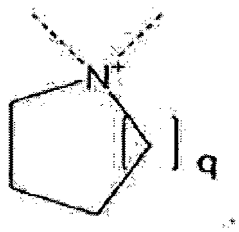

**3.** Verbindung nach Anspruch 1 mit der allgemeinen Formel III,

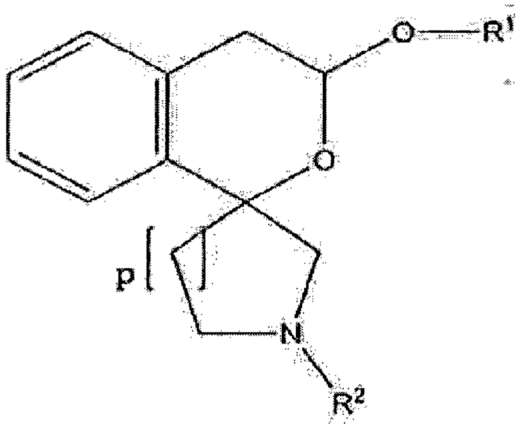

III

wobei

p ausgewählt ist aus 1 oder 2;
$R^1$ ausgewählt ist aus H, $C_{1-6}$-Alkyl;
$R^2$ ausgewählt ist aus Wasserstoff; wahlweise einer gesättigten oder ungesättigten, linearen oder verzweigten, mindestens monosubstituierten aliphatischen $C_{1-18}$-Gruppe; wahlweise mindestens monosubstituiertem $(CH_2)_{1-6}$-Aryl, $(CH_2)_{1-6}$-Heteroaryl, $(CH_2)_{1-6}$-Cycloalkyl, Cycloalkyl, Aryl oder Heterocyclyl;
oder $R^2$ zusammen mit dem gebundenen Stickstoff den Stickstoff in einem Cycloalkyl-Spirokomplex bindet und so ein quartäres Ammonium der Formel X (wobei q 1 oder 2 ist) bildet:

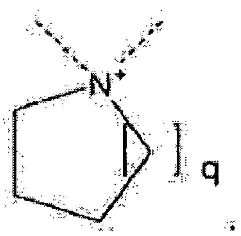

**4.** Verbindung nach Anspruch 2 mit einer der allgemeinen Formeln IIa oder IIb,

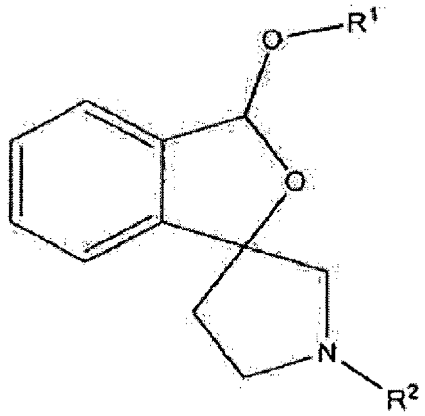

IIa

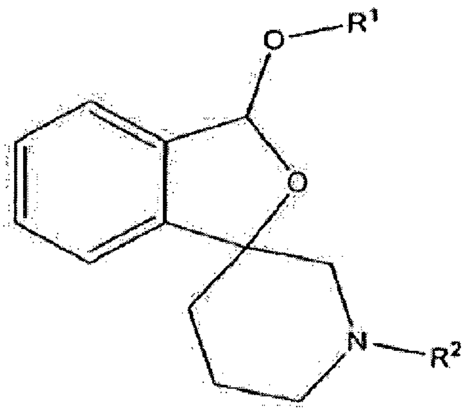

IIb

wobei

$R^1$ ausgewählt ist aus H, $C_{1-6}$-Alkyl;
$R^2$ ausgewählt ist aus Wasserstoff; wahlweise einer gesättigten oder ungesättigten, linearen oder verzweigten, mindestens monosubstituierten aliphatischen $C_{1-18}$-Gruppe; wahlweise mindestens monosubstituiertem $(CH_2)_{1-6}$-Aryl, $(CH_2)_{1-6}$-Heteroaryl, $(CH_2)_{1-6}$-Cycloalkyl, Cycloalkyl, Aryl oder Heterocyclyl;
oder $R^2$ zusammen mit dem gebundenen Stickstoff den Stickstoff in einem Cycloalkyl-Spirokomplex bindet und so ein quartäres Ammonium der Formel X (wobei q 1 oder 2 ist) bildet:

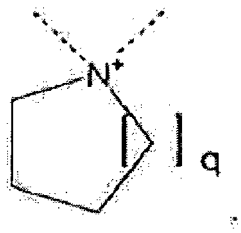

5. Verbindung nach Anspruch 3 mit einer der allgemeinen Formeln IIIa oder IIIb,

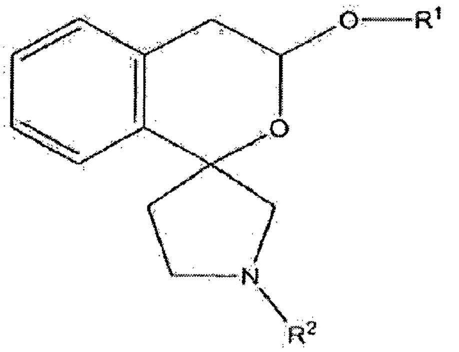

IIIa

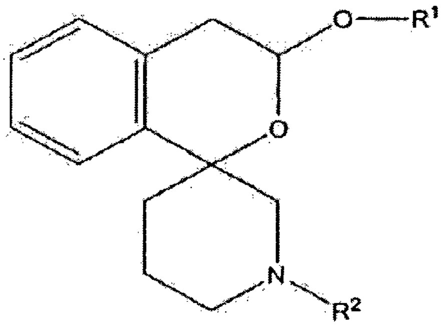

IIIb

wobei

$R^1$ ausgewählt ist aus H, $C_{1-6}$-Alkyl;
$R^2$ ausgewählt ist aus Wasserstoff; wahlweise einer gesättigten oder ungesättigten, linearen oder verzweigten, mindestens monosubstituierten aliphatischen $C_{1-18}$-Gruppe; wahlweise mindestens monosubstituiertem $(CH_2)_{1-6}$-Aryl, $(CH_2)_{1-6}$-Heteroaryl, $(CH2)_{1-6}$-Cycloalkyl, Cycloalkyl, Aryl oder Heterocyclyl;
oder $R^2$ zusammen mit dem gebundenen Stickstoff den Stickstoff in einem Cycloalkyl-Spirokomplex bindet und so ein quartäres Ammonium der Formel X (wobei q 1 oder 2 ist) bildet:

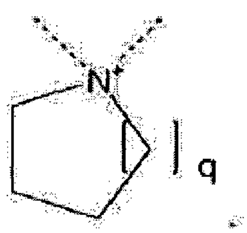

6. Verbindung nach einem der Ansprüche 1 bis 5, **dadurch gekennzeichnet, dass** $R^1$ ausgewählt ist aus H, $CH_3$ oder $C_2H_5$.

7. Verbindung nach einem der Ansprüche 1 bis 6, **dadurch gekennzeichnet, dass** R2 ausgewählt ist aus Wasserstoff; wahlweise einer gesättigten oder ungesättigten, linearen oder verzweigten, mindestens monosubsti-

tuierten aliphatischen $C_{1-18}$-Gruppe; wahlweise mindestens monosubstituiertem $(CH_2)_{1-6}$-Aryl; wahlweise mindestens monosubstituiertem Heteroaryl oder $(CH_2)_{1-6}$-Heteroaryl;
oder **dadurch gekennzeichnet, dass** $R^2$ ausgewählt ist aus
Wasserstoff; wahlweise einer gesättigten oder ungesättigten, linearen oder verzweigten, mindestens monosubstituierten aliphatischen $C_{9-12}$-Gruppe; wahlweise mindestens monosubstituiertem $(CH_2)_{1-6}$-Aryl; wahlweise mindestens monosubstituiertem $(CH_3)_{1-6}$-Alkylheteroaryl;
oder **dadurch gekennzeichnet, dass** $R_2$ zusammen mit dem gebundenen Stickstoff einen Cycloalkyl-Spirokomplex bildet und so ein quartäres Ammonium der Formel X (wobei q 1 oder 2 ist) bildet:

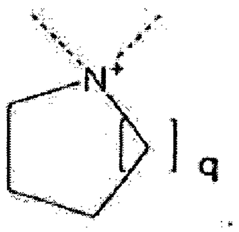

**8.** Verbindung nach Anspruch 7, wobei $R^2$ ausgewählt ist aus Wasserstoff; wahlweise einer gesättigten oder ungesättigten, linearen oder verzweigten, mindestens monosubstituierten aliphatischen $C_{1-18}$-Gruppe; wahlweise mindestens monosubstituiertem $(CH_2)_{1-6}$-Aryl; wahlweise mindestens monosubstituiertem $(CH_3)_{1-6}$-Heteroaryl.

**9.** Verbindung nach Anspruch 7, wobei $R^2$ ausgewählt ist aus Wasserstoff; wahlweise einer gesättigten oder ungesättigten, linearen oder verzweigten, mindestens monosubstituierten aliphatischen $C_{4-10}$-Gruppe; substituiertem oder unsubstituiertem $(CH_2)_{1-4}$ -Aryl; substituiertem oder unsubstituiertem $(CH_3)_{1-4}$-Heteroaryl,
oder $R^2$ ausgewählt ist aus wahlweise mindestens monosubstituiertem, linearen $C_{4-10}$-Alkyl oder wahlweise mindestens monosubstituiertem $(CH_2)_{1-4}$-Alkylheteroaryl.

**10.** Verbindung nach einem der Ansprüche 1 bis 9, **dadurch gekennzeichnet, dass** die Verbindung ausgewählt ist aus

1’-Benzyl-3-methoxy-3,4-dihydrospiro[[2]benzopyran-1,3’-piperidin] (1) (2)
3-Methoxy-1’-(2-phenylethyl)-3,4-dihydrospiro[[2]benzopyran-1,3’-piperidin] (4) (5)
3-Methoxy-1’-(3-phenylpropyl)-3,4-dihydrospiro[[2]benzopyran-1,3’-piperidin] (6) (7)
3-Methoxy-1’-(4-phenylbutyl)-3,4-dihydrospiro[[2]benzopyran-1,3’-piperidin) (8) (9)
1’-Butyl-3-methoxy-3,4-dihydrospiro[[2]benzopyran-1,3’-piperidin] (10) (11)
1’-Hexyl-3-methoxy-3,4-dihydrospiro[[2]benzopyran-1,3’-piperidin] (12) (13)
3-Methoxy-1’-octyl-3,4-dihydrospiro[[2]benzopyran-1,3’-piperidin] (14) (15)
1’-Decyl-3-methoxy-3,4-dihydrospiro[[2]benzopyran-1,3’-piperidin] (16) (17)
1’-Benzyl-3-methoxy-3,4-dihydrospiro[[2]benzopyran-1,3’-pyrrolidin](18)
3-Methoxy-1’-(2-phenylethyl)-3,4-dihydrospiro([2]benzopyran-1,3’-pyrrolidin] (20)
3-Methoxy-1’-(4-phenylbutyl)-3,4-dihydrospiro[[2]benzopyran-1,3’-pyrrolidin] (21)
1’-Butyl-3-methoxy-3,4-dihydrospiro[[2]benzopyran-1,3’-pyrrolidin] (22)
3-Methoxy-1’-octyl-3,4-dihydrospiro[[2]benzopyran-1,3’-pyrrolidin] (23)
1’-Benzyl-3-methoxy-3H-spiro[[2]benzofuran-1,3’-pyrrolidin] (25)
3-Methoxy-1’-(2-phenylethyl)-3H-spiro([2]benzofuran-1,3’-pyrrolidin] (27)
3-Methoxy-1’-(4-phenylbutyl)-3H-spiro[[2]benzofuran-1,3’-pyrrolidin] (28)
1’-Butyl-3-methoxy-3H-spiro[[2]benzofuran-1,3’-pyrrolidin] (29)
3-Methoxy-1’-octyl-3H-spiro[[2]benzofuran-1,3’-pyrrolidin] (30)
N,N-Cyclobutyl-3-methoxy-3H-spiro([2]benzofuran-1,3’-pyrrolidiniumbromid] (31) oder
1’-(4-(1H-imidazol-1-yl)-butyl)-3-methoxy-3H-spiro[[2]benzofuran-1,3’-pyrrolidin] (34);

wahlweise in Form eines der Stereoisomere, Enantiomere oder Diastereomere, eines Racemats oder in Form einer Mischung von mindestens zwei der Stereoisomere, Enantiomere und/oder Diastereomere in einem beliebigen Mischverhältnis oder eines entsprechenden Salzes davon oder eines entsprechenden Solvats davon.

**11.** Pharmazeutische Zusammensetzung, umfassend eine Verbindung nach einem der Ansprüche 1 bis 10 oder ein pharmazeutisch annehmbares Salz oder Solvat davon und einen pharmazeutisch annehmbaren Trägerstoff, Adjuvans oder Trägersubstanz.

**12.** Verwendung einer Verbindung nach einem der Ansprüche 1 bis 10 bei der Herstellung eines Arzneimittels für die Behandlung oder Prophylaxe einer durch Sigma-Rezeptor vermittelten Krankheit oder Zustands.

**13.** Verwendung nach Anspruch 12, wobei die Krankheit Durchfall, Lipoproteinstörungen, metabolisches Syndrom, Behandlung von erhöhten Triglyceridwerten, Chylomikronämie, Hyperlipoproteinämie; Hyperlipidämie, insbesondere gemischte Hyperlipidämie; Hypercholesterinämie, Dysbetalipoproteinämie, Hypertriglyceridämie, einschließlich der sporadischen und familiären Störung (angeborene Hypertriglyceridämie), Migräne, Fettsucht, Arthritis, Bluthochdruck, Arrhythmie, Geschwür, Lern-, Gedächtnis- und Aufmerksamkeitsdefizite, kognitive Störungen, neurodegenerative Krankheiten, demyelinisierende Krankheiten, Abhängigkeit von Drogen und chemischen Substanzen, einschließlich Kokain, Amphetamin, Ethanol und Nikotin, tardive Dyskinesie, ischämischer Schlaganfall, Epilepsie, Schlaganfall, Depression, Stress, psychotischer Zustand, Schizophrenie; Entzündung, Autoimmunkrankheiten oder Krebs ist; oder
wobei die Krankheit Schmerz ist, neuropathischer Schmerz, Entzündungsschmerz oder andere Schmerzzustände, Allodynie und/oder Hyperalgesie oder mechanische Allodynie.

**14.** Verbindung nach einem der Ansprüche 1 bis 10 zur Verwendung bei der Behandlung oder zur Prophylaxe einer durch Sigma-Rezeptor vermittelten Krankheit oder Zustands.

**15.** Verbindung nach einem der Ansprüche 1 bis 10 zur Verwendung bei der Behandlung oder zur Prophylaxe einer durch Sigma-Rezeptor vermittelten Krankheit oder Zustands, wobei die Krankheit Durchfall, Lipoproteinstörungen, metabolisches Syndrom, Behandlung von erhöhten Triglyceridwerten, Chylomikronämie, Hyperlipoproteinämie; Hyperlipidämie, insbesondere gemischte Hyperlipidämie; Hypercholesterinämie, Dysbetalipoproteinämie, Hypertriglyceridämie, einschließlich der sporadischen und familiären Störung (angeborene Hypertriglyceridämie), Migräne, Fettsucht, Arthritis, Bluthochdruck, Arrhythmie, Geschwür, Lern-, Gedächtnis- und Aufmerksamkeitsdefizite, kognitive Störungen, neurodegenerative Krankheiten, demyelinisierende Krankheiten, Abhängigkeit von Drogen und chemischen Substanzen, einschließlich Kokain, Amphetamin, Ethanol und Nikotin, tardive Dyskinesie, ischämischer Schlaganfall, Epilepsie, Schlaganfall, Depression, Stress, psychotischer Zustand, Schizophrenie; Entzündung, Autoimmunkrankheiten oder Krebs ist; oder
wobei die Krankheit Schmerz ist, neuropathischer Schmerz, Entzündungsschmerz oder andere Schmerzzustände, Allodynie und/oder Hyperalgesie oder mechanische Allodynie.

## Revendications

**1.** Composé de formule générale la

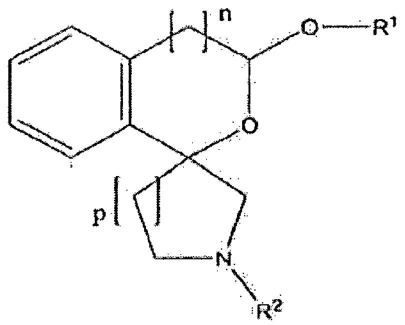

**Ia**

dans laquelle

n est choisi parmi 0 ou 1 ;
p est choisi parmi 1 ou 2 ;
$R^1$ est choisi parmi H, un groupe alkyle en $C_1$-$C_6$;
$R^2$ est choisi parmi un atome d'hydrogène ; un groupe aliphatique en $C_1$-$C_{18}$ facultativement au moins monosubstitué, saturé ou insaturé, linéaire ou ramifié ; un groupe $(CH_2)_{1-6}$-aryle, $(CH_2)_{1-6}$-hétéroaryle, $(CH_2)_{1-6}$-cycloalkyle, cycloalkyle aryle ou hétérocyclyle, facultativement au moins monosubstitué ;

ou $R^2$ avec l'atome d'azote lié se lie à l'atome d'azote sous la forme d'un complexe cyclaolkyl-spiro, formant ainsi un ammonium quaternaire de formule X (q étant 1 ou 2) :

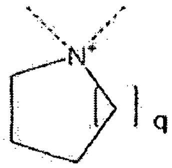

.

**2.** Composé selon la revendication 1, ayant la formule générale II

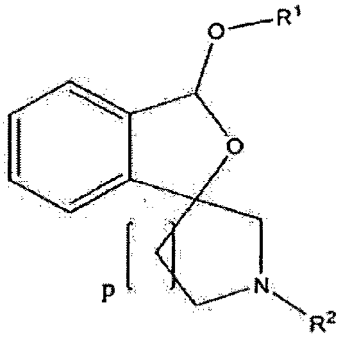

II

dans laquelle

p est choisi parmi 1 ou 2 ;
$R^1$ est choisi parmi H, un groupe alkyle en $C_1$-$C_6$;
$R^2$ est choisi parmi un atome d'hydrogène ; un groupe aliphatique en $C_1$-$C_{18}$ facultativement au moins monosubstitué, saturé ou insaturé, linéaire ou ramifié ; un groupe $(CH_2)_{1-6}$-aryle, $(CH_2)_{1-6}$-hétéroaryle, $(CH_2)_{1-6}$-cycloalkyle, cycloalkyle aryle ou hétérocyclyle, facultativement au moins monosubstitué ;
ou $R^2$ avec l'atome d'azote lié se lie à l'atome d'azote sous la forme d'un complexe cyclaolkyl-spiro, formant ainsi un ammonium quaternaire de formule X (q étant 1 ou 2) :

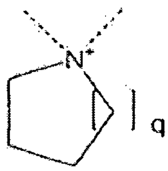

.

**3.** Composé selon la revendication 1, ayant la formule générale III

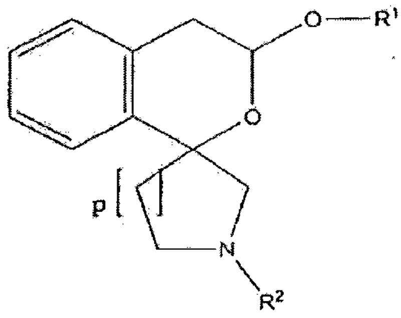

III

dans laquelle

p est choisi parmi 1 ou 2 ;
$R^1$ est choisi parmi H, un groupe alkyle en $C_1$-$C_6$ ;
$R^2$ est choisi parmi un atome d'hydrogène ; un groupe aliphatique en $C_1$-$C_{18}$ facultativement au moins monosubstitué, saturé ou insaturé, linéaire ou ramifié ; un groupe $(CH_2)_{1-6}$-aryle, $(CH_2)_{1-6}$-hétéroaryle, $(CH_2)_{1-6}$-cycloalkyle, cycloalkyle aryle ou hétérocyclyle, facultativement au moins monosubstitué ;
ou $R^2$ avec l'atome d'azote lié se lie à l'atome d'azote sous la forme d'un complexe cyclaolkyl-spiro, formant ainsi un ammonium quaternaire de formule X (q étant 1 ou 2) :

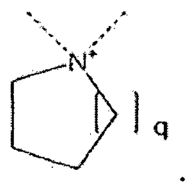

4. Composé selon la revendication 2, ayant l'une quelconque des formules générales IIa ou IIb

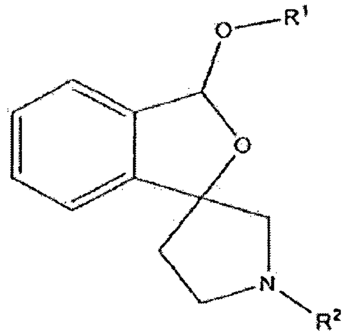

IIa

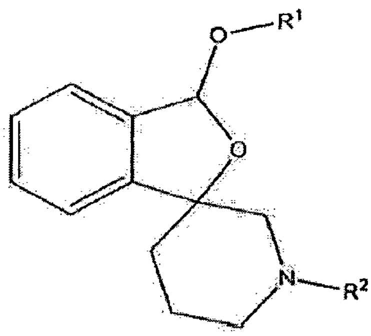

IIb

dans lesquelles

$R^1$ est choisi parmi H, un groupe alkyle en $C_1$-$C_6$;
$P^2$ est choisi parmi un atome d'hydrogène ; un groupe aliphatique en $C_1$-$C_{18}$ facultativement au moins monosubstitué, saturé ou insaturé, linéaire ou ramifié ; un groupe $(CH_2)_{1-6}$-aryle, $(CH_2)_{1-6}$-hétéroaryle, $(CH_2)_{1-6}$-cycloalkyle, cycloalkyle aryle ou hétérocyclyle, facultativement au moins monosubstitué ;
ou $R^2$ avec l'atome d'azote lié se lie à l'atome d'azote sous la forme d'un complexe cyclaolkyl-spiro, formant ainsi un ammonium quaternaire de formule X (q étant 1 ou 2) :

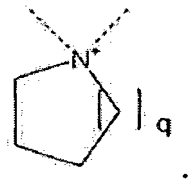

5. Composé selon la revendication 3, ayant l'une quelconque des formules générales IIIa ou IIIb

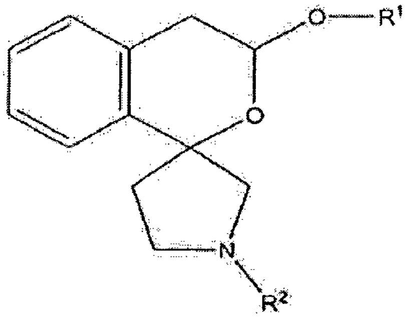

IIIa

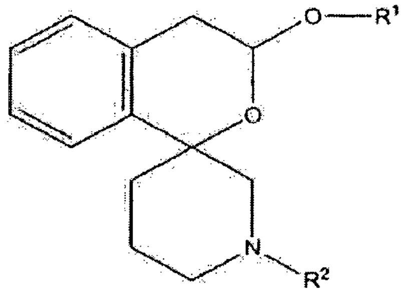

IIIb

dans lesquelles

$R^1$ est choisi parmi H, un groupe alkyle en $C_1$-$C_6$;
$R^2$ est choisi parmi un atome d'hydrogène ; un groupe aliphatique en $C_1$-$C_{18}$ facultativement au moins monosubstitué, saturé ou insaturé, linéaire ou ramifié ; un groupe $(CH_2)_{1-6}$-aryle, $(CH_2)_{1-6}$-hétéroaryle, $(CH_2)_{1-6}$-cycloalkyle, cycloalkyle aryle ou hétérocyclyle, facultativement au moins monosubstitué;
ou $R^2$ avec l'atome d'azote lié se lie à l'atome d'azote sous la forme d'un complexe cyclaolkyl-spiro, formant ainsi un ammonium quaternaire de formule X (q étant 1 ou 2) :

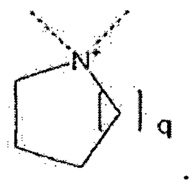

6. Composé selon l'une quelconque des revendications 1 à 5, **caractérisé en ce que** $R^1$ est choisi parmi H, $CH_3$ ou $C_2H_5$.

7. Composé selon l'une quelconque des revendications 1 à 6, **caractérisé en ce que** $R^2$ est choisi parmi
un atome d'hydrogène ; un groupe aliphatique en $C_1$-$C_{18}$ facultativement au moins monosubstitué, saturé ou insaturé, linéaire ou ramifié ; un groupe aryle ou $(CH_2)_{1-6}$-aryle, facultativement au moins monosubstitué; un groupe hétéroaryle ou $(CH_2)_{1-6}$-hétéroaryle, facultativement au moins monosubstitué ;
ou **caractérisé en ce que** $R^2$ est choisi parmi
un atome d'hydrogène ; un groupe aliphatique en $C_4$-$C_{12}$ facultativement au moins monosubstitué, saturé ou insaturé, linéaire ou ramifié ; un groupe $(CH_2)_{1-6}$-aryle facultativement au moins monosubstitué ; un groupe $(CH_2)_{1-6}$-alkylhétéroaryle facultativement au moins monosubstitué ;
ou **caractérisé en ce que** $R^2$ forme avec l'atome d'azote lié un complexe cyclaolkyl-spiro, formant ainsi un ammonium quaternaire de formule X (q étant 1 ou 2) :

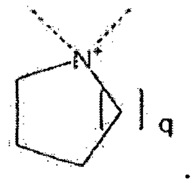

**8.** Composé selon la revendication 7, dans lequel $R^2$ est choisi parmi un atome d'hydrogène ; un groupe aliphatique en $C_1$-$C_{18}$ facultativement au moins monosubstitué, saturé ou insaturé, linéaire ou ramifié, substitué ou non substitué ; un groupe (C-$H_2$)$_{1-6}$-aryle facultativement au moins monosubstitué, un groupe $(CH_2)_{1-6}$-hétéroaryle facultativement au moins monosubstitué.

**9.** Composé selon la revendication 7, dans lequel $R^2$ est choisi parmi un atome d'hydrogène ; un groupe aliphatique en $C_4$-$C_{10}$ facultativement au moins monosubstitué, saturé ou insaturé, linéaire ou ramifié, substitué ou non substitué ; un groupe $(CH_2)_{1-4}$-aryle substitué ou non substitué ; un groupe $(CH_2)_{1-4}$-hétéroaryle substitué ou non substitué, ou $R^2$ est choisi parmi un groupe alkyle en $C_4$-$C_{10}$ linéaire facultativement au moins monosubstitué ou un groupe $(CH_2)_{1-4}$-alkylhétéroaryle facultativement au moins monosubstitué.

**10.** Composé selon l'une quelconque des revendications 1 à 9, **caractérisé en ce que** le composé est choisi parmi

1l'-benzyl-3-méthoxy-3,4-dihydrospiro[[2]benzopyran-1,3'-pipéridine] (1) (2)
3-méthoxy-1'-(2-phényléthyl)-3,4-dihydrospiro[[2]benzopyran-1,3'-pipéridine] (4) (5)
3-méthoxy-1'-(3-phénylpropyl)-3,4-dihydrospiro[[2]benzopyran-1,3'-pipéridine] (6) (7)
3-méthoxy-1'-(4-phénylbutyl)-3,4-dihydrospiro[[2]benzopyran-1,3'-pipéridine] (8) (9)
1'-butyl-3-méthoxy-3,4-dihydrospiro[[2]benzopyran-1,3'-pipéridine] (10) (11)
1'-hexyl-3-méthoxy-3,4-dihydrospiro[[2]benzopyran-1,3'-pipéridine] (12) (13)
3-méthoxy-1'-octyl-3,4-dihydrospiro[[2]benzopyran-1,3'-pipéridine] (14) (15)
1'-décyl-3-méthoxy-3,4-dihydrospiro[[2]benzopyran-1,3'-pipéridine] (16) (17)
1'-benzyl-3-méthoxy-3,4-dihydrospiro[[2]benzopyran-1,3'-pyrrolidine] (18)
3-méthoxy-1'-(2-phényléthyl)-3,4-dihydrospiro[[2]benzopyran-1,3'-pyrrolidine] (20)
3-méthoxy-1'-(4-phénylbutyl)-3,4-dihydrospiro[[2]benzopyran-1,3'-pyrrolidine] (21)
1'-butyl-3-méthoxy-3,4-dihydrospiro[[2]benzopyran-1,3'-pyrrolidine] (22)
3-méthoxy-1'-octyl-3,4-dihydrospiro[[2]benzopyran-1,3'-pyrrolidine] (23)
1'-benzyl-3-méthoxy-3H-spiro[[2]benzofuran-1,3'-pyrrolidine] (25)
3-méthoxy-1'-(2-phényléthyl)3H-spiro [[2]benzofuran-1,3'-pyrrolidine] (27)
3-méthoxy-1'-(4-phénylbutyl)-3H-spiro [[2]benzofuran-1,3'-pyrrolidine] (28)
1'-butyl-3-méthoxy-3H-spiro[[2]benzofuran-1,3'-pyrrolidine] (29)
3-méthoxy-1'-octyl-3H-spiro[[2]benzofuran-1,3'-pyrrolidine] (30)
N,N-cyclobutyl-3-méthoxy-3H-spiro[[2]benzofuran-1,3'-pyrrolidiniumbromure] (31) ou
1'-(4-(1H-imidazol-1-yl)-butyl)-3-méthoxy-3H-spiro[[2]benzofuran-1,3'-pyrrolidine] (34) ;

facultativement sous la forme d'un isomère choisi parmi les stéréoisomères, les énantiomères et les diastéréoisomères, d'un racémate ou sous la forme d'un mélange d'au moins deux des stéréoisomères, énantiomères et/ou diastéréoisomères, selon n'importe quel rapport de mélange, ou d'un sel correspondant de ceux-ci, ou d'un solvate correspondant de ceux-ci.

**11.** Composition pharmaceutique qui comprend un composé tel que défini selon l'une quelconque des revendications 1 à 10 ou un sel pharmaceutiquement acceptable, ou un solvate de celui-ci, et un support, un adjuvant ou un véhicule pharmaceutiquement acceptable.

**12.** Utilisation d'un composé tel que défini selon l'une quelconque des revendications 1 à 10 pour la préparation d'un médicament pour le traitement ou la prophylaxie d'une maladie ou d'une pathologie médiée par le récepteur sigma.

**13.** Utilisation selon la revendication 12 dans laquelle la maladie est la diarrhée, les troubles lipoprotéiques, le syndrome métabolique, les taux élevés de triglycérides, la chylomicronémie, l'hyperlipoprotéinémie, l'hyperlipidémie, en particulier l'hyperlipidémie mixte, l'hypercholestérolémie, la dysbêtalipoprotéinémie, l'hypertriglycéridémie y compris le trouble sporadique et familial (hypertriglycéridémie héréditaire), la migraine, l'obésité, l'arthrite, l'hypertension,

l'arythmie, l'ulcère, les troubles de l'apprentissage, de la mémoire et de l'attention, les troubles de la cognition, les maladies neurodégénératives, les maladies démyélinisantes, la dépendance aux médicaments et aux substances chimiques y compris la cocaïne, les amphétamines, l'éthanol et la nicotine, la dyskinésie tardive, l'accident ischémique cérébral, l'épilepsie, l'accident vasculaire cérébral, la dépression, le stress, la pathologie psychotique, la schizophrénie, l'inflammation, les maladies auto-immunes ou le cancer ; ou
dans laquelle la maladie est la douleur, la douleur neuropathique, la douleur inflammatoire ou d'autres douleurs pathologiques, l'allodynie et/ou l'hyperalgésie, ou l'allodynie mécanique.

**14.** Composé tel que défini selon l'une quelconque des revendications 1 à 10 pour une utilisation dans le traitement ou la prophylaxie d'une maladie ou d'une pathologie médiée par le récepteur sigma.

**15.** Composé tel que défini selon l'une quelconque des revendications 1 à 10 pour une utilisation dans le traitement ou la prophylaxie d'une maladie ou d'une pathologie médiée par le récepteur sigma, dans lequel la maladie est la diarrhée, les troubles lipoprotéiques, le syndrome métabolique, les taux élevés de triglycérides, la chylomicronémie, l'hyperlipoprotéinémie, l'hyperlipidémie, en particulier l'hyperlipidémie mixte, l'hypercholestérolémie, la dysbêtalipoprotéinémie, l'hypertriglycéridémie y compris le trouble sporadique et familial (hypertriglycéridémie héréditaire), la migraine, l'obésité, l'arthrite, l'hypertension, l'arythmie, l'ulcère, les troubles de l'apprentissage, de la mémoire et de l'attention, les troubles de la cognition, les maladies neurodégénératives, les maladies démyélinisantes, la dépendance aux médicaments et aux substances chimiques y compris la cocaïne, les amphétamines, l'éthanol et la nicotine, la dyskinésie tardive, l'accident ischémique cérébral, l'épilepsie, l'accident vasculaire cérébral, la dépression, le stress, la pathologie psychotique, la schizophrénie, l'inflammation, les maladies auto-immunes ou le cancer ; ou
dans lequel la maladie est la douleur, la douleur neuropathique, la douleur inflammatoire ou d'autres douleurs pathologiques, l'allodynie et/ou l'hyperalgésie, ou l'allodynie mécanique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3686186 A
- US 3962259 A
- US 3959475 A
- WO 0107050 A
- WO 0006545 A
- WO 0248152 A


### Non-patent literature cited in the description


- **WALKER, J.M. et al.** *Pharmacological Reviews,* 1990, vol. 42, 355
- **SNYDER, S.H. ; LARGENT, B.L.** *J. Neuropsychiatry,* 1989, vol. 1, 7
- **QUIRION, R. et al.** *Trends Pharmacol. Sci.,* 1992, vol. 13, 85-86
- **HANNER, M. et al.** *Proc. Natl. Acad. Sci.,* 1996, vol. 93, 8072-8077
- *J. Org. Chem.,* 1975, 1427 ff
- *Archiv. Pharm.,* 1993, 513 ff
- *J. Med. Chem.,* 1999, 4478 ff
- *J. Med. Chem.,* 2002, 4923 ff
- *J. Med. Chem.,* 2002, 438 ff
- **KROGSGAARD-LARSEN et al.** Textbook of Drug design and Discovery. Taylor & Francis, April 2002
- **MAIER ; WÜNSCH.** *J.Med.Chem.,* 2002, vol. 45, 438-448
- **MAIER ; WÜNSCH.** *J.Med.Chem.,* 2002, vol. 45, 4923-4930
- **DEHAVEN-HUDKINS, D. L. ; L.C. FLEISSNER ; F. Y. FORD-RICE.** Characterization of the binding of [3H](+)pentazocine to σ recognition sites in guinea pig brain. *Eur. J. Pharmacol.,* 1992, vol. 227, 371-378
- **RADESCA, L. ; W.D. BOWEN ; L. DI PAOLO ; B.R. DE COSTA.** Synthesis and Receptor Binding of Enantiomeric N-Substituted cis-N-[2-(3,4-Dichlorophenyl)ethyl]-2-(1-pyrrolidinyl)cyclohexylamines as High-Affinity σ Receptor Ligands. *J. Med. Chem.,* 1991, vol. 34, 3065-3074
- **LANGA, F. ; CODONY X. ; TOVAR V. ; LAVADO A. ; GIMÉNEZ E. ; COZAR P. ; CANTERO M. ; DORDAL A. ; HERNÁNDEZ E. ; PÉREZ R.** Generation and phenotypic analysis of sigma receptor type I (Sigma1) knockout mice. *European Journal of Neuroscience,* 2003, vol. 18, 2188-2196
- **LOWRY, O.H. ; N.J. ROSEBROUGH ; A.L. FARR ; R.J. RANDALL.** Protein measurement with the Folin phenol reagent. *J. Biol. Chem,* 1951, vol. 193, 265